# EUROPEAN PATENT APPLICATION

(11) **EP 1 674 479 A1**
(43) Date of publication of application: **28.06.2006**
(21) Application number: 05250446.1
(22) Date of filing: 28.01.2005
(51) Int. Cl.: C07K 14/705, C07K 16/28

(54) **Modulation of Fc Gamma receptors for optimizing immunotherapy**

(30) Priority: 22.12.2004 US 638422 P; 30.12.2004 US 640685 P; 27.01.2005 US 647616 P
(71) Applicant: MEMORIAL SLOAN-KETTERING CANCER CENTER, New York, NY 10021-6007 (US)
(72) Inventor: Boruchov, Adam, New York NY 10021 (US); Young, James, W., Pelham Manor, New York 10803-2936 (US)
(74) Representative: Roques, Sarah Elizabeth

(57) **Abstract**

The present invention relates to methods for modulating the maturation of dendritic cells. In particular, the invention provides methods for (a) promoting the maturation of dendritic cells thereby producing mature dendritic cells, and (b) preventing the maturation of dendritic cells thereby preventing maturation and producing tolerogenic dendritic cells. The present invention further relates to methods for treating autoimmune diseases with tolerogenic dendritic cells produced by methods of the invention. The present invention also relates to methods for treating cancer, neoplastic diseases, and infectious diseases with mature dendritic cells produced by methods of the present invention. The invention moreover relates to kits that provide the materials for conducting the methods of the invention.

## Description

Each of the applications and patents cited in this text, as well as each document or reference cited in each of the applications and patents (including during the prosecution of each issued patent; "application cited documents"), and each of the PCT and foreign applications or patents corresponding to and/or claiming priority from any of these applications and patents, and each of the documents cited or referenced in each of the application cited documents, are hereby expressly incorporated herein by reference, and may be employed in the practice of the invention. More generally, documents or references are cited in this text, either in a Reference List before the claims, or in the text itself; and, each of these documents or references ("herein cited references"), as well as each document or reference cited in each of the herein cited references (including any manufacturer's specifications, instructions, etc.), is hereby expressly incorporated herein by reference.

Reference is made to U.S. Application Serial Number 10/643857, filed on August 14, 2003, the contents of which are expressly incorporated herein by reference.

### 1. FIELD OF THE INVENTION

The present invention relates to methods for modulating the maturation of dendritic cells. In particular, the invention provides methods for (a) promoting the maturation of dendritic cells thereby producing mature dendritic cells, and (b) preventing the maturation of dendritic cells thereby preventing maturation and producing tolerogenic dendritic cells. The present invention further relates to methods for treating autoimmune diseases with tolerogenic dendritic cells produced by methods of the invention. The present invention also relates to methods for treating cancer, neoplastic diseases, and infectious diseases with mature dendritic cells produced by methods of the present invention. The invention moreover relates to kits that provide the materials for conducting the methods of the invention.

### 2. BACKGROUND OF THE INVENTION

### 2.1 Fc RECEPTORS AND THEIR ROLES IN THE IMMUNE SYSTEM

The interaction of antibody-antigen complexes with cells of the immune system results in a wide array of responses, ranging from effector functions such as antibody-dependent cytotoxicity, mast cell degranulation, and phagocytosis to immunomodulatory signals such as regulating lymphocyte proliferation and antibody secretion. All these interactions are initiated through the binding of the Fc domain of antibodies or immune complexes to specialized cell surface receptors on hematopoietic cells. The diversity of cellular responses triggered by antibodies and immune complexes results from the structural heterogeneity of Fc receptors. Fc receptors share structurally related ligand binding domains which presumably mediate intracellular signaling.

The Fc receptors, members of the immunoglobulin gene superfamily of proteins, are surface glycoproteins that can bind the Fc portion of immunoglobulin molecules. Each member of the family recognizes immunoglobulins of one or more isotypes through a recognition domain on the a chain of the Fc receptor. Fc receptors are defined by their specificity for immunoglobulin subtypes. Fc receptors for IgG are referred to as FcγR, for IgE as FcεR, and for IgA as FcαR. Different accessory cells bear Fc receptors for antibodies of different isotype, and the isotype of the antibody determines which accessory cells will be engaged in a given response (reviewed by Ravetch J.V. *et al.* 1991, *Annu. Rev. Immunol.* 9: 457-92; Gerber J.S. *et al.* 2001 *Microbes and Infection,* 3: 131-139; Billadeau D.D. *et al.* 2002, *The Journal of Clinical Investigation,* 2(109): 161-1681; Ravetch J.V. *et al.* 2000, *Science,* 290: 84-89; Ravetch J.V. *et al.,* 2001 *Annu. Rev. Immunol.* 19:275-90; Ravetch J.V. 1994, *Cell,* 78(4): 553-60). The different Fc receptors, the cells that express them, and their isotype specificity is summarized in Table 1 (adapted from Immunobiology: The Immune System in Health and Disease, 4^{th} ed. 1999, Elsevier Science Ltd/Garland Publishing, New York).

### Fcγ Receptors

Each member of this family is an integral membrane glycoprotein, possessing extracellular domains related to a C2-set of immunoglobulin-related domains, a single membrane spanning domain and an intracytoplasmic domain of variable length. There are three known FcγRs, designated FcγRI(CD64), FcγRII(CD32), and FcγRIII(CD16). The three receptors are encoded by distinct genes; however, the extensive homology between the three family members suggest they arose from a common progenitor perhaps by gene duplication. This invention specifically focuses on FeγRII(CD32).

### FcγRII(CD32)

FcγRII proteins are 40KDa integral membrane glycoproteins which bind only the complexed IgG due to a low affinity for monomeric Ig (10⁶ M⁻¹). This receptor is the most widely expressed FcγR, present on all hematopoietic cells, including monocytes, macrophages, B cells, NK cells, neutrophils, mast cells, and platelets. FcγRII has only two immunoglobulin-like regions in its immunoglobulin binding chain and hence a much lower affinity for IgG than FcγRI. There are three human FcγRII genes (FcγRII-A, FcγRII-B, FcγRII-C), all of which bind IgG in aggregates or immune complexes.

Distinct differences within the cytoplasmic domains of FcγRII-A and FcγRII-B create two functionally heterogenous responses to receptor ligation. The fundamental difference is that the A isoform initiates intracellular signaling leading to cell activation such as phagocytosis and respiratory burst, whereas the B isoform initiates inhibitory signals, e.g., inhibiting B-cell activation.

### Signaling through FcγRs

Both activating and inhibitory signals are transduced through the FcγRs following ligation. These diametrically opposing functions result from structural differences among the different receptor isoforms. Two distinct domains within the cytoplasmic signaling domains of the receptor called immunoreceptor tyrosine based activation motifs (ITAMs) or immunoreceptor tyrosine based inhibitory motifs (ITIMS) account for the different responses. The recruitment of different cytoplasmic enzymes to these structures dictates the outcome of the FcγR-mediated cellular responses. ITAM-containing FcγR complexes include FcγRI, FcγRIIA, FcγRIIIA, whereas ITIM-containing complexes only include FcγRIIB.

Human neutrophils express the FcγRIIA gene. FcγRIIA clustering via immune complexes or specific antibody cross-linking serves to aggregate ITAMs along with receptor-associated kinases which facilitate ITAM phosphorylation. ITAM phosphorylation serves as a docking site for Syk kinase, activation of which results in activation of downstream substrates (e.g., PI₃K). Cellular activation leads to release of proinflammatory mediators.

The FcγRIIB gene is expressed on B lymphocytes; its extracellular domain is 96% identical to FcγRIIA and binds IgG complexes in an indistinguishable manner. The presence of an ITIM in the cytoplasmic domain of FcγRIIB defines this inhibitory subclass of FcγR. Recently the molecular basis of this inhibition was established. When colligated along with an activating FcγR, the ITIM in FcγRIIB becomes phosphorylated and attracts the SH2 domain of the inosital polyphosphate 5'-phosphatase (SHIP), which hydrolyzes phosphoinositol messengers released as a consequence of ITAM-containing FcyR- mediated tyrosine kinase activation, consequently preventing the influx of intracellular Ca⁺⁺. Thus crosslinking of FcγRIIB dampens the activating response to FcγR ligation and inhibits cellular responsiveness. B cell activation, B cell proliferation and antibody secretion is thus aborted.

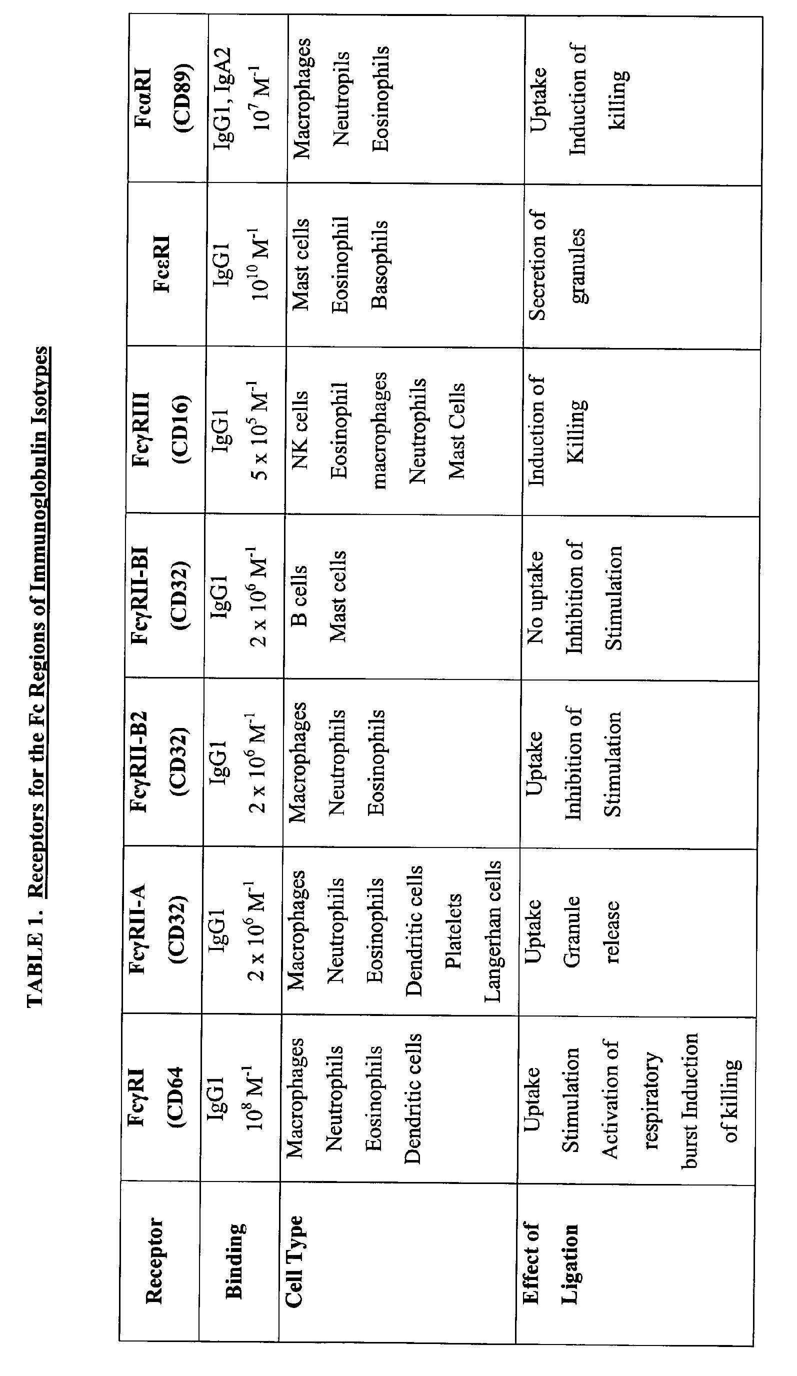

### 2.2 DISEASES OF RELEVANCE

### 2.2.1 CANCER

A neoplasm, or tumor, is a neoplastic mass resulting from abnormal uncontrolled cell growth which can be benign or malignant. Benign tumors generally remain localized. Malignant tumors are collectively termed cancers. The term "malignant" generally means that the tumor can invade and destroy neighboring body structures and spread to distant sites to cause death (for review, *see* Robbins and Angell, *1976, Basic Pathology,* 2d Ed., W.B. Saunders Co., Philadelphia, pp. 68-122). Cancer can arise in many sites of the body and behave differently depending upon its origin. Cancerous cells destroy the part of the body in which they originate and then spread to other part(s) of the body where they start new growth and cause more destruction.

More than 1.2 million Americans develop cancer each year. Cancer is the second leading case of death in the United States and if current trends continue, cancer is expected to be the leading cause of the death by the year 2010. Lung and prostate cancer are the top cancer killers for men in the United States. Lung and breast cancer are the top cancer killers for women in the United States. One in two men in the United States will be diagnosed with cancer at some time during his lifetime. One in three women in the United States will be diagnosed with cancer at some time during her lifetime.

A cure for cancer has yet to be found. Current treatment options, such as surgery, chemotherapy and radiation treatment, are oftentimes either ineffective or present serious side effects.

### Cancer Therapy

Currently, cancer therapy may involve surgery, chemotherapy, hormonal therapy and/or radiation treatment to eradicate neoplastic cells in a patient *(See,* for example, Stockdale, 1998, "Principles of Cancer Patient Management", in Scientific American: Medicine, vol. 3, Rubenstein and Federman, eds., Chapter 12, Section IV). Recently, cancer therapy could also involve biological therapy or immunotherapy. All of these approaches pose significant drawbacks for the patient. Surgery, for example, may be contraindicated due to the health of the patient or may be unacceptable to the patient. Additionally, surgery may not completely remove the neoplastic tissue. Radiation therapy is only effective when the neoplastic tissue exhibits a higher sensitivity to radiation than normal tissue, and radiation therapy can also often elicit serious side effects. Hormonal therapy is rarely given as a single agent and although can be effective, is often used to prevent or delay recurrence of cancer after other treatments have removed the majority of the cancer cells. Biological therapies/immunotherapies are limited in number and may produce side effects such as rashes or swellings, flu-like symptoms, including fever, chills and fatigue, digestive tract problems or allergic reactions.

With respect to chemotherapy, there are a variety of chemotherapeutic agents available for treatment of cancer. A significant majority of cancer chemotherapeutics act by inhibiting DNA synthesis, either directly, or indirectly by inhibiting the biosynthesis of the deoxyribonucleotide triphosphate precursors, to prevent DNA replication and concomitant cell division *(See,* for example, *Gilman et al.,* Goodman and Gilman's: The Pharmacological Basis of Therapeutics, Eighth Ed. (Pergamom Press, New York, 1990)). These agents, which include alkylating agents, such as nitrosourea, anti-metabolites, such as methotrexate and hydroxyurea, and other agents, such as etoposides, campathecins, bleomycin, doxorubicin, daunorubicin, etc., although not necessarily cell cycle specific, kill cells during S phase because of their effect on DNA replication. Other agents, specifically colchicine and the vinca alkaloids, such as vinblastine and vincristine, interfere with microtubule assembly resulting in mitotic arrest. Chemotherapy protocols generally involve administration of a combination of chemotherapeutic agents to increase the efficacy of treatment.

Despite the availability of a variety of chemotherapeutic agents, chemotherapy has many drawbacks *(See,* for example, Stockdale, 1998, "Principles Of Cancer Patient Management" in Scientific American Medicine, vol. 3, Rubenstein and Federman, eds., ch. 12, sect. 10). Almost all chemotherapeutic agents are toxic, and chemotherapy causes significant, and often dangerous, side effects, including severe nausea, bone marrow depression, immunosuppression, *etc.* Additionally, even with administration of combinations of chemotherapeutic agents, many tumor cells are resistant or develop resistance to the chemotherapeutic agents. In fact, those cells resistant to the particular chemotherapeutic agents used in the treatment protocol often prove to be resistant to other drugs, even those agents that act by mechanisms different from the mechanisms of action of the drugs used in the specific treatment; this phenomenon is termed pleiotropic drug or multidrug resistance. Thus, because of drug resistance, many cancers prove refractory to standard chemotherapeutic treatment protocols.

There is a significant need for alternative cancer treatments, particularly for treatment of cancer that has proved refractory to standard cancer treatments, such as surgery, radiation therapy, chemotherapy, and hormonal therapy. A promising alternative is immunotherapy, in which cancer cells are specifically targeted by cancer antigen-specific antibodies. Major efforts have been directed at harnessing the specificity of the immune response, for example, hybridoma technology has enabled the development of tumor selective monoclonal antibodies *(See* Green *M.C. et al.,* 2000 *Cancer Treat Rev.,* 26: 269-286; Weiner LM, 1999 *Semin Oncol.* 26(suppl. 14):43-51), and in the past few years, the Food and Drug Administration has approved the first MAbs for cancer therapy: Rituxin (anti-CD20) for non-Hodgkin's Lymphoma and Herceptin [anti-(c-erb-2/HER-2)] for metastatic breast cancer (Suzanne A. Eccles, 2001, *Breast Cancer Res.,* 3: 86-90). However, the potency of antibody effector function, e.g., to mediate antibody dependent cellular cytotoxicity ("ADCC") is an obstacle to such treatment. Methods to improve the efficacy of such immunotherapy are thus needed.

### 2.2.2 INFLAMMATORY DISEASES AND AUTOIMMUNE DISEASES

Inflammation is a process by which the body's white blood cells and chemicals protect our bodies from infection by foreign substances, such as bacteria and viruses. It is usually characterized by pain, swelling, warmth and redness of the affected area. Chemicals known as cytokines and prostaglandins control this process, and are released in an ordered and self-limiting cascade into the blood or affected tissues. This release of chemicals increases the blood flow to the area of injury or infection, and may result in the redness and warmth. Some of the chemicals cause a leak of fluid into the tissues, resulting in swelling. This protective process may stimulate nerves and cause pain. These changes, when occurring for a limited period in the relevant area, work to the benefit of the body.

In autoimmune and/or inflammatory disorders, the immune system triggers an inflammatory response when there are no foreign substances to fight and the body's normally protective immune system causes damage to its own tissues by mistakenly attacking self. There are many different autoimmune disorders which affect the body in different ways. For example, the brain is affected in individuals with multiple sclerosis, the gut is affected in individuals with Crohn's disease, and the synovium, bone and cartilage of various joints are affected in individuals with rheumatoid arthritis. As autoimmune disorders progress destruction of one or more types of body tissues, abnormal growth of an organ, or changes in organ function may result. The autoimmune disorder may affect only one organ or tissue type or may affect multiple organs and tissues. Organs and tissues commonly affected by autoimmune disorders include red blood cells, blood vessels, connective tissues, endocrine glands (e.g., the thyroid or pancreas), muscles, joints, and skin. Examples of autoimmune disorders include, but are not limited to, Hashimoto's thyroiditis, pernicious anemia, Addison's disease, type 1 diabetes, rheumatoid arthritis, systemic lupus erythematosus, dermatomyositis, Sjogren's syndrome, dermatomyositis, lupus erythematosus, multiple sclerosis, autoimmune inner ear disease myasthenia gravis, Reiter's syndrome, Graves disease, autoimmune hepatitis, familial adenomatous polyposis and ulcerative colitis.

Rheumatoid arthritis (RA) and juvenile rheumatoid arthritis are types of inflammatory arthritis. Arthritis is a general term that describes inflammation in joints. Some, but not all, types of arthritis are the result of misdirected inflammation. Besides rheumatoid arthritis, other types of arthritis associated with inflammation include the following: psoriatic arthritis, Reiter's syndrome, ankylosing spondylitis arthritis, and gouty arthritis. Rheumatoid arthritis is a type of chronic arthritis that occurs in joints on both sides of the body (such as both hands, wrists or knees). This symmetry helps distinguish rheumatoid arthritis from other types of arthritis. In addition to affecting the joints, rheumatoid arthritis may occasionally affect the skin, eyes, lungs, heart, blood or nerves.

Rheumatoid arthritis affects about 1% of the world's population and is potentially disabling. There are approximately 2.9 million incidences of rheumatoid arthritis in the United States. Two to three times more women are affected than men. The typical age that rheumatoid arthritis occurs is between 25 and 50. Juvenile rheumatoid arthritis affects 71,000 young Americans (aged eighteen and under), affecting six times as many girls as boys.

Rheumatoid arthritis is an autoimmune disorder where the body's immune system improperly identifies the synovial membranes that secrete the lubricating fluid in the joints as foreign. Inflammation results, and the cartilage and tissues in and around the joints are damaged or destroyed. In severe cases, this inflammation extends to other joint tissues and surrounding cartilage, where it may erode or destroy bone and cartilage and lead to joint deformities. The body replaces damaged tissue with scar tissue, causing the normal spaces within the joints to become narrow and the bones to fuse together. Rheumatoid arthritis creates stiffness, swelling, fatigue, anemia, weight loss, fever, and often, crippling pain. Some common symptoms of rheumatoid arthritis include joint stiffness upon awakening that lasts an hour or longer; swelling in a specific finger or wrist joints; swelling in the soft tissue around the joints; and swelling on both sides of the joint. Swelling can occur with or without pain, and can worsen progressively or remain the same for years before progressing.

The diagnosis of rheumatoid arthritis is based on a combination of factors, including: the specific location and symmetry of painful joints, the presence of joint stiffness in the morning, the presence of bumps and nodules under the skin (rheumatoid nodules), results of X-ray tests that suggest rheumatoid arthritis, and/or positive results of a blood test called the rheumatoid factor. Many, but not all, people with rheumatoid arthritis have the rheumatoid-factor antibody in their blood. The rheumatoid factor may be present in people who do not have rheumatoid arthritis. Other diseases can also cause the rheumatoid factor to be produced in the blood. That is why the diagnosis of rheumatoid arthritis is based on a combination of several factors and not just the presence of the rheumatoid factor in the blood.

The typical course of the disease is one of persistent but fluctuating joint symptoms, and after about 10 years, 90% of sufferers will show structural damage to bone and cartilage. A small percentage will have a short illness that clears up completely, and another small percentage will have very severe disease with many joint deformities, and occasionally other manifestations of the disease. The inflammatory process causes erosion or destruction of bone and cartilage in the joints. In rheumatoid arthritis, there is an autoimmune cycle of persistent antigen presentation, T-cell stimulation, cytokine secretion, synovial cell activation, and joint destruction. The disease has a major impact on both the individual and society, causing significant pain, impaired function and disability, as well as costing millions of dollars in healthcare expenses and lost wages. *(See,* for example, the NIH website and the NIAID website).

Currently available therapy for arthritis focuses on reducing inflammation of the joints with anti-inflammatory or immunosuppressive medications. The first line of treatment of any arthritis is usually anti-inflammatories, such as aspirin, ibuprofen and Cox-2 inhibitors such as celecoxib and rofecoxib. "Second line drugs" include gold, methotrexate and steroids. Although these are well-established treatments for arthritis, very few patients remit on these lines of treatment alone. Recent advances in the understanding of the pathogenesis of rheumatoid arthritis have led to the use of methotrexate in combination with antibodies to cytokines or recombinant soluble receptors. For example, recombinant soluble receptors for tumor necrosis factor (TNF)-α have been used in combination with methotrexate in the treatment of arthritis. However, only about 50% of the patients treated with a combination of methotrexate and anti-TNF-α agents such as recombinant soluble receptors for TNF-α show clinically significant improvement. Many patients remain refractory despite treatment. Difficult treatment issues still remain for patients with rheumatoid arthritis. Many current treatments have a high incidence of side effects or cannot completely prevent disease progression. So far, no treatment is ideal, and there is no cure. Novel therapeutics are needed that more effectively treat rheumatoid arthritis and other autoimmune disorders.

### 3. SUMMARY OF THE INVENTION

The present invention provides methods for modulating the differentiation of dendritic cells. More specifically, the present invention provides methods for modulating the maturation of dendritic cells. In particular, the invention provides methods for (a) promoting the maturation of dendritic cells thereby producing mature dendritic cells, and (b) preventing the maturation of dendritic cells thereby preventing maturation and/or inducing phenotypic changes resulting in the production of tolerogenic dendritic cells. Without being bound by theory, immature dendritic cells, such as, but not limited to, monocyte derived dendritic cells or myeloid blood dendritic cells, coexpress activating and inhibitory Fc gamma receptors. The present inventors have found that targeting activating Fc gamma receptors, i.e., preferentially or specifically activating the signaling pathway(s) downstream of activating Fc gamma receptors, results in the maturation of dendritic cells. Targeting inhibitory Fc gamma receptors, *i.e.,* preferentially or specifically activating the signaling pathway(s) downstream of inhibitory Fc gamma receptors, prevents maturation and results in phenotypic changes related to tolerogenic dendritic cells.

Targeting of activating Fc gamma receptors can be achieved by different approaches. In certain embodiments of the invention, activating Fc gamma receptors are targeted by preferential ligation of activating Fc gamma receptors over inhibitory Fc gamma receptors. Preferential ligation of activating Fc gamma receptors but not inhibitory Fc gamma receptors can be accomplished by blocking inhibitory Fc gamma receptors and ligating activating Fc gamma receptors. In certain embodiments, activating Fc gamma receptors are targeted by inhibiting signaling through inhibitory Fc gamma receptors and activating signaling through activating Fc gamma receptors. In even other embodiments, activating Fc gamma receptors are targeted by blocking inhibitory Fc gamma receptors and activating signaling through activating Fc gamma receptors. In certain, more specific embodiments, activating Fc gamma receptor is CD32a and inhibitory Fc gamma receptor is CD32b.

In certain embodiments, the invention provides a method for promoting the maturation of an immature dendritic cell, wherein the method comprises: a) contacting the immature dendritic cell with an anti-CD32b antibody that blocks ligation of CD32b but not ligation of CD32a; and b) activating CD32a signaling in the immature dendritic cell. In certain, more specific embodiments, CD32a signaling is activated by contacting the immature dentritic cell with a CD32a agonist that binds with specificity to CD32a.

The invention further comprises a method for promoting the maturation of an immature dendritic cell, wherein the method comprises: a) inhibiting CD32b signaling in the immature dendritic cell; and activating CD32a signaling in the immature dendritic cell. In more specific embodiments, CD32b signaling is inhibited by contacting the immature dendritic cell with an anti-CD32b antibody that blocks ligation of CD32b but not ligation of CD32a. CD32b signaling can also be inhibited by contacting the immature dentritic cell with a CD32b antagonist that binds with specificity to CD32b. In certain embodiments, CD32a signaling is activated by contacting the immature dentritic cell with complexed IgG. CD32a signaling can be activated by contacting the immature dentritic cell with a CD32a agonist that binds with specificity to CD32a.

The method further provides a method for promoting the maturation of an immature dendritic cell, wherein the method comprises contacting the immature dendritic cell with an anti-CD32b antibody that blocks ligation of CD32b but not ligation of CD32a, wherein the anti-CD32b antibody is an IgG. In certain embodiments, the method further comprises activating CD32a signaling in the immature dendritic cell. CD32a signaling can be activated by contacting the immature dentritic cell with complexed IgG. CD32a signaling can be activated by contacting the immature dentritic cell with a CD32a agonist that binds with specificity to CD32a.

The invention provides a method for promoting the maturation of a population of immature dendritic cells, wherein the method comprises: a) enriching CD32a-expressing cells in the population; and b) activating CD32a signaling in cells of the population resulting from step (a). CD32a signaling can be activated by contacting the cells with complexed IgG. CD32a signaling can be activated by contacting the cells with a CD32a agonist that binds with specificity to CD32a. The method can further comprise inhibiting CD32b signaling in cells of the population resulting from step (a). CD32b signaling can be inhibited by contacting the cells with an anti-CD32b antibody that blocks ligation of CD32b but not ligation of CD32a. CD32b signaling can be inhibited by contacting the cells with a CD32a antagonist that binds with specificity to CD32b.

A method of the invention for promoting maturation of dendritic cells optionally further comprises contacting the immature dendritic cell with IL-6, IFN-gamma, PGE2 or combinations thereof. In a specific embodiment, the immature dendritic cell are contacted with IL-6, IFN-gamma, PGE2 or combinations thereof before contacting the immature dendritic cells with the agent that blocks ligation.

Enriching of CD32a expressing cells can be accomplished by contacting a population of immature dendritic cells with IL-6, IFN-gamma, PGE2 or combinations thereof. Enriching of CD32a expressing cells can be accomplished by FACS.

In a specific embodiment, the anti-CD32b antibody that blocks ligation of CD32b is monoclonal antibody 2B6.

The invention also provides a method for preventing the maturation of an immature dendritic cell, wherein the method comprises: a) contacting the immature dendritic cell with an anti-CD32a antibody that blocks ligation of CD32a but not ligation of CD32b; and b) activating CD32b signaling in the immature dendritic cell. In a specific embodiment, CD32b signaling is activated by contacting the immature dentritic cell with a CD32b agonist that binds with specificity to CD32b. The invention further provides a method for preventing the maturation of an immature dendritic cell, wherein the method comprises: inhibiting CD32a signaling in an immature dendritic cell; and b) activating CD32b signaling in the immature dendritic cell. CD32a signaling can be inhibited by contacting the immature dendritic cell with an anti-CD32a antibody that blocks ligation of CD32a but not ligation of CD32b. CD32a signaling can be inhibited by contacting the immature dentritic cell with a CD32a antagonist that binds with specificity to CD32a. CD32b signaling can be activated by contacting the immature dentritic cell with a CD32b agonist that binds with specificity to CD32b. CD32b signaling can be activated by contacting the immature dentritic cell with complexed IgG.

The invention further provides a method for inhibiting the maturation of an immature dendritic cell, wherein the method comprises contacting the immature dendritic cell with an anti-CD32a antibody that blocks ligation of CD32a but not ligation of CD32b, wherein the anti-CD32a antibody is an IgG. In certain, more specific embodiments, the method further comprises contacting the immature dendritic cell with soluble IgG monomer, TGF-beta, or IFN-alpha. In more specific embodiments, the contacting step with soluble IgG monomer, TGF-beta, or IFN-alpha is performed before contacting the cells with the anti-CD32a antibody. The anti-CD32a antibody can be monoclonal antibody IV.3.

The invention further provides a method for promoting the maturation of a population of immature dendritic cells, wherein the method comprises: a) contacting the population with IL-6, IFN-gamma, PGE2, or combinations thereof, to decrease expression of CD32b; and b) activating CD32a signaling in the cells of the population resulting from step (a). The invention also provides a method for promoting the maturation of a population of immature dendritic cells, wherein the method comprises: a) contacting the population with IFN-gamma, PGE2, LPS CD40L, or combinations thereof, to increase expression of CD23a; and b) activating CD32a signaling in the cells of the population resulting from step (a). Such methods may optionally further comprising inhibiting CD32b signaling in cells of the population resulting from step (a). CD32b signaling can be inhibited by contacting the cells with an anti-CD32b antibody that blocks ligation of CD32b but not ligation of CD32a. CD32b signaling can be inhibited by contacting the cells with a CD32b antagonist that binds with specificity to CD32b. CD32a signaling can be activated by contacting the cells with a CD32a agonist that binds with specificity to CD32a. CD32a signaling can be activated by contacting the cells with complexed IgG.

The invention provides a method for inhibiting the maturation of an immature dendritic cell, wherein the method comprises: a) contacting the immature dendritic cell with soluble IgG monomer, TGF-beta, IFN-alpha or combinations thereof; to increase expression of CD32b and b) activating CD32b signaling in the immature dendritic cell. Such method may optionally further comprise inhibiting CD32a signaling in the immature dendritic cell. CD32a signaling can be inhibited by contacting the immature dendritic cell with an anti-CD32a antibody that blocks ligation of CD32a but not ligation of CD32b. CD32a signaling can be inhibited by contacting the immature dentritic cell with a CD32a antagonist that binds with specificity to CD32a. CD32b signaling can be activated by contacting the immature dentritic cell with a CD32b agonist that binds with specificity to CD32b. CD32b signaling can be activated by contacting the immature dentritic cell with complexed IgG.

The invention also provides a method for promoting the maturation of an immature dendritic cell, wherein the method comprises contacting the immature dendritic cell with IgG that has a higher avidity for CD32a than for CD32b. In a specific embodiment, the immature dendritic cell has at least one allele of the H variant of human CD32a and the IgG is human IgG.

The invention further provides a method for inhibiting the maturation of an immature dendritic cell, wherein the method comprises contacting the immature dendritic cell with IgG that has a lower avidity for CD32a than for CD32b. The IgG can be IgG4 or IgG3. In certain specific embodiments, the immature dendritic cell can be homozygous for the R variant of human CD32a and the IgG can be IgG2.

CD32a signaling can for example be activated in a cell by contacting the cell with complexed IgG or with immobilized IgG. CD32b signaling can be activated in a cell by contacting the cell with complexed IgG or with immobilized IgG.

The present invention also provides methods for identifying molecules that block ligation of an Fc gamma receptor and for identifying molecules that inhibit signaling through an Fc gamma receptor.

The invention provides a method for identifying a molecule that blocks ligation of CD32a receptor more than ligation of CD32b receptor, said method comprising: a) contacting an immature dendritic cell that co-expresses CD32a and CD32b with a molecule; b) contacting the immature dendritic cell with complexed IgG or with immobilized IgG; c) determining the degree of maturation of the dendritic cell, wherein the molecule blocks ligation of CD32a receptor more than ligation of CD32b receptor if the dendritic cell is less matured in the presence of the molecule as compared to a control dendritic cell in the absence of the molecule.

The invention provides a method for identifying a molecule that blocks ligation of CD32a receptor more than ligation of CD32b receptor, said method comprising: a) contacting a population of immature dendritic cells that co-express CD32a and CD32b with a molecule; b) contacting the population with immobilized IgG or with complexed IgG; c) determining the amount of matured dendritic cells in the population of dendritic cells, wherein the molecule blocks ligation of CD32a receptor more than ligation of CD32b receptor if less dendritic cells in the population are matured in the presence of the molecule as compared to a control population of dendritic cells in the absence of the molecule.

The invention provides a method for identifying a molecule that blocks ligation of CD32b receptor more than ligation of CD32a receptor, said method comprising: a) contacting an immature dendritic cell that co-expresses CD32b and CD32a with a molecule; b) contacting the immature dendritic cell with immobilized IgG or with complexed IgG; c) determining the degree of maturation of the dendritic cell, wherein the molecule blocks ligation of CD32b receptor more than ligation of CD32a receptor if the dendritic cell is more matured in the presence of the molecule as compared to a control dendritic cell in the absence of the molecule.

The invention provides a method for identifying a molecule that blocks ligation of CD32b receptor more than ligation of CD32a receptor, said method comprising: a) contacting a population of immature dendritic cells that co-express CD32b and CD32a with a molecule; b) contacting the population with immobilized IgG or with complexed IgG; c) determining the amount of matured dendritic cells in the population, wherein the molecule blocks ligation of CD32b receptor more than ligation of CD32a receptor if more dendritic cells in the population of dendritic cells are matured in the presence of the molecule as compared to a control population of dendritic cells in the absence of the molecule.

The amount of matured dendritic cells can for example be determined by measuring the expression levels of CD83 and/or ILT3 in the population of dendritic cells. The amount of matured dendritic cells can also be determined by measuring the levels of cytokines secreted by the dendritic cells. The cytokine can be IL-8 or TNFalpha.

The invention provides a method for identifying a molecule that modifies the ratio of CD32a to CD32b expression on an immature dendritic cell, wherein the method comprises: a) contacting an immature dendritic cell that co-expresses CD32a and CD32b with a molecule; b) measuring the ratio of CD32a to CD32b expression on the dendritic cell, wherein the molecule modifies the ratio of CD32a to CD32b expression on a dendritic cell if the ratio of CD32a to CD32b expression on the dendritic cell as determined in step (b) is different from the ratio of CD32a to CD32b expression on a dendritic cell in the absence of the molecule.

The invention provides a method for producing a tolerogenic dendritic cell, wherein the method comprises: activating CD32b signaling in the immature dendritic cell. In certain embodiments, the method further comprises inhibiting CD32a signaling in an immature dendritic cell. CD32a signaling can be inhibited by contacting an immature dendritic cell with an anti-CD32a specific antibody that blocks ligation of CD32a. CD32a signaling can be inhibited by contacting the immature dendritic cell with a CD32a antagonist that binds with specificity to CD32a.

The invention provides a method for producing an antigen-specific tolerogenic dendritic cell, wherein the method comprises: a) contacting an immature dendritic cell with an anti-CD32a specific antibody that blocks ligation of CD32a; b) activating CD32b signaling in the immature dendritic cell; and c) targeting an antigen to the dendritic cell. An antigen can be targeted to a dendritic cell via conjugation of the antigen to a CD32b binding agent that binds CD32b and is internalized by the cell.

The methods of the invention for producing tolerogenic dendritic cells can be performed *ex vivo* or *in vivo.*

The invention further provides a method for producing an antigen-specific tolerogenic dendritic cell that induces tolerance against an immunogenic cell, wherein the method comprises: contacting an immature dendritic cell with an anti-CD32a specific antibody that blocks ligation of CD32a; activating CD32b signaling in the immature dendritic cell; and contacting the dendritic cell with a complex of the immunogenic cell and an antibody.

The methods of the invention for producing an antigen-specific tolerogenic dendritic cells can be performed *ex vivo* or *in vivo.*

The invention further provides a method for treating an autoimmune disease in a subject, wherein the method comprises: a) obtaining an immature dendritic cell from the subject; b) contacting the immature dendritic cell with an anti-CD32a specific antibody that blocks ligation of CD32a; c) activating CD32b signaling in the immature dendritic cell; and d) administering the resulting dendritic cell to the subject.

The invention further provides a method for treating an autoimmune disease in a subject, wherein the method comprises: a) obtaining an immature dendritic cell from the subject; b) contacting the immature dendritic cell with an anti-CD32a specific antibody that blocks ligation of CD32a; c) activating CD32b signaling in the immature dendritic cell;d) targeting the mature dendritic cell obtained from steps (a) to (c) with a self-antigen; and e) administering the mature dendritic cell to the subject. In a specific embodiment, the self-antigen is the antigen in the subject that is the target of the autoimmune disease.

The invention further provides a method for treating an autoimmune disease in a subject, wherein the method comprises: a) obtaining an immature dendritic cell from the subject; b) contacting the immature dendritic cell with an anti-CD32a specific antibody that blocks ligation of CD32a; c) activating CD32b signaling in the immature dendritic cell; d) contacting the mature dendritic cell resulting from steps (a) to (c) with tissue that is the target of the autoimmune disease in the subject, wherein the tissue is bound by an antibody; and e) administering the mature dendritic cell to the subject.

The autoimmune disease can be alopecia areata, ankylosing spondylitis, antiphospholipid syndrome, autoimmune Addison's disease, autoimmune diseases of the adrenal gland, autoimmune hemolytic anemia, autoimmune hepatitis, autoimmune oophoritis and orchitis, autoimmune thrombocytopenia, Behcet's disease, bullous pemphigoid, cardiomyopathy, celiac sprue-dermatitis, chronic fatigue immune dysfunction syndrome (CFIDS), chronic inflammatory demyelinating polyneuropathy, Churg-Strauss syndrome, cicatrical pemphigoid, CREST syndrome, cold agglutinin disease, Crohn's disease, discoid lupus, essential mixed cryoglobulinemia, fibromyalgia-fibromyositis, glomerulonephritis, Graves' disease, Guillain-Barre, Hashimoto's thyroiditis, idiopathic pulmonary fibrosis, idiopathic thrombocytopenia purpura (ITP), IgA neuropathy, juvenile arthritis, lichen planus, lupus erthematosus, Ménière's disease, mixed connective tissue disease, multiple sclerosis, type 1 or immune-mediated diabetes mellitus, myasthenia gravis, pemphigus vulgaris, pernicious anemia, polyarteritis nodosa, polychrondritis, polyglandular syndromes, polymyalgia rheumatica, polymyositis and dermatomyositis, primary agammaglobulinemia, primary biliary cirrhosis, psoriasis, psoriatic arthritis, Raynauld's phenomenon, Reiter's syndrome, Rheumatoid arthritis, sarcoidosis, scleroderma, Sjögren's syndrome, stiff-man syndrome, systemic lupus erythematosus, lupus erythematosus, takayasu arteritis, temporal arteristis/ giant cell arteritis, ulcerative colitis, uveitis, vasculitides such as dermatitis herpetiformis vasculitis, vitiligo, or Wegener's granulomatosis.

The invention further provides a method for treating graft-versus-host-disease in a host, wherein the method comprises: a) obtaining an immature dendritic cell from the host; b) contacting the immature dendritic cell with an anti-CD32a specific antibody that blocks ligation of CD32a; c) activating CD32b signaling in the immature dendritic cell; and d) administering the mature dendritic cell to the host.

The invention further provides a method for treating graft-versus-host-disease in a host, wherein the method comprises: a) obtaining an immature dendritic cell from the host; b) contacting the immature dendritic cell with an anti-CD32a specific antibody that blocks ligation of CD32a; c) activating CD32b signaling in the immature dendritic cell; d) targeting the mature dendritic cell that results from steps (a) to (c) with the antigen that is the target of the graft-versus-host-disease in the host; and e) administering the dendritic cell to the host.

The invention further provides a method for treating graft-versus-host-disease in a host, wherein the method comprises: a) obtaining an immature dendritic cell from the host; b) contacting the immature dendritic cell with an anti-CD32a specific antibody that blocks ligation of CD32a; c) activating CD32b signaling in the immature dendritic cell; d) contacting the mature dendritic cell that results from steps (a) to (c) with graft tissue bound to an antibody; and e) administering the dendritic cell to the subject.

The methods involving the generation of tolerogenic dendritic cells may optionally further comprise contacting the immature dendritic cell with soluble IgG monomer, TGF-beta, or IFN-alpha.

CD32b signaling can be activated in the cell by contacting a cell with complexed IgG or with immobilized IgG. CD32b signaling can be activated in a cell by contacting the cell with a CD32b agonist that binds with specificity to CD32b.

The invention further provides methods for producing mature dendritic cells suitable as adjuvant in a vaccine, wherein the method comprises: a) activating CD32a signaling in the immature dendritic cell; and b) formulating the mature dendritic cell obtained in step (a) into the vaccine. Such a method may optionally further comprise inhibiting CD32b signaling in an immature dendritic cell. CD32b signaling can be inhibited by contacting an immature dendritic cell with an anti-CD32b specific antibody that blocks ligation of CD32b. CD32b signaling can be inhibited by contacting the immature dendritic cell with a CD32b antagonist that binds with specificity to CD32b. The method may optionally further comprise contacting the immature dendritic cell with IL-6, IFN-gamma, or PGE2.

The invention also provides a method for producing a vaccine against an antigen, wherein the method comprises: a) contacting an immature dendritic cell with an anti-CD32b specific antibody that blocks ligation of CD32b; b) activating CD32a signaling in the immature dendritic cell; and c) targeting the antigen to the mature dendritic cell obtained in step (b). The vaccine can be an anti-cancer vaccine or a vaccine against an infectious disease. The method may optionally further comprise contacting the immature dendritic cell with IL-6, IFN-gamma, or PGE2.

The invention also provides a method for stimulating and/or expanding a T cell *ex vivo,* wherein the method comprises: a) contacting an immature dendritic cell with an anti-CD32b specific antibody that blocks ligation of CD32b; b) activating CD32a signaling in the immature dendritic cell; c) targeting an antigen to the mature dendritic cell obtained in steps (a) to (b), and d) contacting a T cell with the mature antigen-specific dendritic cell obtained in step (c). The method may optionally further comprise contacting the immature dendritic cell with IL-6, IFN-gamma, or PGE2. The T cell can be CD4 positive or CD8 positive. The T cells that are stimulated and/or expanded can then be administered to a subject.

An immature dendritic cell that can be used with the methods of the invention can for example be a monocyte-derived dendritic cell (moDCs).

The invention provides a method of providing activated T cells to a tumor site in a subject comprising a) inhibiting CD32b signaling in CD32b-expressing dendritic cells of the subject; b) activating CD32a signaling in CD32a-expressing dendritic cells of the subject; and c) promoting dendritic cell activation of the T cells, thereby providing activated T cells to a tumor site in the subject. CD32b signaling can be inhibited by contacting the CD32b-expressing dendritic cell with an anti-CD32b antibody that blocks ligation of CD32b but not ligation of CD32a. CD32b signaling can be inhibited by contacting the CD32b-expressing dendritic cell with a CD32b antagonist that binds with specificity to CD32b. CD32a signaling can be activated by contacting the CD32a expressing dentritic cell with complexed IgG. CD32a signaling can be activated by contacting the CD32a expressing dentritic cell with a CD32a agonist that binds with specificity to CD32a. Inhibition of CD32b signaling in CD32b-expressing dendritic cells and/or activation of CD32a signaling in CD32a-expressing dendritic cells, may be conducted ex-vivo. In certain embodiments, the method results in a humoral response in the subject.

The invention also provides kits comprising an anti-CD32b antibody that blocks ligation of CD32b but not ligation of CD32a; and IgG. Optionally, a kit of the invention may further comprise one or more of IL-6, IFN-gamma, and PGE2. The anti-CD32b antibody can be the monoclonal antibody 2B6.

The invention also provides kits comprising an anti-CD32a antibody that blocks ligation of CD32a but not ligation of CD32b; and IgG. Optionally, a kit of the invention may further comprise one or more of soluble IgG monomer, TGF-beta, and IFN-alpha. The anti-CD32a antibody can be monoclonal antibody IV.3.

The invention also provides kits comprising (i) IL-6, IFN-gamma, PGE2, or LPS and CD40L; and (ii) IgG. Optionally, a kit of the invention may further comprise means to immobilize IgG or means to complex IgG. A kit may further comprise means for isolating immature dendritic cells.

The invention also provides a method for treating in a subject a disorder that can be treated by lowering IL10 levels in the subject, wherein the method comprises administering to the subject antibodies that block ligation of CD32a. Such a disorder can be rheumatoid arthritis, systemic lupus erythematosus, HIV infection, organ transplant rejection, or burn-induced immunosuppression.

The invention also provides a method for treating in a subject a disorder that can be treated by lowering IL6 levels, wherein the method comprises administering to the subject antibodies that block ligation of CD32a. Such a disorder can be multiple myeloma, lymphoma, Waldenstrom's Macroglobulinemia, Castleman's Disease, rheumatoid arthritis, post-(bone marrow or whole organ) transplant lymphoproliferative disorder (PTLD), prostate cancer, autoimmunity, autoimmune hemolytic anemia (AIHA), amyloidosis, Crohn's disease, renal cell carcinoma, cancer-related cachexia/anorexia, cancer-related muscle atrophy, overwhelming infections/sepsis, herpes virus reactivation, or immunosuppression associated with alcohol consumption prior to burn injuires.

### 3.1 DEFINITIONS

As used herein, the term "block the ligation of an Fc gamma receptor" (e.g., block ligation of CD32a) and analogous terms refer to prevention of ligation between the Fc gamma receptor and IgG.

As used herein, the term "targeting of an Fc gamma receptor" (e.g., targeting of CD32a) and analogous terms refer to preferential or specific activation of the signaling events downstream of a particular Fc gamma receptor that are normally triggered by ligation of the Fc gamma receptor to IgG. If the signaling cascade downstream of a first Fc gamma receptor is activated preferentially, it can be activated at least 2 times, 5 times, 10 times, 25 times, 50 times, 100 times, 500 times, 1000 times, 10 000 times, 100 000 times or at least 1000 000 times higher levels than any signaling cascade downstream of any other Fc gamma receptor. If the signaling cascade downstream of a first Fc gamma receptor is activated preferentially, it can be activated at most 2 times, 5 times, 10 times, 25 times, 50 times, 100 times, 500 times, 1000 times, 10 000 times, 100 000 times or at most 1000 000 times higher levels than any signaling cascade downstream of any other Fc gamma receptor. Targeting of a first Fc gamma receptor in a cell can for example be accomplished by blocking other Fc gamma receptors on the cell and activation of the signaling cascade downstream of the first Fc gamma receptor, e.g., by ligating the Fc gamma receptor to complexed IgG. Targeting of a first Fc gamma receptor in a cell can also be accomplished by inhibiting the signaling downstream of other Fc gamma receptors on the cell and activation of the signaling cascade downstream of the first Fc gamma receptor, e.g., by ligating the Fc gamma receptor to complexed IgG.

As used herein, the term "specifically binds to an Fc gamma receptor" (e.g., CD32a or CD32b) and analogous terms refer to antibodies or fragments thereof that specifically bind to a particular Fc gamma receptor or a fragment thereof and do not specifically bind to other Fc receptors, in particular to other Fc gamma receptors. Further it is understood to one skilled in the art, that an antibody that specifically binds to a particular Fc gamma receptor, may bind through the variable domain or the constant domain of the antibody. If the antibody that specifically binds to a particular Fc gamma receptor binds through its variable domain, it is understood to one skilled in the art that it is not aggregated, i.e., is monomeric. In preferred embodiments, an antibody that binds to a particular Fc gamma receptor binds to the native form of the Fc gamma receptor. Preferably, antibodies or fragments that specifically bind to a particular Fc gamma receptor or a fragment thereof do not cross-react with other antigens. Antibodies or fragments that specifically bind to a particular Fc gamma receptor can be identified, for example, by immunoassays, BIAcore, or other techniques known to those of skill in the art.

As used herein, the term "preferentially binds to an Fc gamma receptor" (e.g., CD32a or CD32b) and analogous terms refer to antibodies that bind to a particular Fc gamma receptor with higher affinity than to other peptides or polypeptides as determined by, e.g., immunoassays, BIAcore, or other assays known in the art. *See, e.g.,* Paul, ed., 1989, Fundamental Immunology Second Edition, Raven Press, New York at pages 332-336 for a discussion regarding antibody specificity. In certain embodiments, an antibody that preferentially binds a first Fc gamma receptor binds the first Fc gamma receptor with at least 2 times, 5 times, 10 times, 25 times, 50 times, 100 times, 500 times, 1000 times, 10 000 times, 100 000 times or at least 1000 000 times higher affinity than any other Fc gamma receptor. In certain embodiments, an antibody that preferentially binds a first Fc gamma receptor binds the first Fc gamma receptor with at most 2 times, 5 times, 10 times, 25 times, 50 times, 100 times, 500 times, 1000 times, 10 000 times, 100 000 times or at most 1000 000 times higher affinity than any other Fc gamma receptor.

As used herein, the term "native Fc gamma receptor" (e.g., native CD32a or native 32b) refers to Fc gamma receptor which is endogenously expressed and present on the surface of a cell. In some embodiments, native Fc gamma receptor encompasses a protein that is recombinantly expressed in a mammalian cell. Preferably, the native Fc gamma receptor is not expressed in a bacterial cell, *i.e., E. coli.* Most preferably the native Fc gamma receptor is not denatured, i.e., it is in its biologically active conformation.

As used herein, the terms "antibody" and "antibodies" refer to monoclonal antibodies, multispecific antibodies, human antibodies, humanized antibodies, synthetic antibodies, chimeric antibodies, camelized antibodies, single-chain Fvs (scFv), single chain antibodies, Fab fragments, F(ab') fragments, disulfide-linked Fvs (sdFv), intrabodies, and anti-idiotypic (anti-Id) antibodies (including, e.g., anti-Id and anti-anti-Id antibodies to antibodies of the invention), and epitope-binding fragments of any of the above. In particular, antibodies include immunoglobulin molecules and immunologically active fragments of immunoglobulin molecules, *i.e.,* molecules that contain an antigen binding site. Immunoglobulin molecules can be of any type (e.g., IgG, IgE, IgM, IgD, IgA and IgY), class (e.g., IgG₁, IgG₂, IgG₃, IgG₄, IgA₁ and IgA₂) or subclass.

As used herein, the term "derivative" in the context of polypeptides or proteins refers to a polypeptide or protein that comprises an amino acid sequence which has been altered by the introduction of amino acid residue substitutions, deletions or additions. The term "derivative" as used herein also refers to a polypeptide or protein which has been modified, i.e, by the covalent attachment of any type of molecule to the polypeptide or protein. For example, but not by way of limitation, an antibody may be modified, e.g., by glycosylation, acetylation, pegylation, phosphorylation, amidation, derivatization by known protecting/blocking groups, proteolytic cleavage, linkage to a cellular ligand or other protein, *etc.* A derivative polypeptide or protein may be produced by chemical modifications using techniques known to those of skill in the art, including, but not limited to specific chemical cleavage, acetylation, formylation, metabolic synthesis of tunicamycin, *etc*. Further, a derivative polypeptide or protein derivative possesses a similar or identical function as the polypeptide or protein from which it was derived.

As used herein, the terms "disorder" and "disease" are used interchangeably to refer to a condition in a subject. In particular, the term "autoimmune disease" is used interchangeably with the term "autoimmune disorder" to refer to a condition in a subject characterized by cellular, tissue and/or organ injury caused by an immunologic reaction of the subject to its own cells, tissues and/or organs. The term "inflammatory disease" is used interchangeably with the term "inflammatory disorder" to refer to a condition in a subject characterized by inflammation, preferably chronic inflammation. Autoimmune disorders may or may not be associated with inflammation. Moreover, inflammation may or may not be caused by an autoimmune disorder. Thus, certain disorders may be characterized as both autoimmune and inflammatory disorders.

As used herein, the term "cancer" refers to a neoplasm or tumor resulting from abnormal uncontrolled growth of cells. As used herein, cancer explicitly includes, leukemias and lymphomas. The term "cancer" refers to a disease involving cells that have the potential to metastasize to distal sites and exhibit phenotypic traits that differ from those of non-cancer cells, for example, formation of colonies in a three-dimensional substrate such as soft agar or the formation of tubular networks or weblike matrices in a three-dimensional basement membrane or extracellular matrix preparation. Non-cancer cells do not form colonies in soft agar and form distinct sphere-like structures in three-dimensional basement membrane or extracellular matrix preparations. Cancer cells acquire a characteristic set of functional capabilities during their development, albeit through various mechanisms. Such capabilities include evading apoptosis, self sufficiency in growth signals, insensitivity to anti-growth signals, tissue invasion/metastasis, limitless explicative potential, and sustained angiogenesis. The term "cancer cell" is meant to encompass both pre-malignant and malignant cancer cells. In some embodiments, cancer refers to a benign tumor, which has remained localized. In other embodiments, cancer refers to a malignant tumor, which has invaded and destroyed neighboring body structures and spread to distant sites. In yet other embodiments, the cancer is associated with a specific cancer antigen.

As used herein, the term "immunomodulatory agent" and variations thereof including, but not limited to, immunomodulatory agents, refer to an agent that modulates a host's immune system. In certain embodiments, an immunomodulatory agent is an immunosuppressant agent. In certain other embodiments, an immunomodulatory agent is an immunostimulatory agent. Immunomodatory agents include, but are not limited to, small molecules, peptides, polypeptides, fusion proteins, antibodies, inorganic molecules, mimetic agents, and organic molecules.

As used herein, the term "epitope" refers to a fragment of a polypeptide or protein having antigenic or immunogenic activity in an animal, preferably in a mammal, and most preferably in a human. An epitope having immunogenic activity is a fragment of a polypeptide or protein that elicits an antibody response in an animal. An epitope having antigenic activity is a fragment of a polypeptide or protein to which an antibody immunospecifically binds as determined by any method well-known to one of skill in the art, for example by immunoassays. Antigenic epitopes need not necessarily be immunogenic.

As used herein, the term "fragment" refers to a peptide or polypeptide comprising an amino acid sequence of at least 5 contiguous amino acid residues, at least 10 contiguous amino acid residues, at least 15 contiguous amino acid residues, at least 20 contiguous amino acid residues, at least 25 contiguous amino acid residues, at least 40 contiguous amino acid residues, at least 50 contiguous amino acid residues, at least 60 contiguous amino residues, at least 70 contiguous amino acid residues, at least contiguous 80 amino acid residues, at least contiguous 90 amino acid residues, at least contiguous 100 amino acid residues, at least contiguous 125 amino acid residues, at least 150 contiguous amino acid residues, at least contiguous 175 amino acid residues, at least contiguous 200 amino acid residues, or at least contiguous 250 amino acid residues of the amino acid sequence of another polypeptide. In a specific embodiment, a fragment of a polypeptide retains at least one function of the polypeptide. Preferably, antibody fragments are epitope binding fragments.

As used herein, the term "humanized antibody" refers to forms of non-human (e.g., murine) antibodies that are chimeric antibodies which contain minimal sequence derived from non-human immunoglobulin. For the most part, humanized antibodies are human immunoglobulins (recipient antibody) in which hypervariable region residues of the recipient are replaced by hypervariable region residues from a non-human species (donor antibody) such as mouse, rat, rabbit or non-human primate having the desired specificity, affinity, and capacity. In some instances, Framework Region (FR) residues of the human immunoglobulin are replaced by corresponding non-human residues. Furthermore, humanized antibodies may comprise residues which are not found in the recipient antibody or in the donor antibody. These modifications are made to further refine antibody performance. In general, the humanized antibody will comprise substantially all of at least one, and typically two, variable domains, in which all or substantially all of the hypervariable regions correspond to those of a non-human immunoglobulin and all or substantially all of the FRs are those of a human immunoglobulin sequence. The humanized antibody may comprise at least a portion of an immunoglobulin constant region (Fc) that has been altered by the introduction of amino acid residue substitutions, deletions or additions (i.e., mutations). In some embodiments, a humanized antibody is a derivative. Such a humanized antibody comprises amino acid residue substitutions, deletions or additions in one or more non-human CDRs. The humanized antibody derivative may have substantially the same binding, better binding, or worse binding when compared to a non-derivative humanized antibody. In specific embodiments, one, two, three, four, or five amino acid residues of the CDR have been substituted, deleted or added (i.e., mutated). For further details in humanizing antibodies, see European Patent Nos. EP 239,400, EP 592,106, and EP 519,596; International Publication Nos. WO 91/09967 and WO 93/17105; U.S. Patent Nos. 5,225,539, 5,530,101, 5,565,332, 5,585,089, 5,766,886, and 6,407,213; and Padlan, 1991, *Molecular Immunology* 28(4/5):489-498; Studnicka et al., 1994, *Protein Engineering* 7(6):805-814; Roguska et al., 1994, *PNAS* 91:969-973; Tan et al., 2002, *J. Immunol.* 169:1119-25; Caldas et al., 2000, *Protein Eng.* 13:353-60; Morea et al., 2000, *Methods* 20:267-79; Baca et al., 1997, *J. Biol. Chem.* 272:10678-84; Roguska et al., 1996, *Protein Eng.* 9:895-904; Couto et al., 1995, *Cancer Res.* 55 (23 Supp):5973s-5977s; Couto et al., 1995, *Cancer Res.* 55:1717-22; Sandhu, 1994, *Gene* 150:409-10; Pedersen et al., 1994, *J*. *Mol. Biol.* 235:959-73; Jones et al., 1986, *Nature* 321:522-525; Reichmann et al., 1988, *Nature* 332:323-329; and Presta, 1992, *Curr. Op. Struct. Biol.* 2:593-596.

As used herein, the term "hypervariable region" refers to the amino acid residues of an antibody which are responsible for antigen binding. The hypervariable region comprises amino acid residues from a "Complementarity Determining Region" or "CDR" (i.e., residues 24-34 (L1), 50-56 (L2) and 89-97 (L3) in the light chain variable domain and 31-35 (H1), 50-65 (H2) and 95-102 (H3) in the heavy chain variable domain; Kabat et al., *Sequences of Proteins of Immunological Interest,* 5th Ed. Public Health Service, National Institutes of Health, Bethesda, MD. (1991)) and/or those residues from a "hypervariable loop" (i.e., residues 26-32 (L1), 50-52 (L2) and 91-96 (L3) in the light chain variable domain and 26-32 (H1), 53-55 (H2) and 96-101 (H3) in the heavy chain variable domain; Chothia and Lesk, 1987, *J. Mol. Biol.* 196:901-917). CDR residues for Eph099B-208.261 and Eph099B-233.152 are listed in Table 1. "Framework Region" or "FR" residues are those variable domain residues other than the hypervariable region residues as herein defined.

As used herein, the terms "single-chain Fv" or "scFv" refer to antibody fragments comprise the VH and VL domains of antibody, wherein these domains are present in a single polypeptide chain. Generally, the Fv polypeptide further comprises a polypeptide linker between the VH and VL domains which enables the scFv to form the desired structure for antigen binding. For a review of sFv see Pluckthun in *The Pharmacology of Monoclonal Antibodies,* vol. 113, Rosenburg and Moore eds. Springer-Verlag, New York, pp. 269-315 (1994). In specific embodiments, scFvs include bispecific scFvs and humanized scFvs.

As used herein, the terms "nucleic acids" and "nucleotide sequences" include DNA molecules (e.g., cDNA or genomic DNA), RNA molecules (e.g., mRNA), combinations of DNA and RNA molecules or hybrid DNA/RNA molecules, and analogs of DNA or RNA molecules. Such analogs can be generated using, for example, nucleotide analogs, which include, but are not limited to, inosine or tritylated bases. Such analogs can also comprise DNA or RNA molecules comprising modified backbones that lend beneficial attributes to the molecules such as, for example, nuclease resistance or an increased ability to cross cellular membranes. The nucleic acids or nucleotide sequences can be single-stranded, double-stranded, may contain both single-stranded and double-stranded portions, and may contain triple-stranded portions, but preferably is double-stranded DNA.

As used herein, the terms "subject" and "patient" are used interchangeably. As used herein, a subject is preferably a mammal such as a non-primate (e.g., cows, pigs, horses, cats, dogs, rats etc.) and a primate (e.g., monkey and human), most preferably a human.

As used herein, the terms "treat," "treating" and "treatment" refer to the eradication, reduction or amelioration of symptoms of a disease or disorder related to the loss of regulation in the Fc receptor signaling pathway or to enhance the therapeutic efficacy of another therapy, e.g., a therapeutic antibody, vaccine therapy. In some embodiments,, treatment refers to the eradication, removal, modification, or control of primary, regional, or metastatic cancer tissue that results from the administration of one or more therapeutic agents. In certain embodiments, such terms refer to the minimizing or delaying the spread of cancer resulting from the administration of one or more therapeutic agents to a subject with such a disease.

As used herein, the phrase "side effects" encompasses unwanted and adverse effects of a prophylactic or therapeutic agent. Adverse effects are always unwanted, but unwanted effects are not necessarily adverse. An adverse effect from a prophylactic or therapeutic agent might be harmful or uncomfortable or risky. Side effects from chemotherapy include, but are not limited to, gastrointestinal toxicity such as, but not limited to, early and late-forming diarrhea and flatulence, nausea, vomiting, anorexia, leukopenia, anemia, neutropenia, asthenia, abdominal cramping, fever, pain, loss of body weight, dehydration, alopecia, dyspnea, insomnia, dizziness, mucositis, xerostomia, and kidney failure, as well as constipation, nerve and muscle effects, temporary or permanent damage to kidneys and bladder, flu-like symptoms, fluid retention, and temporary or permanent infertility. Side effects from radiation therapy include but are not limited to fatigue, dry mouth, and loss of appetite. Side effects from biological therapies/immunotherapies include but are not limited to rashes or swellings at the site of administration, flu-like symptoms such as fever, chills and fatigue, digestive tract problems and allergic reactions. Side effects from hormonal therapies include but are not limited to nausea, fertility problems, depression, loss of appetite, eye problems, headache, and weight fluctuation. Additional undesired effects typically experienced by patients are numerous and known in the art, *see, e.g.,* the *Physicians' Desk Reference* (56^{th} ed., 2002), which is incorporated herein by reference in its entirety.

As used herein, a "therapeutically effective amount" refers to to the amount of therapeutic agent sufficient to delay or minimize the onset of disease, e.g., delay or minimize the spread of cancer or to reduce the symptoms of an autoimmune disease. A therapeutically effective amount may also refer to the amount of the therapeutic agent that provides a therapeutic benefit in the treatment or management of a disease. Further, a therapeutically effective amount with respect to a therapeutic agent of the invention means that amount of therapeutic agent alone, or in combination with other therapies, that provides a therapeutic benefit in the treatment or management of a disease, e.g., sufficient to enhance the therapeutic efficacy of a therapeutic antibody sufficient to treat or manage a disease.

As used herein, the terms "manage," "managing" and "management" refer to the beneficial effects that a subject derives from administration of a prophylactic or therapeutic agent, which does not result in a cure of the disease. In certain embodiments, a subject is administered one or more prophylactic or therapeutic agents to "manage" a disease so as to prevent the progression or worsening of the disease.

As used herein, the terms "prevent", "preventing" and "prevention" refer to the prevention of the recurrence or onset of one or more symptoms of a disorder in a subject resulting from the administration of a prophylactic or therapeutic agent.

As used herein, the term "in combination" refers to the use of more than one prophylactic and/or therapeutic agents. The use of the term "in combination" does not restrict the order in which prophylactic and/or therapeutic agents are administered to a subject with a disorder, e.g., hyperproliferative cell disorder, especially cancer. A first prophylactic or therapeutic agent can be administered prior to (e.g., 1 minute, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (e.g., 1 minute, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second prophylactic or therapeutic agent to a subject which had, has, or is susceptible to a disorder. The prophylactic or therapeutic agents are administered to a subject in a sequence and within a time interval such that the agent of the invention can act together with the other agent to provide an increased benefit than if they were administered otherwise. Any additional prophylactic or therapeutic agent can be administered in any order with the other additional prophylactic or therapeutic agents.

### 4. BRIEF DESCRIPTION OF THE DRAWINGS

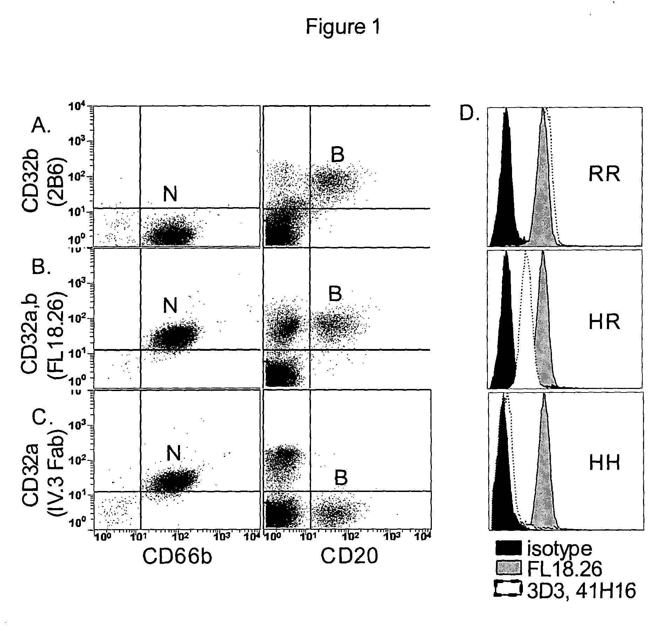

**Figure 1** indicates that 2B6 specifically detects an extracellular domain of CD32b. In Figure 1A, MAb 2B6 is shown to detect CD32b on B cells (B), but not CD32a on neutrophils. MAb FL18.26 detects CD32a or CD32b, and stains neutrophils as well as B cells. In contrast, MAb IV.3 (Fab) detects CD32a on neutrophils, but not CD32b on B cells. In Figure 1B, some MAbs that are non-specific for the a or b isoforms are able to distinguish between the polymorphic variants of CD32a that derive from the a single nucleotide polymorphism at amino acid 131. MAb FL18.26, binds both the R131 and H131 subtypes of CD32a, whereas MAb 3D3, recognizes only the R131 subtype [Gosselin, 1990 #16523]. Samples from RR individuals are stained equally by the two clones, whereas HH samples are only detected by clone FL18.26. Samples from HR individuals display an intermediate pattern.

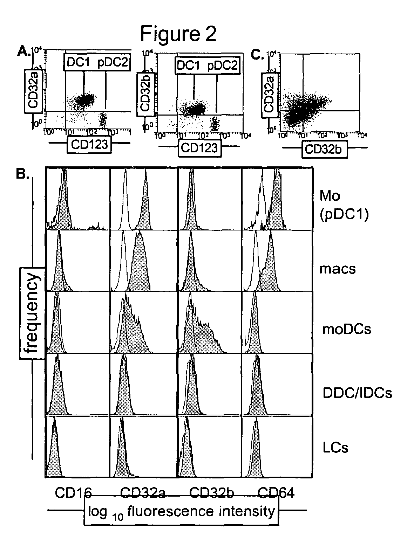

**Figure 2** depicts monocytes, DC1, and cultured MoDCs and macrophages that all express a range of FcgRs, whereas LCs, DDC-IDCs, and pDC2 lack detectable surface expression of all FcgRs. In Figure 2A, freshly isolated PBMCs were labeled with a cocktail of fluorochrome-conjugated MAbs and gated for HLA-DR bright cells that were lineage marker- negative. CD32a (left) and CD32b (right) are both expressed by CD123 ^{moderate} DC1, though CD123^{bright} pDC2 express neither. In Figure 2B, freshly isolated CD 14⁺ monocyte precursors to DC1 (pDC1), and macrophages (macs), cultured from plastic-adherent monocytes in 10% PHS-RPMI without additional cytokines, express similar FcgR profiles. MoDCs, LCs, and DDC-IDCs were studied as immature DCs, confirmed by absence of surface CD83, despite the presence of intracellular CD83 indicating commitment to DC-specific differentiation. Open histograms correspond to isotype controls, and filled histograms represent staining of the indicated FcgR. In Figure 2C, CD32a and CD32b were each expressed on 40-50% of moDCs, most often on the same sub-population of DCs.

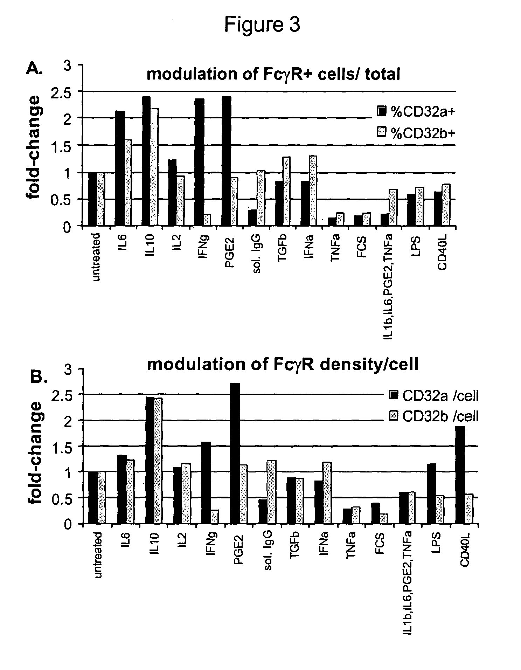

**Figure 3** depicts various stimuli that modulate the balanced expression of CD32a and CD32b on immature moDCs. In Figure 3A, exposure of immature moDCs indicated reagents affected CD32a and CD32b expression as shown. All cultures used 1% NHS, except those in the bottom panel that used FCS.

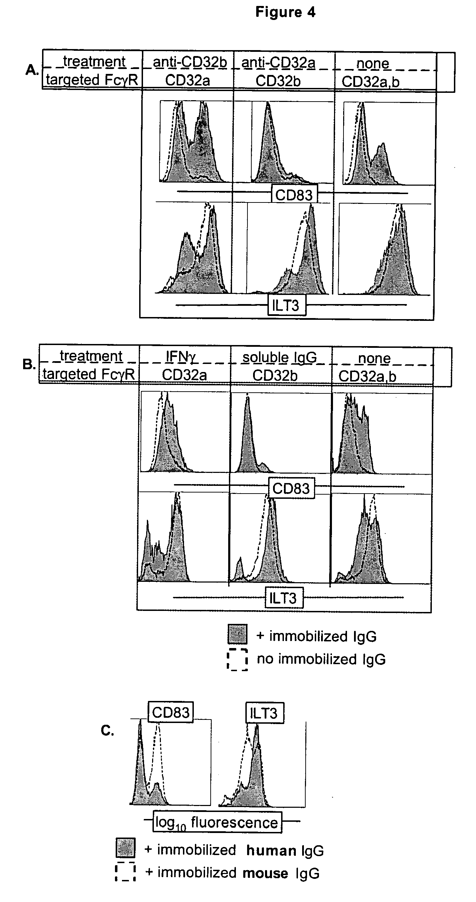

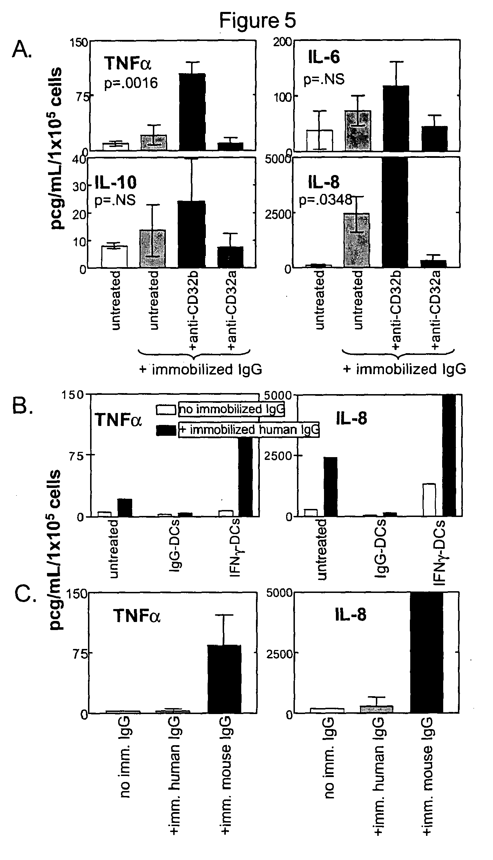

**Figure 4** indicates that ligation of CD32a or CD32b on immature moDCs has the opposing effects on the maturation phenotype. CD32a or CD32b were targeted by first incubating moDCs with blocking antibodies against CD32b or CD32a, respectively. CD32a and CD32b were targeted simultaneously by incubating moDCs with isotype controls. DCs were then re-cultured in complete medium with GMCSF and IL4 at 37° C in 96-well plates pre-coated with human IgG (filled histograms) or on untreated plates(open histograms). Cells were harvested at 24 or 48 hours and assessed DC phenotype by flow cytometry. Selective ligation of CD32a matured DCs as evidenced by increased expression of CD83, with coincident down-regulation of the inhibitory molecule ILT3. Co-ligation of CD32b limited CD32a- induced DC maturation, though selective ligation of CD32b did not significantly affect DC maturation. Figure 4A depicts one representative sample from 6 individuals bearing the HH or HR subtypes of CD32a. Figure 4B depicts the functional significance ofIFNg- and soluble IgG- mediated shifts in the balance between CD32a and CD32b. IFNg-treated moDCs, which predominantly expressed CD32a, and soluble IgG-treated moDCs, which favored expression of CD32b, were co-cultured with (filled histograms) or without (open histograms) immobilized human IgG. Cells were harvested at 24-48 hours and assessed phenotype. Almost all IFNg-treated cells were matured after co-culture with immobilized IgG, as shown by increased surface expression of CD83 and down-regulation of ILT-3. In contrast, the lack of upregulation of CD83 and the increased expression of ILT-3 suggest that immobilized IgG predominantly ligated CD32b on soluble IgG-treated cells. IN Figure 4C, common polymorphisms of CD32a are shown to effect a functional shift in the balance between CD32a and CD32b. In contrast to results obtained from CD32aHH or CD32aHR samples, CD32aRR samples were not matured to the same extent after co-culture with immobilized human IgG (closed histograms). Using immobilized mouse IgG as ligand (open histograms), however, which ligates CD32a and not CD32b in these individuals, led to maturation that was similar to conditions specifically targeting CD32a on CD32aHH or CD32aHR samples (Figure 5A, filled histograms).

**Figure 5** indicates that co-ligation of CD32b limits CD32a-mediated cytokine release. CD32a, CD32b or both were targeted on immature moDCs using blocking mAbs (Figure 5A) or IFNg and soluble IgG- mediated shifts in the expression of CD32a and CD32b (Figure 5B) or differences in CD32a and CD32b avidity for mouse and human IgG (Figure 5C) as described previously. After 2 days, cell free supernatants were harvested and frozen for batched processing. Supernatants were thawed once and cytokines were measured using a flow cytometry-based multiplexed bead assay. Targeting CD32a led to the greatest increase in inflammatory TH2 cytokines (Figure 5A). Co-targeting CD32b limited this effect, and targeting CD32b alone was not significantly different from untargeted moDCs. IFNg-treated moDCs released cytokines in proportion to CD32A-targeted DCs whereas soluble IgG-treated moDCs were similar to CD32b-targeted DCs in not significantly increasing cytokine production (Figure 5B). Cytokine release from RR samples was much greater after co-culture with immobilized mouse IgG then with immobilized human IgG (Figure 5C).

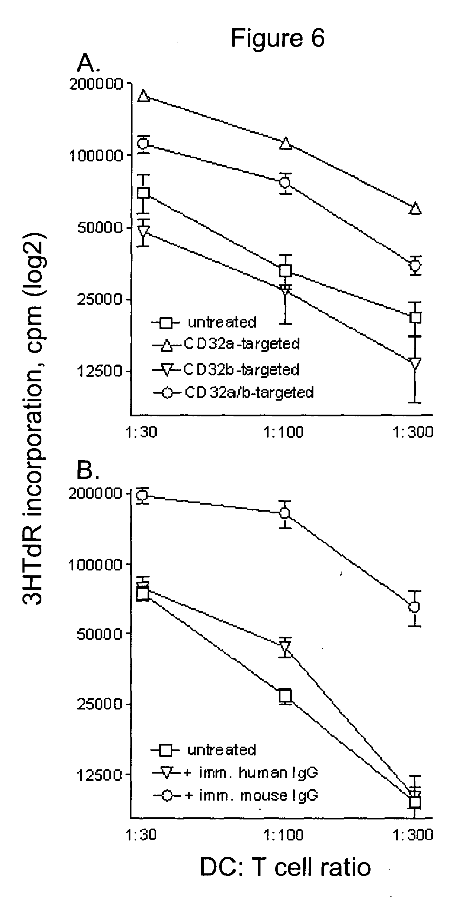

**Figure 6** indicates that targeting CD32a or CD32b affects DC allo-stimulatory capacity in an allogeneic mixed leukocyte reaction (MLR). CD32a, CD32b or both were targeted on immature moDCs as described previously. After 2 days, cells were harvested and washed, then re-cultured moDCs without additional cytokines with 10⁵ allogeneic T cells in a round-bottom 96-well plates. DC doses ranged from 3000 to 300 cells per well, yielding starting DC-T ratios from 1:30 to 1:300. [3H]TdR uptake by proliferating allogeneic T cells over the last 12 h of a 4-5 day culture was measured as an index of DC immunogenicity. The averaged triplicate means ± SEM for [3H]TdR incorporation by T cells stimulated by respective DCs are depicted logarithmically (log2) against the γ-axis. CD32a-targeted DCs were the most potent stimulators. Co-targeting CD32b limited this effect. Targeting CD32b alone did not enhance stimulatory capacity over untreated cells.

### 5. DESCRIPTION OF THE PREFERRED EMBODIMENTS

### 5.1 MODULATION OF MATURATION OF DENDRITIC CELLS

The present invention provides methods for modulating the differentiation of dendritic cells. More specifically, the present invention provides methods for modulating the maturation of dendritic cells. In particular, the invention provides methods for (a) promoting the maturation of dendritic cells thereby producing mature dendritic cells, and (b) preventing the maturation of dendritic cells thereby preventing maturation and/or inducing phenotypic changes resulting in the production of tolerogenic dendritic cells. Without being bound by theory, immature dendritic cells, such as, but not limited to, monocyte derived dendritic cells or myeloid blood dendritic cells, coexpress activating and inhibitory Fc gamma receptors. The present inventors have found that targeting activating Fc gamma receptors, *i.e.,* preferentially or specifically activating the signaling pathway(s) downstream of activating Fc gamma receptors, results in the maturation of dendritic cells. Targeting inhibitory Fc gamma receptors, i.e., preferentially or specifically activating the signaling pathway(s) downstream of inhibitory Fc gamma receptors, prevents maturation and results in phenotypic changes related to tolerogenic dendritic cells.

Targeting of activating Fc gamma receptors can be achieved by different approaches. In certain embodiments of the invention, activating Fc gamma receptors are targeted by preferential ligation of activating Fc gamma receptors over inhibitory Fc gamma receptors. Preferential ligation of activating Fc gamma receptors but not inhibitory Fc gamma receptors can be accomplished by blocking inhibitory Fc gamma receptors and ligating activating Fc gamma receptors. In certain embodiments, activating Fc gamma receptors are targeted by inhibiting signaling through inhibitory Fc gamma receptors and activating signaling through activating Fc gamma receptors. In even other embodiments, activating Fc gamma receptors are targeted by blocking inhibitory Fc gamma receptors and activating signaling through activating Fc gamma receptors. In certain, more specific embodiments, activating Fc gamma receptor is CD32a and inhibitory Fc gamma receptor is CD32b.

Signaling events downstream of an Fc gamma receptor can be activated using an agonist of the Fc gamma receptor; signaling events downstream of an Fc gamma receptor can be inhibited using an antagonist of the Fc gamma receptor. Thus, signaling events downstream of an activating Fc gamma receptor, such as, e.g., CD32a, can be activated using an agonist of the activating Fc gamma receptor, such as, e.g., an agonist of CD32a. Signaling events downstream of an activating Fc gamma receptor, such as, e.g., CD32a, can be inhibited using an antagonist of the activating Fc gamma receptor, such as, e.g., an antagonist of CD32a. Signaling events downstream of an inhibitory Fc gamma receptor, such as, e.g., CD32b, can be activated using an agonist of the inhibitory Fc gamma receptor, such as, e.g., an agonist of CD32b. Signaling events downstream of an inhibitory Fc gamma receptor, such as, e.g., CD32b, can be inhibited using an antagonist of the inhibitory Fc gamma receptor, such as, e.g., an antagonist of CD32b.

Targeting of inhibitory Fc gamma receptors can be achieved by different approaches. In certain embodiments of the invention, inhibitory Fc gamma receptors are targeted by preferential ligation of inhibitory Fc gamma receptors over activating Fc gamma receptors. Preferential ligation of inhibitory Fc gamma receptors but not activating Fc gamma receptors can be accomplished by blocking activating Fc gamma receptors and ligating inhibitory Fc gamma receptors. In certain embodiments, inhibitory Fc gamma receptors are targeted by inhibiting signaling through activating Fc gamma receptors and activating signaling through inhibitory Fc gamma receptors. In even other embodiments, inhibitory Fc gamma receptors are targeted by blocking activating Fc gamma receptors and activating signaling through inhibitory Fc gamma receptors. In certain, more specific embodiments, activating Fc gamma receptor is CD32a and inhibitory Fc gamma receptor is CD32b.

In addition to targeting either activating Fc gamma receptors or inhibitory Fc gamma receptors specifically, the relative expression levels of activating Fc gamma receptors to inhibitory Fc gamma receptors can be modulated to affect the maturation of dendritic cells. Thus, if dendritic cell maturation is to be activated, the immature dendritic cells are contacted with an agent that increases the level of activating Fc gamma receptor relative to the inhibitory Fc gamma receptor and activating Fc gamma receptors are targeted to activate maturation of the dendritic cells. Conversely, if dendritic cell maturation is to be inhibited, the immature dendritic cells are contacted with an agent that increases the level of inhibitory Fc gamma receptor relative to the level of activating Fc gamma receptor and inhibitory Fc gamma receptors are targeted to prevent maturation of the dendritic cells.

Dendritic cells that can be used as starting material for the methods of the invention include monocyte-derived dendritic cells (moDCs), myeloid blood dendritic cells (DC1), dermal-interstitial DCs and Langerhans'cells. Other dendritic cells that can be used with the methods of the invention include, but are not limited to, plasmacytoid dendritic cells and IL-16 dendritic cells (TPO dendritic cells; see Della Bella, S. et al. 2004, Blood 104(13):4020-4028).

Targeting of activating Fc gamma receptors versus inhibitory Fc gamma receptors can be a preferential process. For example, if targeting is accomplished by preferential ligation of activating Fc gamma receptors, the ratio of ligated activating Fc gamma receptors over the total number of activating Fc gamma receptors is higher than the ratio of ligated inhibitory Fc gamma receptors over the total number of inhibitory Fc gamma receptors. Similarly, preferential ligation of inhibitory Fc gamma receptors versus activating Fc gamma receptors results in a ratio of ligated inhibitory Fc gamma receptors over total number of inhibitory Fc gamma receptors that is higher than the ratio of ligated activating Fc gamma receptors over the total number of activating Fc gamma receptors.

The dendritic cells that are produced by promoting the maturation can be used, *inter alia,* as adjuvants in vaccines or to educate T cells *ex vivo* or *in vivo.* The tolerogenic dendritic cells that are produced by preventing the maturation can be used, *inter alia,* to educate T cells *ex vivo* or *in vivo* for the treatment of auto-immune diseases, transplant rejection, or graft-versus-host diseases associated with bone marrow transplantation or other medical conditions (e.g. transfusion-related graft-versus-host disease).

In certain embodiments, the present invention is, among others, directed at (i) targeting activating Fc gamma receptors specifically and thereby promoting dendritic cell maturation; and (ii) targeting inhibitory Fc gamma receptors specifically and thereby promoting the generation of tolerogenic dendritic cells.

In general, targeting of activating Fc gamma receptors involves two steps: a) blocking of the inhibitory Fc gamma receptors or inhibition of the signaling events downstream of inhibitory Fc gamma receptors; and b) activation of signaling events downstream of activating Fc gamma receptors, e.g., by ligating the activating Fc gamma receptors with complexed IgG or with immobilized IgG. Without being bound by a particular mechanism, immobilization of IgG simulates complexed or bound IgG. Without being bound by theory, if activating Fc gamma receptors are targeted by blocking inhibitory Fc gamma receptors and ligation of activating Fc gamma receptors, activating Fc gamma receptors are targeted because blocking of the inhibitory Fc gamma receptors prevents ligation of inhibitory Fc gamma receptors with IgG.

In general, targeting of inhibitory Fc gamma receptors involves two steps: a) blocking of the activating Fc gamma receptors or inhibition of the signaling events downstream of activating Fc gamma receptors; and b) activation of signaling events downstream of inhibitory Fc gamma receptors, e.g., by ligating the inhibitory Fc gamma receptors with complexed IgG or with immobilized IgG. Without being bound by a particular mechanism, immobilization of IgG simulates complexed or bound IgG. Without being bound by theory, if inhibitory Fc gamma receptors are targeted by blocking activating Fc gamma receptors and ligation of inhibitory Fc gamma receptors, inhibitory Fc gamma receptors are targeted because blocking of the activating Fc gamma receptors prevents ligation of activating Fc gamma receptors with IgG.

In certain embodiments, the blocking antibody is derivatized to prevent concurrent ligation of the Fc fragment of the blocking antibody to Fc gamma receptors. In specific embodiments, the antibody can be conjugated at the Fc fragment to prevent ligation. In other specific embodiments, a Fab fragment of the blocking antibody is used or any other derivative that lacks all or a portion of the Fc domain, particularly the portion of the Fc domain that binds Fc receptors.

Targeting of activating or inhibitory Fc gamma receptors may also be accomplished by a single step that involves the specific ligation of the activating or inhibitory Fc gamma receptors with selective, specific anti-receptor antibodies. Without being bound by theory, activating or inhibitory Fc gamma receptors are specifically targeted by this procedure because of the specificity and selectivity of the antibody. An antibody that preferentially recognizes CD32a over CD32b can, for example, be the monoclonal antibody IV.3. An antibody that preferentially recognizes CD32b over CD32a can, for example, be the monoclonal antibody 2B6.

In particular, the present invention provides methods for promoting the maturation of dendritic cells. In certain embodiments of the invention, dendritic cells are first contacted with an agent that preferentially blocks CD32b over CD32a. Such an agent can, for example, be an anti-CD32b antibody. The dendritic cells are subsequently contacted with complexed IgG. The IgG can be immobilized to simulate complexed or bound IgG. In certain aspects, the agent that preferentially blocks CD32b over CD32a blocks CD32b at least 2 times, 5 times, 10 times, 50 times, 100 times, 500 times, 1000 times, 5000 times, 10,000 times, 50,000 times, or at least 100,000 times more effectively than CD32a. In certain aspects, the agent that preferentially blocks CD32b over CD32a blocks CD32b at most 2 times, 5 times, 10 times, 50 times, 100 times, 500 times, 1000 times, 5000 times, 10,000 times, 50,000 times, or at most, 100,000 times more effectively than CD32a. In certain embodiments of the invention, dendritic cells are (i) contacted with an antibody that preferentially blocks inhibitory Fc gamma receptors over activating Fc gamma receptors; and (ii) contacted with complexed IgG. In certain embodiments, the dendritic cells are contacted with IL-6, IFN-gamma, and/or PGE2 before the dendritic cells are contacted with the agent that preferentially blocks CD32b over CD32a. Without being bound by theory, contacting the dendritic cells with IL-6, IFN-gamma, and/or PGE2 increases the ratio of CD32a over CD32b. Blocking CD32b preferentially and increasing the CD32a to CD32b ratio act in concert to ensure specific ligation of the activating Fc gamma receptor CD32a. An antibody that preferentially blocks CD32b over CD32a can, for example, be the monoclonal antibody 2B6.

In certain embodiments of the invention, the maturation of dendritic cells can be activated by (i) contacting the dendritic cells with an agent that increases the level of activating Fc gamma receptors on dendritic cells relative to the level of inhibitory Fc gamma receptors; and (ii) contacting the dendritic cells with complexed IgG. In certain specific aspects, such an agent that increases the level of activating Fc gamma receptors on a dendritic cell relative to the level of inhibitory Fc gamma receptors on the dendritic cell is IFN-gamma, PGE2, CD40L, or LPS, or a combination thereof. In certain embodiments of the invention, the maturation of dendritic cells can be activated by (i) contacting a population of dendritic cells with an agent that increases the number of dendritic cells displaying detectable numbers of activating Fc gamma receptors on their cell surface relative to the number of dendritic cells displaying detectable numbers of inhibitory Fc gamma receptors on their cell surface in the population of dendritic cells; and (ii) contacting the population of dendritic cells with IgG. The IgG can be immobilized to simulate complexed or bound IgG. Examples of agents that increase the number of dendritic cells displaying activating Fc gamma receptors on their cell surface relative to the number of dendritic cells displaying inhibitory Fc gamma receptors on their cell surface in the population of dendritic cells include IL-6, IFN-gamma, and PGE2, and combinations thereof. In certain embodiments, the activating Fc gamma receptor is CD32a and the inhibitory Fc gamma receptor is CD32b.

In certain embodiments, the invention provides methods for inhibiting the maturation of a dendritic cell. To inhibit the maturation of dendritic cells, the dendritic cells are first contacted with an agent that preferentially blocks CD32a over CD32b; and, subsequently, the dendritic cells are contacted with complexed IgG. Such an agent can be an anti-CD32a antibody. The IgG can be immobilized to simulate complexed or bound IgG. In certain aspects, the agent that preferentially blocks CD32a over CD32b blocks CD32a at least 2 times, 5 times, 10 times, 50 times, 100 times, 500 times, 1000 times, 5000 times, 10,000 times, 50,000 times, or at least 100,000 times more effectively than CD32b. In certain aspects, the agent that preferentially blocks CD32a over CD32b blocks CD32a at most 2 times, 5 times, 10 times, 50 times, 100 times, 500 times, 1000 times, 5000 times, 10,000 times, 50,000 times, or at most 100,000 times more effectively than CD32b. In certain embodiments of the invention, dendritic cells are (i) contacted with an antibody that preferentially blocks inhibitory Fc gamma receptors over activating Fc gamma receptors; and (ii) contacted with complexed IgG. In certain embodiments, the method further comprises contacting the dendritic cells with soluble IgG, TGF-beta, or IFN-alpha before the dendritic cells are contacted with the agent that preferentially blocks CD32a over CD32b. Without being bound by theory, contacting the dendritic cells with soluble IgG, TGF-beta, or IFN-alpha increases the ratio of CD32b over CD32a. Blocking CD32a preferentially and increasing the CD32b to CD32a ratio act in concert to ensure specific ligation of the inhibitory Fc gamma receptor CD32b. An antibody that preferentially blocks CD32b over CD32a can for example be the monoclonal antibody IV.3.

In certain embodiments of the invention, the maturation of dendritic cells can be inhibited by (i) contacting the dendritic cells with an agent that decreases the level of activating Fc gamma receptors on a dendritic cell relative to the level of inhibitory Fc gamma receptors on the dendritic cell; and (ii) contacting the dendritic cell with complexed IgG. In certain specific aspects, such an agent that decreases the level of activating Fc gamma receptors on a dendritic cell relative to the level of inhibitory Fc gamma receptors on the dendritic cell is soluble IgG or IFNalpha, or a combination thereof. In certain embodiments of the invention, the maturation of dendritic cells can be activated by (i) contacting a population of dendritic cells with an agent that increases the number of dendritic cells displaying detectable numbers of activating Fc gamma receptors on their cell surface relative to the number of dendritic cells displaying detectable numbers of inhibitory Fc gamma receptors on their cell surface in the population of dendritic cells; and (ii) contacting the dendritic cell with complexed IgG. The IgG can be immobilized to simulate complexed or bound IgG. Examples of agents that increase the number of dendritic cells displaying activating Fc gamma receptors on their cell surface relative to the number of dendritic cells displaying inhibitory Fc gamma receptor on their cell surface in the population of dendritic cells include soluble IgG, TGF-beta, and IFN-alpha, and combinations thereof. In certain embodiments, the activating Fc gamma receptor is CD32a and the inhibitory Fc gamma receptor is CD32b.

In certain embodiments, the invention is directed to methods for modulating the maturation of dendritic cells by using different IgGs with different avidities for activating Fc gamma receptors versus inhibitory Fc gamma receptors. In certain embodiments, the invention provides a method for promoting the maturation of a dendritic cell, wherein the method comprises contacting the dendritic cell with IgG that has a higher avidity for activating Fc gamma receptors than for inhibitory Fc gamma receptors. Similarly, the maturation of dendritic cells can be inhibited by contacting the dendritic cells with IgG that has a lower avidity for activating Fc gamma receptors than for inhibitory Fc gamma receptors. In certain embodiments, the invention provides a method for promoting the maturation of a dendritic cell, wherein the method comprises contacting the dendritic cell with IgG that has a higher avidity for CD32a than for CD32b. Similarly, the maturation of dendritic cells can be inhibited by contacting the dendritic cells with IgG that has a lower avidity CD32a than for CD32b.

In specific embodiments, IgG4, which binds CD32b but not CD32a, can be used to target CD32b. IgG3, which has a higher affinity for CD32b than for CD32a, can be used to preferentially target CD32b. Human IgG2, which does not bind to the R131 variant of CD32a, can be used to target CD32b in dendritic cells that only express the R131 variant of CD32a. Mouse IgG1, which is bound by the R131 variant of CD32a can be used to target CD32a in dendritic cells that only express the R131 variant of CD32a. Human IgG2 can be used to target CD32a in cells that express at least one allele of the H 131 variant of CD32a.

Thus, according to the present invention, targeting of either activating Fc gamma receptors or inhibitory Fc gamma receptors can be achieved by (i) blocking one type of Fc gamma receptors (i.e., either activating Fc gamma receptors or inhibitory Fc gamma receptors) and activation of signaling events downstream of the other Fc gamma receptor, e.g., by ligation; (ii) altering the ratio between activating Fc gamma receptors and inhibitory Fc gamma receptors; (iii) ligating Fc gamma receptors with complexed IgG, the avidity of which is either higher for activating Fc gamma receptors than for inhibitory Fc gamma receptors or *vice versa.* Any combination of the three approaches is also within the scope of the present invention.

The methods of the invention can be used to reverse the maturation of a matured dendritic cell. In certain embodiments, inhibitory Fc gamma receptors, such as, e.g., CD32b, that are expressed on the matured dendritic cell can be targeted to reverse maturation of a matured dendritic cell. Optionally, the matured dendritic cell can be contacted with an agent that increases the expression of inhibitory Fc gamma receptors on the matured dendritic cell.

In certain embodiments, to promote maturation of dendritic cells, immature dendritic cells are first enriched for CD32a expressing immature dendritic cells. Enrichment of CD32a expressing cells can be accomplished, e.g., by selecting CD32a expressing cells from a population of cells or contacting the immature dendritic cells with an agent that promotes expression of CD32a. In one aspect, expression of CD32a relative to CD32b is increased. In a specific embodiment, plasmacytoid dendritic cells are used with the methods of the invention. Without being limited by theory, plasmacytoid dendritic cells express CD32a but not CD32b.

### 5.2 AGENTS THAT BLOCK INHIBITORY Fcγ RECEPTORS

Any agent that preferentially blocks inhibitory Fcγ receptors over activating Fcγ receptors can be used with the methods and compositions of the invention. In certain aspects, the agent that preferentially blocks inhibitory Fcγ receptors over activating Fcγ receptors blocks inhibitory Fcγ receptors at least 2 times, 5 times, 10 times, 50 times, 100 times, 500 times, 1000 times, 5000 times, 10,000 times, 50,000 times, or at least 100,000 times more effectively than activating Fcγ receptors. In certain aspects, the agent that preferentially blocks inhibitory Fcγ receptors over activating Fcγ receptors blocks inhibitory Fcγ receptors at most 2 times, 5 times, 10 times, 50 times, 100 times, 500 times, 1000 times, 5000 times, 10,000 times, 50,000 times, or at most 100,000 times more effectively than activating Fcγ receptors. In a specific embodiment, the agent that preferentially blocks inhibitory Fcγ receptors over activating Fcγ receptors blocks only inhibitory Fcγ receptors.

Without being bound by theory, the agent that preferentially blocks inhibitory Fcγ receptors over activating Fcγ receptors binds preferentially to the inhibitory Fcγ receptors and prevents ligation of the inhibitory Fcγ receptor with IgG. Blockage of ligation may occur by any mechanism, e.g., through steric hindrance or conformational changes of the receptor upon binding to the agent. Thus, any agent that preferentially binds to inhibitory Fcγ receptors such that it prevents ligation of the inhibitory Fcγ receptor to IgG without mimicking ligation to IgG can be used with the methods of the invention.

The agent that preferentially blocks inhibitory Fcγ receptors over activating Fcγ receptors can be, without being limited to, a peptide, a nucleic acid, a protein, a small organic molecule, a sugar, or a lipid. In certain embodiments, the agent is an antibody or a fragment of an antibody against inhibitory Fcγ receptors. The antibody can be a derivatized antibody, such as a humanized antibody. The antibody can be an IgG. In certain embodiments, the anti-inhibitory Fcγ receptor IgG can be used to concurrently block inhibitory Fcγ receptors (with the Fab fragment of the anti-inhibitory Fcγ receptor IgG) and ligate activating Fcγ receptors (with the Fc fragment of the anti-inhibitory Fcγ receptor IgG). In other embodiments, anti-inhibitory Fcγ receptor antibodies are used to block inhibitory Fcγ receptors and complexed or immobilized IgG is used for ligation of the activating Fcγ receptors. In these embodiments, ligation between the Fc fragment of the anti-inhibitory Fcγ receptor antibody and the activating Fcγ receptors is to be avoided for example by using a Fab fragment of the blocking antibody or any other derivative that lacks all or a portion of the Fc domain, particularly the portion of the Fc domain that binds Fc receptors. Methods for engineering the Fc domain of an antibody are described, e.g., in WO 2004/063351 published on July 29, 2004 (PCT/US2004/000643 filed January 9, 2004), which is incorporated herein in its entirety. In other embodiments, the Fc fragment of the anti-inhibitory Fcγ receptor antibody is conjugated to a molecule such that ligation is prevented.

In certain embodiments, the agent that preferentially blocks inhibitory Fcγ receptors over activating Fcγ receptors preferentially blocks FcγRIIB (CD32b). In certain embodiments, the agent that preferentially blocks FcγRIIB is an anti-FcγRIIB-specific antibody (see section 5.4).

In certain aspects of the invention, agents that preferentially inhibit the expression of inhibitory Fcγ receptors can be used with the methods of the invention.

### 5.3 AGENTS THAT BLOCK ACTIVATING Fcγ RECEPTORS

Any agent that preferentially blocks activating Fcγ receptors over inhibitory Fcγ receptors can be used with the methods and compositions of the invention. In certain aspects, the agent that preferentially blocks activating Fcγ receptors over inhibitory Fcγ receptors blocks activating Fcγ receptors at least 2 times, 5 times, 10 times, 50 times, 100 times, 500 times, 1000 times, 5000 times, 10,000 times, 50,000 times, or at least 100,000 times more effectively than inhibitory Fcγ receptors. In certain aspects, the agent that preferentially blocks activating Fcγ receptors over inhibitory Fcγ receptors blocks activating Fcγ receptors at most 2 times, 5 times, 10 times, 50 times, 100 times, 500 times, 1000 times, 5000 times, 10,000 times, 50,000 times, or at most 100,000 times more effectively than inhibitory Fcγ receptors. In a specific embodiment, the agent that preferentially blocks activating Fcγ receptors over inhibitory Fcγ receptors blocks only activating Fcγ receptors.

Without being bound by theory, the agent that preferentially blocks activating Fcγ receptors over inhibitory Fcγ receptors binds preferentially to the activating Fcγ receptors and prevents ligation of the activating Fcγ receptor with IgG. Blockage of ligation may occur by any mechanism, e.g., through steric hindrance or conformational changes of the receptor upon binding to the agent. Thus, any agent that preferentially binds to activating Fcγ receptors such that it prevents ligation of the activating Fcγ receptor to IgG without mimicking ligation to IgG can be used with the methods of the invention.

The agent that preferentially blocks activating Fcγ receptors over inhibitory Fcγ receptors can be, without being limited to, a peptide, a nucleic acid, a protein, a small organic molecule, a sugar, or a lipid. In certain embodiments, the agent is an antibody or a fragment of an antibody against activating Fcγ receptors. The antibody can be a derivatized antibody, such as a humanized antibody. The antibody can be an IgG. In certain embodiments, the anti-activating Fcγ receptor IgG can be used to concurrently block activating Fcγ receptors (with the Fab fragment of the anti-activating Fcγ receptor IgG) and ligate inhibitory Fcγ receptors (with the Fc fragment of the anti-inhibitory Fcγ receptor IgG). In other embodiments, anti-activating Fcγ receptor antibodies are used to block activating Fcγ receptors and complexed or immobilized IgG is used for ligation of the inhibitory Fcγ receptors. In these embodiments, ligation between the Fc fragment of the anti-activating Fcγ receptor antibody and the inhibitory Fcγ receptors is to be avoided for example by using a Fab fragment of the blocking antibody or any other derivative that lacks all or a portion of the Fc domain, particularly the portion of the Fc domain that binds Fc receptors. Methods for engineering the Fc domain of an antibody are described, e.g., in WO 2004/063351 published on July 29, 2004 (PCT/US2004/000643 filed January 9, 2004), which is incorporated herein in its entirety. In other embodiments, the Fc fragment of the anti-inhibitory Fcγ receptor antibody is conjugated to a molecule such that ligation is prevented.

In certain embodiments, the agent that preferentially blocks activating Fcγ receptors over inhibitory Fcγ receptors preferentially blocks FcγRIIA (CD32a). In certain embodiments, the agent that preferentially blocks FcγRIIA is an anti-FcγRIIA-specific antibody (see section 5.5).

In certain aspects of the invention, agents that preferentially inhibit the expression of activating Fcγ receptors can be used with the methods of the invention.

### 5.4 FcγRIIB-SPECIFIC ANTIBODIES

Antibodies (preferably monoclonal antibodies) or fragments thereof that specifically bind FcγRIIB (CD32b), preferably human FcγRIIB, more preferably native human FcγRIIB with a greater affinity than said antibodies or fragments thereof bind FcγRIIA (CD32a), preferably human FcγRIIA, more preferably native human FcγRIIA can be used with the methods and compositions of the invention. Preferably, such antibodies bind the extracellular domain of native human FcγRIIB. In certain embodiments, the antibodies or fragments thereof bind to FcγRIIB with an affinity greater than two-fold, four fold, 6 fold, 10 fold, 20 fold, 50 fold, 100 fold, 1000 fold, 10⁴ fold, 10⁵ fold, 10⁶ fold, 10⁷ fold, or 10⁸ fold than said antibodies or fragments thereof bind FcγRIIA. In one particular embodiment, the antibody is a mouse monoclonal antibody produced by clone 2B6 or 3H7, having ATCC accession numbers PTA-4591 and PTA-4592, respectively. Hybridomas producing antibodies of the invention have been deposited with the American Type Culture Collection (10801 University Blvd., Manassas, VA. 20110-2209) on August 13, 2002 under the provisions of the Budapest Treaty on the International Recognition of the Deposit of Microorganisms for the Purposes of Patent Procedures, and assigned accession numbers PTA-4591 (for hybridoma producing 2B6) and PTA-4592 (for hybridoma producing 3H7), respectively and are incorporated herein by reference. In certain embodiments, the antibodies are human or have been humanized, preferably a humanized version of the antibody produced by clone 3H7 or 2B6. In yet other embodiments, the antibodies that preferentially block inhibitory Fcγ receptors do not bind Fc activation receptors, e.g., FcγIIIA, FcγIIIB, *etc.*

In certain embodiments, an anti-FcγRIIB-specific antibody that binds to the IgG binding site of the inhibitory Fc gamma receptor can be used with the methods of the invention.

In one embodiment, the FcγRIIB-specific antibody is not the monoclonal antibody designated KB61, as disclosed in Pulford *et al.,* 1986 *(Immunology,* 57*:* 71-76) or the monoclonal antibody designated MAbII8D2 as disclosed in Weinrich *et al.,* 1996, *(Hybridoma,* 15(2):109-6).

In a specific embodiment, a FcγRIIB-specific antibody that can be used with the methods and compositions of the invention does not bind to the same epitope and/or does not compete with binding with the monoclonal antibody KB61 or II8D2. Preferably, the FcγRIIB-specific antibody of the invention does not bind the amino acid sequence SDPNFSI corresponding to positions 135-141 of FcγRIIb2 isoform.

Other antibodies that can be used with the invention, include antibodies that are produced by clones including but not limited to 1D5, 2E1, 2H9, 2D11, and 1F2 having ATCC Accession numbers, PTA-5958, PTA-5961, PTA-5962, PTA-5960, PTA-5959, respectively. Hybridomas producing the above-identified clones were deposited with the American Type Culture Collection (10801 University Blvd., Manassas, VA. 20110-2209) on May 7, 2004, respectively and are incorporated herein by reference.

Antibodies or fragments thereof that can be used with the methods of the invention can comprise an amino acid sequence of a variable heavy chain and/or variable light chain that is at least 45%, at least 50%, at least 55%, at least 60%, at least 65%, at least 70%, at least 75%, at least 80%, at least 85%, at least 90%, at least 95%, or at least 99% identical to the amino acid sequence of the variable heavy chain and/or light chain of the mouse monoclonal antibody produced by clone 2B6 or 3H7 having ATCC accession numbers PTA-4591 and PTA-4592, respectively. The determination of percent identity of two amino acid sequences can be determined by any method known to one skilled in the art, including BLAST protein searches.

The present invention also encompasses the use of antibodies or antibody fragments that specifically bind FcγRIIB with greater affinity than said antibodies or fragments thereof binds FcγRIIA, wherein said antibodies or antibody fragments are encoded by a nucleotide sequence that hybridizes to the nucleotide sequence of the mouse monoclonal antibody produced by clone 2B6 or 3H7 having ATCC accession numbers PTA-4591 and PTA-4592, respectively, under stringent conditions. In a preferred embodiment, the invention provides antibodies or fragments thereof that specifically bind FcγRIIB with greater affinity than said antibodies or fragments thereof bind FcγRIIA, said antibodies or antibody fragments comprising a variable light and/or variable heavy chain encoded by a nucleotide sequence that hybridizes under stringent conditions to the nucleotide sequence of the variable light and/or variable heavy chain of the mouse monoclonal antibody produced by clone 2B6 or 3H7 having ATCC accession numbers PTA-4591 and PTA-4592, respectively, under stringent conditions. In another preferred embodiment, the invention provides antibodies or fragments thereof that specifically bind FcγRIIB with greater affinity than said antibodies or fragments thereof bind FcγRIIA, said antibodies or antibody fragments comprising one or more CDRs encoded by a nucleotide sequence that hybridizes under stringent conditions to the nucleotide sequence of one or more CDRs of the mouse monoclonal antibody produced by clone 2B6 or 3H7 with ATCC accession numbers PTA-4591 and PTA-4592, respectively. Stringent hybridization conditions include, but are not limited to, hybridization to filter-bound DNA in 6X sodium chloride/sodium citrate (SSC) at about 45°C followed by one or more washes in 0.2X SSC/0.1% SDS at about 50-65°C, highly stringent conditions such as hybridization to filter-bound DNA in 6X SSC at about 45°C followed by one or more washes in 0.1X SSC/0.2% SDS at about 60°C, or any other stringent hybridization conditions known to those skilled in the art *(see,* for example, Ausubel, F.M. *et al.,* eds. 1989 Current Protocols in Molecular Biology, vol. 1, Green Publishing Associates, Inc. and John Wiley and Sons, Inc., NY at pages 6.3.1 to 6.3.6 and 2.10.3), incorporated herein by reference.

The use of antibodies with the methods of the invention is described in more detail in section 5.6.

### 5.5 FcγRIIA-SPECIFIC ANTIBODIES

Antibodies (preferably monoclonal antibodies) or fragments thereof that specifically bind FcγRIIA (CD32a), preferably human FcγRIIA, more preferably native human FcγRIIA with a greater affinity than said antibodies or fragments thereof bind FcγRIIB (CD32b), preferably human FcγRIIB, more preferably native human FcγRIIB can be used with the methods and compositions of the invention. Preferably, such antibodies bind the extracellular domain of native human FcγRIIA. In certain embodiments, the antibodies or fragments thereof bind to FcγRIIA with an affinity greater than two-fold, four fold, 6 fold, 10 fold, 20 fold, 50 fold, 100 fold, 1000 fold, 10⁴ fold, 10⁵ fold, 10⁶ fold, 10⁷ fold, or 10⁸ fold than said antibodies or fragments thereof bind FcγRIIB. In one particular embodiment, the antibody is a mouse monoclonal antibody produced by clone IV.3, having ATCC accession number HB0217. In certain embodiments, the antibodies are human or have been humanized, preferably a humanized version of the antibody produced by clone IV.3. In yet other embodiments, the antibodies that preferentially block activating Fcγ receptors do not bind inhibitory Fc gamma receptors.

In certain embodiments, an anti-FcγRIIA-specific antibody that binds to the IgG binding site of the activating Fc gamma receptor can be used with the methods of the invention.

Antibodies or fragments thereof that can be used with the methods of the invention can comprise an amino acid sequence of a variable heavy chain and/or variable light chain that is at least 45%, at least 50%, at least 55%, at least 60%, at least 65%, at least 70%, at least 75%, at least 80%, at least 85%, at least 90%, at least 95%, or at least 99% identical to the amino acid sequence of the variable heavy chain and/or light chain of the mouse monoclonal antibody produced by clone IV.3. The determination of percent identity of two amino acid sequences can be determined by any method known to one skilled in the art, including BLAST protein searches.

The present invention also encompasses the use of antibodies or antibody fragments that specifically bind FcγRIIA with greater affinity than said antibodies or fragments thereof binds FcγRIIB, wherein said antibodies or antibody fragments are encoded by a nucleotide sequence that hybridizes to the nucleotide sequence of the mouse monoclonal antibody produced by clone IV.3, under stringent conditions. In a preferred embodiment, the invention provides antibodies or fragments thereof that specifically bind FcγRIIA with greater affinity than said antibodies or fragments thereof bind FcγRIIB, said antibodies or antibody fragments comprising a variable light and/or variable heavy chain encoded by a nucleotide sequence that hybridizes under stringent conditions to the nucleotide sequence of the variable light and/or variable heavy chain of the mouse monoclonal antibody produced by clone IV.3, under stringent conditions. In another preferred embodiment, the invention provides antibodies or fragments thereof that specifically bind FcγRIIA with greater affinity than said antibodies or fragments thereof bind FcγRIIB, said antibodies or antibody fragments comprising one or more CDRs encoded by a nucleotide sequence that hybridizes under stringent conditions to the nucleotide sequence of one or more CDRs of the mouse monoclonal antibody produced by clone IV.3. Stringent hybridization conditions include, but are not limited to, hybridization to filter-bound DNA in 6X sodium chloride/sodium citrate (SSC) at about 45°C followed by one or more washes in 0.2X SSC/0.1% SDS at about 50-65°C, highly stringent conditions such as hybridization to filter-bound DNA in 6X SSC at about 45°C followed by one or more washes in 0.1X SSC/0.2% SDS at about 60°C, or any other stringent hybridization conditions known to those skilled in the art *(see,* for example, Ausubel, F.M. *et al.,* eds. 1989 Current Protocols in Molecular Biology, vol. 1, Green Publishing Associates, Inc. and John Wiley and Sons, Inc., NY at pages 6.3.1 to 6.3.6 and 2.10.3), incorporated herein by reference.

The use of antibodies with the methods of the invention is described in more detail in section 5.6.

### 5.6 USE OF ANTIBODIES WITH THE METHODS OF THE INVENTION

Antibodies that can be used with the methods of the invention include, but are not limited to, monoclonal antibodies, synthetic antibodies, recombinantly produced antibodies, multispecific antibodies, human antibodies, humanized antibodies, chimeric antibodies, camelized antibodies, single-chain Fvs (scFv), single chain antibodies, Fab fragments, F(ab') fragments, disulfide-linked Fvs (sdFv), intrabodies, and epitope-binding fragments of any of the above.

The antibodies used in the methods of the invention may be from any animal origin including birds and mammals (e.g., human, non-human primate, murine, donkey, sheep, rabbit, goat, guinea pig, camel, horse, or chicken). Preferably, the antibodies are human or humanized monoclonal antibodies. As used herein, "human" antibodies include antibodies having the amino acid sequence of a human immunoglobulin and include antibodies isolated from human immunoglobulin libraries or libraries of synthetic human immunoglobulin coding sequences or from mice that express antibodies from human genes.

The antibodies used in the methods of the present invention may be monospecific, bispecific, trispecific or of greater multispecificity. Multispecific antibodies may immunospecifically bind to different epitopes of FcγRIIB or immunospecifically bind to both an epitope of FcγRIIB as well a heterologous epitope, such as a heterologous polypeptide or solid support material. *See, e.g.,* International Publication Nos. WO 93/17715, WO 92/08802, WO 91/00360, and WO 92/05793; Tutt, *et al.,* 1991, *J. Immunol.* 147:60-69; U.S. Patent Nos. 4,474,893, 4,714,681, 4,925,648, 5,573,920, and 5,601,819; and Kostelny *et al.,* 1992, *J Immunol.* 148:1547-1553; Todorovska *et al.,* 2001 *Journal of Immunological Methods,* 248:47-66.

In a specific embodiment, an antibody used in the methods of the present invention is an antibody or an antigen-binding fragment thereof (e.g., comprising one or more complementarily determining regions (CDRs), preferably all 6 CDRs) of the antibody produced by clone 2B6 or 3H7 with ATCC accession numbers PTA-4591 and PTA-4592, respectively (e.g., the heavy chain CDR3). In another embodiment, an antibody used in the methods of the present invention binds to the same epitope as the mouse monoclonal antibody produced from clone 2B6 or 3H7 with ATCC accession numbers PTA-4591 and PTA-4592, respectively and/or competes with the mouse monoclonal antibody produced from clone 2B6 or 3H7 with ATCC accession numbers PTA-4591 and PTA-4592, respectively as determined, e.g., in an ELISA assay or other appropriate competitive immunoassay, and also binds Fcγ-RIIB with a greater affinity than said antibody or a fragment thereof binds FcγRIIA.

The antibodies used in the methods of the invention include derivatives that are modified, i.e, by the covalent attachment of any type of molecule to the antibody such that covalent attachment. For example, but not by way of limitation, the antibody derivatives include antibodies that have been modified, e.g., by glycosylation, acetylation, pegylation, phosphorylation, amidation, derivatization by known protecting/blocking groups, proteolytic cleavage, linkage to a cellular ligand or other protein, *etc.* Any of numerous chemical modifications may be carried out by known techniques, including, but not limited to, specific chemical cleavage, acetylation, formylation, metabolic synthesis of tunicamycin, *etc.* Additionally, the derivative may contain one or more non-classical amino acids.

For some uses, including *in vivo* use of antibodies in humans and *in vitro* detection assays, it may be preferable to use human, chimeric or humanized antibodies. Completely human antibodies are particularly desirable for therapeutic treatment of human subjects. Human antibodies can be made by a variety of methods known in the art including phage display methods described above using antibody libraries derived from human immunoglobulin sequences. *See* also U.S. Patent Nos. 4,444,887 and 4,716,111; and International Publication Nos. WO 98/46645, WO 98/50433, WO 98/24893, WO 98/16654, WO 96/34096, WO 96/33735, and WO 91/10741; each of which is incorporated herein by reference in its entirety.

Human antibodies can also be produced using transgenic mice which are incapable of expressing functional endogenous immunoglobulins, but which can express human immunoglobulin genes. For example, the human heavy and light chain immunoglobulin gene complexes may be introduced randomly or by homologous recombination into mouse embryonic stem cells. Alternatively, the human variable region, constant region, and diversity region may be introduced into mouse embryonic stem cells in addition to the human heavy and light chain genes. The mouse heavy and light chain immunoglobulin genes may be rendered non-functional separately or simultaneously with the introduction of human immunoglobulin loci by homologous recombination. In particular, homozygous deletion of the J_{H} region prevents endogenous antibody production. The modified embryonic stem cells are expanded and microinjected into blastocysts to produce chimeric mice. The chimeric mice are then bred to produce homozygous offspring which express human antibodies. The transgenic mice are immunized using conventional methodologies with a selected antigen, e.g., all or a portion of a polypeptide of the invention. Monoclonal antibodies directed against the antigen can be obtained from the immunized, transgenic mice using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA, IgM and IgE antibodies. For an overview of this technology for producing human antibodies, *see* Lonberg and Huszar *(*1995*, Int. Rev. Immunol.* 13:65-93, which is incorporated herein by reference in its entirety). For a detailed discussion of this technology for producing human antibodies and human monoclonal antibodies and protocols for producing such antibodies, *see, e.g.,* International Publication Nos. WO 98/24893, WO 96/34096, and WO 96/33735; and U.S. Patent Nos. 5,413,923, 5,625,126, 5,633,425, 5,569,825, 5,661,016, 5,545,806, 5,814,318, and 5,939,598, which are incorporated by reference herein in their entirety. In addition, companies such as Abgenix, Inc. (Freemont, CA) and Medarex (Princeton, NJ) can be engaged to provide human antibodies directed against a selected antigen using technology similar to that described above.

A chimeric antibody is a molecule in which different portions of the antibody are derived from different immunoglobulin molecules such as antibodies having a variable region derived from a non-human antibody and a human immunoglobulin constant region. Methods for producing chimeric antibodies are known in the art. *See* e.g., Morrison, 1985, *Science* 229:1202; Oi *et al.,* 1986, *BioTechniques* 4:214; Gillies *et al.,* 1989, *J. Immunol. Methods* 125:191-202; and U.S. Patent Nos. 6,311,415, 5,807,715, 4,816,567, and 4,816,397, which are incorporated herein by reference in their entirety. Chimeric antibodies comprising one or more CDRs from a non-human species and framework regions from a human immunoglobulin molecule can be produced using a variety of techniques known in the art including, for example, CDR-grafting (EP 239,400; International Publication No. WO 91/09967; and U.S. Patent Nos. 5,225,539, 5,530,101, and 5,585,089), veneering or resurfacing (EP 592,106; EP 519,596; Padlan, 1991, *Molecular Immunology* 28(4/5):489-498; Studnicka *et al.,* 1994, *Protein Engineering* 7:805; and Roguska *et al.,* 1994, *PNAS* 91:969), and chain shuffling (U.S. Patent No. 5,565,332). Each of the above-identified references is incorporated herein by reference in its entirety.

Often, framework residues in the framework regions will be substituted with the corresponding residue from the CDR donor antibody to alter, preferably improve, antigen binding. These framework substitutions are identified by methods well known in the art, e.g., by modeling of the interactions of the CDR and framework residues to identify framework residues important for antigen binding and sequence comparison to identify unusual framework residues at particular positions. *(See, e.g.,* U.S. Patent No. 5,585,089; and Riechmann *et al.,* 1988, *Nature* 332:323, which are incorporated herein by reference in their entireties.)

A humanized antibody is an antibody, a variant or a fragment thereof which is capable of binding to a predetermined antigen and which comprises a framework region having substantially the amino acid sequence of a human immunoglobulin and a CDR having substantially the amino acid sequence of a non-human immunoglobulin. A humanized antibody comprises substantially all of at least one, and typically two, variable domains in which all or substantially all of the CDR regions correspond to those of a non-human immunoglobulin (i.e., donor antibody) and all or substantially all of the framework regions are those of a human immunoglobulin consensus sequence. Preferably, a humanized antibody also comprises at least a portion of an immunoglobulin constant region (Fc), typically that of a human immunoglobulin. Ordinarily, the antibody will contain both the light chain as well as at least the variable domain of a heavy chain. The antibody also may include the CH1, hinge, CH2, CH3, and CH4 regions of the heavy chain. The humanized antibody can be selected from any class of immunoglobulins, including IgM, IgG, IgD, IgA and IgE, and any isotype, including IgG₁, IgG₂, IgG₃ and IgG₄. Usually the constant domain is a complement fixing constant domain where it is desired that the humanized antibody exhibit cytotoxic activity, and the class is typically IgG₁. Where such cytotoxic activity is not desirable, the constant domain may be of the IgG₂ class. The humanized antibody may comprise sequences from more than one class or isotype, and selecting particular constant domains to optimize desired effector functions is within the ordinary skill in the art. The framework and CDR regions of a humanized antibody need not correspond precisely to the parental sequences, e.g., the donor CDR or the consensus framework may be mutagenized by substitution, insertion or deletion of at least one residue so that the CDR or framework residue at that site does not correspond to either the consensus or the import antibody. Such mutations, however, will not be extensive. Usually, at least 75% of the humanized antibody residues will correspond to those of the parental framework region (FR) and CDR sequences, more often 90%, and most preferably greater than 95%. Humanized antibodies can be produced using variety of techniques known in the art, including but not limited to, CDR-grafting (European Patent No. EP 239,400; International Publication No. WO 91/09967; and U.S. Patent Nos. 5,225,539, 5,530,101, and 5,585,089), veneering or resurfacing (European Patent Nos. EP 592,106 and EP 519,596; Padlan, 1991, *Molecular Immunology* 28(4/5):489-498; Studnicka *et al.,* 1994, *Protein Engineering* 7(6):805-814; and Roguska *et al.,* 1994, *PNAS* 91:969-973), chain shuffling (U.S. Patent No. 5,565,332), and techniques disclosed in, e.g., U.S. Patent Nos. 6,407,213, 5,766,886, 5,585,089, International Publication No. WO 9317105, Tan *et al.,* 2002, *J. Immunol.* 169:1119-25, Caldas *et al.,* 2000, *Protein Eng.* 13:353-60, Morea *et al.,* 2000, *Methods* 20:267-79, Baca *et al.,* 1997, *J. Biol. Chem.* 272:10678-84, Roguska *et al.,* 1996, *Protein Eng*. 9:895-904, Couto et al., 1995, *Cancer Res. 55* (23 Supp):5973s-5977s, Couto *et al.,* 1995, *Cancer Res.* 55:1717-22, Sandhu, 1994, *Gene* 150:409-10, *Pedersen et al.,* 1994, J. *Mol. Biol.* 235:959-73, Jones *et al.,* 1986, *Nature* 321:522-525, Riechmann *et al.,* 1988, *Nature* 332:323, and Presta, 1992, *Curr. Op. Struct. Biol.* 2:593-596. Often, framework residues in the framework regions will be substituted with the corresponding residue from the CDR donor antibody to alter, preferably improve, antigen binding. These framework substitutions are identified by methods well known in the art, e.g., by modeling of the interactions of the CDR and framework residues to identify framework residues important for antigen binding and sequence comparison to identify unusual framework residues at particular positions. *(See, e.g.,* U.S. Patent No. 5,585,089; and Riechmann et al., 1988, *Nature* 332:323, which are incorporated herein by reference in their entireties.)

Single domain antibodies, including camelized single domain antibodies (*See* e.g., Muyldermans *et al.,* 2001, *Trends Biochem. Sci.* 26:230; Nuttall *et al.,* 2000, *Cur. Pharm. Biotech.* 1:253; Reichmann and Muyldermans, 1999, *J. Immunol. Meth.* 231:25; International Publication Nos. WO 94/04678 and WO 94/25591; U.S. Patent No. 6,005,079; which are incorporated herein by reference in their entireties) may also be used with the methods of the invention. For example, a single domain antibody comprises two VH domains with modifications such that single domain antibody is formed.

The methods of the present invention also encompass the use of antibodies or fragments thereof that have half-lives (e.g., serum half-lives) in a mammal, preferably a human, of greater than 15 days, preferably greater than 20 days, greater than 25 days, greater than 30 days, greater than 35 days, greater than 40 days, greater than 45 days, greater than 2 months, greater than 3 months, greater than 4 months, or greater than 5 months. The increased half-lives of the antibodies of the present invention or fragments thereof in a mammal, preferably a human, results in a higher serum titer of said antibodies or antibody fragments in the mammal, and thus, reduces the frequency of the administration of said antibodies or antibody fragments and/or reduces the concentration of said antibodies or antibody fragments to be administered. Antibodies or fragments thereof having increased *in vivo* half-lives can be generated by techniques known to those of skill in the art. For example, antibodies or fragments thereof with increased *in vivo* half-lives can be generated by modifying (e.g., substituting, deleting or adding) amino acid residues identified as involved in the interaction between the Fc domain and the FcRn receptor. The antibodies that can be used with the methods of the invention may be engineered by methods described in Ward *et al.* to increase biological half-lives *(See* U.S. 6,277,375 B1). For example, antibodies may be engineered in the Fc-hinge domain to have increased *in vivo* or serum half-lives.

Antibodies or fragments thereof with increased *in vivo* half-lives can be generated by attaching to said antibodies or antibody fragments polymer molecules such as high molecular weight polyethyleneglycol (PEG). PEG can be attached to said antibodies or antibody fragments with or without a multifunctional linker either through site-specific conjugation of the PEG to the N- or C- terminus of said antibodies or antibody fragments or via epsilon-amino groups present on lysine residues. Linear or branched polymer derivatization that results in minimal loss of biological activity will be used. The degree of conjugation will be closely monitored by SDS-PAGE and mass spectrometry to ensure proper conjugation of PEG molecules to the antibodies. Unreacted PEG can be separated from antibody-PEG conjugates by, e.g., size exclusion or ion-exchange chromatography.

The antibodies that can be used with the methods of the invention may also be modified by the methods and coupling agents described by Davis *et al. (See* U.S. 4,179,337) in order to provide compositions that can be injected into the mammalian circulatory system with substantially no immunogenic response.

The present invention encompasses the use of antibodies comprising modifications preferably, in the Fc region that modify the binding affinity of the antibody to one or more FcγR. Methods for modifying antibodies with modified binding to one or more FcγR are known in the art, *see, e.g.,* PCT Publication Nos. WO 99/58572, WO 99/51642, WO 98/23289, WO 89/07142, WO 88/07089, and U.S. Patent Nos. 5,843,597 and 5,642,821, each of which is incorporated herein by reference in their entirety.

In certain embodiments, antibodies with altered oligosaccharide content can be used with the methods of the invention. Oligosaccharides as used herein refer to carbohydrates containing two or more simple sugars and the two terms may be used interchangeably herein. Carbohydrate moieties of the instant invention will be described with reference to commonly used nomenclature in the art. For a review of carbohydrate chemistry, *see, e.g.,* Hubbard *et al.,* 1981 *Ann. Rev. Biochem.,* 50: 555-583, which is incorporated herein by reference in its entirety. This nomenclature includes for example, Man which represents mannose; GlcNAc which represents 2-N-acetylglucosamine; Gal which represents galactose; Fuc for fucose and Glc for glucose. Sialic acids are described by the shorthand notation NeuNAc for 5-N-acetylneuraminic acid, and NeuNGc for 5-glycolneuraminic.

In general, antibodies contain carbohydrate moeities at conserved positions in the constant region of the heavy chain, and up to 30% of human IgGs have a glycosylated Fab region. IgG has a single N-linked biantennary carbohydrate structure at Asn 297 which resides in the CH2 domain (Jefferis *et al.,* 1998, *Immunol. Rev.* 163: 59-76; Wright *et al.,* 1997, *Trends Biotech* 15: 26-32). Human IgG typically has a carbohydrate of the following structure; GlcNAc(Fucose)-GlcNAc-Man-(ManGlcNAc)₂. However variations among IgGs in carbohydrate content does occur which leads to altered function, *see, e.g.,* Jassal *et al.,* 2001 *Bichem. Biophys. Res. Commun.* 288: 243-9; Groenink *et al.,* 1996 *J. Immunol.* 26: 1404-7; Boyd *et al.,* 1995 *Mol. Immunol.* 32: 1311-8; Kumpel *et al.,* 1994, *Human Antibody Hybridomas,* 5: 143-51. The invention encompasses antibodies comprising a variation in the carbohydrate moiety that is attached to Asn 297. In one embodiment, the carbohydrate moiety has a galactose and/or galactose-sialic acid at one or both of the terminal GlcNAc and/or a third GlcNac arm (bisecting GlcNAc).

In some embodiments, the antibodies that can be used with the methods of the invention are substantially free of one or more selected sugar groups, e.g., one or more sialic acid residues, one or more galactose residues, one or more fucose residues. An antibody that is substantially free of one or more selected sugar groups may be prepared using common methods known to one skilled in the art, including for example recombinantly producing an antibody of the invention in a host cell that is defective in the addition of the selected sugar groups(s) to the carbohydrate moiety of the antibody, such that about 90-100% of the antibody in the composition lacks the selected sugar group(s) attached to the carbohydrate moiety. Alternative methods for preparing such antibodies include for example, culturing cells under conditions which prevent or reduce the addition of one or more selected sugar groups, or post-translational removal of one or more selected sugar groups.

In a specific embodiment, a substantially homogenous antibody preparation, wherein about 80-100% of the antibody in the composition lacks a fucose on its carbohydrate moiety, e.g., the carbohydrate attachment on Asn 297, can be used with the methods of the invention. The antibody may be prepared for example by (a) use of an engineered host cell that is deficient in fucose metabolism such that it has a reduced ability to fucosylate proteins expressed therein; (b) culturing cells under conditions which prevent or reduce fusocylation; (c) post-translational removal of fucose, e.g., with a fucosidase enzyme; or (d) purification of the antibody so as to select for the product which is not fucosylated. Most preferably, nucleic acid encoding the desired antibody is expressed in a host cell that has a reduced ability to fucosylate the antibody expressed therein. Preferably the host cell is a dihydrofolate reductase deficient chinese hamster ovary cell (CHO), e.g., a Lec 13 CHO cell (lectin resistant CHO mutant cell line; Ribka & Stanley, 1986, *Somatic Cell & Molec. Gen.* 12(1): 51-62; Ripka *et al.,* 1986 *Arch. Biochem. Biophys.* 249(2): 533-45), CHO-K1, DUX-B11, CHO-DP12 or CHO-DG44, which has been modified so that the antibody is not substantially fucosylated. Thus, the cell may display altered expression and/or activity for the fucoysltransferase enzyme, or another enzyme or substrate involved in adding fucose to the N-linked oligosaccharide so that the enzyme has a diminished activity and/or reduced expression level in the cell. For methods to produce antibodies with altered fucose content, *see, e.g.,* WO 03/035835 and Shields *et al.,* 2002, *J. Biol. Chem.* 277(30): 26733-40; both of which are incorporated herein by reference in their entirety.

In some embodiments, the altered carbohydrate modifications modulate one or more of the following: solubilization of the antibody, facilitation of subcellular transport and secretion of the antibody, promotion of antibody assembly, conformational integrity, and antibody-mediated effector function. In a specific embodiment the altered carbohydrate modifications enhance antibody mediated effector function relative to the antibody lacking the carbohydrate modification. Carbohydrate modifications that lead to altered antibody mediated effector function are well known in the art (for *e.g., see* Shields R.L. *et al.,* 2001, *J. Biol. Chem.* 277(30): 26733-40; Davies J. *et al.,* 2001, *Biotechnology & Bioengineering,* 74(4): 288-294). In another specific embodiment, the altered carbohydrate modifications enhance the binding of antibodies of the invention to Fc□RIIB receptor. Altering carbohydrate modifications in accordance with the methods of the invention includes, for example, increasing the carbohydrate content of the antibody or decreasing the carbohydrate content of the antibody. Methods of altering carbohydrate contents are known to those skilled in the art, *see, e.g.,* Wallick *et al.,* 1988, *Journal of Exp. Med.* 168(3): 1099-1109; Tao *et al.,* 1989 *Journal of Immunology,* 143(8): 2595-2601; Routledge *et al.,* 1995 *Transplantation,* 60(8): 847-53; Elliott *et al.* 2003; *Nature Biotechnology,* 21: 414-21; Shields *et al.* 2002 *Journal of Biological Chemistry,* 277(30): 26733-40; all of which are incorporated herein by reference in their entirety.

In some embodiments, antibodies that can be used with the methods of the invention comprise one or more glycosylation sites, so that one or more carbohydrate moieties are covalently attached to the antibody. In other embodiments, the antibodies comprise one or more glycosylation sites and one or more modifications in the Fc region, such as those disclosed *supra* and those known to one skilled in the art. In preferred embodiments, the one or more modifications in the Fc region modify the affinity of the antibody for an Fcγ receptor. In some embodiments, the antibodies comprise one or more modifications of amino acids that are directly or indirectly known to interact with a carbohydrate moiety of the antibody, including but not limited to amino acids at positions 241, 243, 244, 245, 245, 249, 256, 258, 260, 262, 264, 265, 296, 299, and 301. Amino acids that directly or indirectly interact with a carbohydrate moiety of an antibody are known in the art, *see, e.g.,* Jefferis *et al.,* 1995 *Immunology Letters,* 44: 111-7, which is incorporated herein by reference in its entirety.

Antibodies can be modified by introducing one or more glycosylation sites into one or more sites of the antibodies, preferably without altering the functionality of the antibody, e.g., binding activity to CD32a or CD32b. Glycosylation sites may be introduced into the variable and/or constant region of the antibodies of the invention. As used herein, "glycosylation sites" include any specific amino acid sequence in an antibody to which an oligosaccharide (i.e., carbohydrates containing two or more simple sugars linked together) will specifically and covalently attach. Oligosaccharide side chains are typically linked to the backbone of an antibody via either N-or O-linkages. N-linked glycosylation refers to the attachment of an oligosaccharide moiety to the side chain of an asparagine residue. O-linked glycosylation refers to the attachment of an oligosaccharide moiety to a hydroxyamino acid, e.g., serine, threonine. The antibodies may comprise one or more glycosylation sites, including N-linked and O-linked glycosylation sites. Any glycosylation site for N-linked or O-linked glycosylation known in the art may be used in accordance with the instant invention. An exemplary N-linked glycosylation site that is useful in accordance with the methods of the present invention, is the amino acid sequence: Asn-X-Thr/Ser, wherein X may be any amino acid and Thr/Ser indicates a threonine or a serine. Such a site or sites may be introduced into an antibody of the invention using methods well known in the art to which this invention pertains. *See,* for example, "In Vitro Mutagenesis," Recombinant DNA: A Short Course, J. D. *Watson, et al.* W.H. Freeman and Company, New York, 1983, chapter 8, pp. 106-116, which is incorporated herein by reference in its entirety. An exemplary method for introducing a glycosylation site into an antibody of the invention may comprise: modifying or mutating an amino acid sequence of the antibody so that the desired Asn-X-Thr/Ser sequence is obtained.

The carbohydrate content of an antibody that can be used with the methods of the invention can be modified by adding or deleting a glycosylation site. Methods for modifying the carbohydrate content of antibodies are well known in the art and encompassed within the invention, *see, e.g.,* U.S. Patent No. 6,218,149; EP 0 359 096 B1; U.S. Publication No. US 2002/0028486; WO 03/035835; U.S. Publication No. 2003/0115614; U.S. Patent No. 6,218,149; U.S. Patent No. 6,472,511; all of which are incorporated herein by reference in their entirety. In other embodiments, the carbohydrate content of an antibody can be modified by deleting one or more endogenous carbohydrate moieties of the antibody.

Standard techniques known to those skilled in the art can be used to introduce mutations in the nucleotide sequence encoding an antibody, or fragment thereof, including, e.g., site-directed mutagenesis and PCR-mediated mutagenesis, which results in amino acid substitutions. Preferably, the derivatives include less than 15 amino acid substitutions, less than 10 amino acid substitutions, less than 5 amino acid substitutions, less than 4 amino acid substitutions, less than 3 amino acid substitutions, or less than 2 amino acid substitutions relative to the original antibody or fragment thereof. In a preferred embodiment, the derivatives have conservative amino acid substitutions made at one or more predicted non-essential amino acid residues.

### 5.6.1 ANTIBODY CONJUGATES

Antibodies that are recombinantly fused to heterologous polypeptides (i.e., an unrelated polypeptide; or portion thereof, preferably at least 10, at least 20, at least 30, at least 40, at least 50, at least 60, at least 70, at least 80, at least 90 or at least 100 amino acids of the polypeptide) or chemically conjugated to a moiety (including both covalently and non-covalently conjugations) can be used with the methods of the invention. The fusion/conjugation does not necessarily need to be direct, but may occur through linker sequences. *See e.g.,* PCT publication Number WO 93/2 1232; EP 439,095; Naramura *et al., Immunol. Lett.,* 39:91-99, 1994; U.S. Patent 5,474,981; Gillies *et al., PNAS,* 89:1428-1432, 1992; and Fell *et al., J. Immunol.,* 146:2446-2452, 1991, which are incorporated herein by reference in their entireties.

In certain embodiments, fusion to a heterologous polypeptide or conjugation to a moiety provides bulk to facilitate blockage of ligation by the antibody.

Further, an antibody may be conjugated to a therapeutic agent or drug moiety that modifies a given biological response. Therapeutic agents or drug moieties are not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin *(i.e.,* PE-40), or diphtheria toxin, ricin, gelonin, and pokeweed antiviral protein, a protein such as tumor necrosis factor, interferons including, but not limited to, α-interferon (IFN-α), β-interferon (IFN-β), nerve growth factor (NGF), platelet derived growth factor (PDGF), tissue plasminogen activator (TPA), an apoptotic agent (e.g., TNF-α, TNF-β, AIM I as disclosed in PCT Publication No. WO 97/33899), AIM II (see, PCT Publication No. WO 97/34911), Fas Ligand (Takahashi *et al., J. Immunol.,* 6:1567-1574, 1994), and VEGI (PCT Publication No. WO 99/23105), a thrombotic agent or an anti-angiogenic agent (e.g., angiostatin or endostatin), or a biological response modifier such as, for example, a lymphokine (e.g., interleukin-1 ("IL-1"), interleukin-2 ("IL-2"), interleukin-6 ("IL-6"), granulocyte macrophage colony stimulating factor ("GM-CSF"), and granulocyte colony stimulating factor ("G-CSF")), macrophage colony stimulating factor, ("M-CSF"), or a growth factor (*e.g.*, growth hormone ("GH"); proteases, or ribonucleases.

Antibodies can be fused to marker sequences, such as a peptide to facilitate purification. In preferred embodiments, the marker amino acid sequence is a hexa-histidine peptide, such as the tag provided in a pQE vector (QIAGEN, Inc., 9259 Eton Avenue, Chatsworth, CA, 91311), among others, many of which are commercially available. As described in Gentz *et al., Proc. Natl. Acad. Sci. USA,* 86:821-824, 1989, for instance, hexa-histidine provides for convenient purification of the fusion protein. Other peptide tags useful for purification include, but are not limited to, the hemagglutinin "HA" tag, which corresponds to an epitope derived from the influenza hemagglutinin protein *(*Wilson *et al., Cell,* 37:767 1984) and the FLAG-tag (Knappik *et al., Biotechniques,* 17(4):754-761, 1994).

Heterologous polypeptides may be fused or conjugated to a Fab fragment, Fd fragment, Fv fragment, F(ab)₂ fragment, or portion thereof. Methods for fusing or conjugating polypeptides to antibody portions are known in the art*. See, e.g.,* U.S. Patent Nos. 5,336,603, 5,622,929, 5,359,046, 5,349,053, 5,447,851, and 5,112,946; EP 307,434; EP 367,166; International Publication Nos. WO 96/04388 and WO 91/06570; Ashkenazi *et al.,* 1991, *PNAS* 88: 10535-10539; Zheng *et al.,* 1995, *J. Immunol.* 154:5590-5600; and Vil *et al.,* 1992, *PNAS* 89:11337- 11341 (said references incorporated by reference in their entireties).

Additional fusion proteins may be generated through the techniques of gene-shuffling, motif-shuffling, exon-shuffling, and/or codon-shuffling (collectively referred to as "DNA shuffling"). DNA shuffling may be employed to alter the activities of antibodies of the invention or fragments thereof (e.g., antibodies or fragments thereof with higher affinities and lower dissociation rates). *See,* generally, U.S. Patent Nos. 5,605,793; 5,811,238; 5,830,721; 5,834,252; and 5,837,458, and Patten *et al.,* 1997, *Curr. Opinion Biotechnol.* 8:724-33; Harayama, 1998, *Trends Biotechnol.* 16:76; Hansson, *et al.,* 1999, *J. Mol. Biol.* 287:265; and Lorenzo and Blasco, 1998, *BioTechniques* 24:308 (each of these patents and publications are hereby incorporated by reference in its entirety). Antibodies or fragments thereof, or the encoded antibodies or fragments thereof, may be altered by being subjected to random mutagenesis by error-prone PCR, random nucleotide insertion or other methods prior to recombination. One or more portions of a polynucleotide encoding an antibody or antibody fragment may be recombined with one or more components, motifs, sections, parts, domains, fragments, *etc.* of one or more heterologous molecules.

Techniques for conjugating additional moieties to antibodies are well known; *see, e.g.,* Arnon *et al.,* "Monoclonal Antibodies For Immunotargeting Of Drugs In Cancer Therapy", in *Monoclonal Antibodies And Cancer Therapy,* Reisfeld *et al.* (eds.), 1985, pp. 243-56, Alan R. Liss, Inc.); Hellstrom *et al.,* "Antibodies For Drug Delivery", in *Controlled Drug Delivery (2nd Ed.),* Robinson *et al.* (eds.), 1987, pp. 623-53, Marcel Dekker, Inc.); Thorpe, "Antibody Carriers Of Cytotoxic Agents In Cancer Therapy: A Review", in *Monoclonal Antibodies '84: Biological And Clinical Applications,* Pinchera *et al.* (eds.), 1985, pp. 475-506); "Analysis, Results, And Future Prospective Of The Therapeutic Use Of Radiolabeled Antibody In Cancer Therapy", in *Monoclonal Antibodies For Cancer Detection And Therapy,* Baldwin *et al.* (eds.), 1985, pp. 303-16, Academic Press; and Thorpe *et al., Immunol. Rev.,* 62:119-58, 1982.

An antibody can be conjugated to a second antibody to form an antibody heteroconjugate as described by Segal in U.S. Patent No. 4,676,980, which is incorporated herein by reference in its entirety.

Antibodies may also be attached to solid supports, which are particularly useful for immunoassays or purification of the target antigen. Such solid supports include, but are not limited to, glass, cellulose, polyacrylamide, nylon, polystyrene, polyvinyl chloride or polypropylene.

### 5.7 AGONISTS OF INHIBITORY Fc GAMMA RECEPTOR SIGNALING

Any agent that preferentially activates the signaling cascade downstream of inhibitory Fc gamma receptors, e.g., downstream of CD32b, over activating Fcγ receptor signaling can be used with the methods and compositions of the invention. In certain aspects, the agent that preferentially activates inhibitory Fcγ receptor signaling over activating Fcγ receptor signaling activates inhibitory Fcγ receptor signaling at least 2 times, 5 times, 10 times, 50 times, 100 times, 500 times, 1000 times, 5000 times, 10,000 times, 50,000 times, or at least 100,000 times more effectively than activating Fcγ receptor signaling. In certain aspects, the agent that preferentially activates inhibitory Fcγ receptor signaling over activating Fcγ receptor signaling activates inhibitory Fcγ receptor signaling at most 2 times, 5 times, 10 times, 50 times, 100 times, 500 times, 1000 times, 5000 times, 10,000 times, 50,000 times, or at most 100,000 times more effectively than activating Fcγ receptor signaling. In a specific embodiment, the agent specifically activates inhibitory Fcγ receptor signaling.

The agent that preferentially activates inhibitory Fcγ receptor signaling over activating Fcγ receptor signaling can be, without being limited to, a peptide, a nucleic acid, a protein, a small organic molecule, a sugar, a lipid, or an antibody.

In a specific embodiment, an agent that preferentially activates the signaling cascade downstream of inhibitory Fc gamma receptors over activating Fcγ receptor signaling is a c-reactive protein.

### 5.8 AGONISTS OF ACTIVATING Fc GAMMA RECEPTOR SIGNALING

Any agent that preferentially activates the signaling cascade downstream of activating Fc gamma receptors, e.g., downstream of CD32b, over inhibitory Fcγ receptor signaling can be used with the methods and compositions of the invention. In certain aspects, the agent that preferentially activates activating Fcγ receptor signaling over inhibitory Fcγ receptor signaling activates activating Fcγ receptor signaling at least 2 times, 5 times, 10 times, 50 times, 100 times, 500 times, 1000 times, 5000 times, 10,000 times, 50,000 times, or at least 100,000 times more effectively than inhibitory Fcγ receptor signaling. In certain aspects, the agent that preferentially activates activating Fcγ receptor signaling over inhibitory Fcγ receptor signaling activates activating Fcγ receptor signaling at most 2 times, 5 times, 10 times, 50 times, 100 times, 500 times, 1000 times, 5000 times, 10,000 times, 50,000 times, or at most 100,000 times more effectively than inhibitory Fcγ receptor signaling. In a specific embodiment, the agent specifically activates activating Fcγ receptor signaling.

The agent that preferentially activates activating Fcγ receptor signaling over inhibitory Fcγ receptor signaling can be, without being limited to, a peptide, a nucleic acid, a protein, a small organic molecule, a sugar, a lipid, or an antibody.

In a specific embodiment, an agonist of CD32a signaling is the monoclonal antibody C1KM5.

### 5.9 SCREENING METHODS

In certain embodiments, the invention provides screening methods for identifying a molecule that preferentially blocks activating Fc gamma receptors over inhibitory Fc gamma receptors. For the identification of such molecules, an immature dendritic cell that co-expresses activating Fc gamma receptors and inhibitory Fc gamma receptors is contacted with a test molecule. Subsequently, the dendritic cell is contacted with immobilized IgG and the degree of maturation of the dendritic cell is measured. A molecule that blocks activating Fc gamma receptors preferentially over inhibitory Fc gamma receptors is identified if the dendritic cell is less mature in the presence of the molecule as compared to a control dendritic cell in the absence of the molecule. In certain embodiments, the invention provides screening methods for identifying a molecule that blocks CD32a receptor more than CD32b receptor. For the identification of such molecules, an immature dendritic cell that co-expresses CD32a and CD32b is contacted with a test molecule. Subsequently, the dendritic cell is contacted with immobilized IgG and the degree of maturation of the dendritic cell is measured. A molecule that blocks CD32a preferentially over CD32b is identified if the dendritic cell is less mature in the presence of the molecule as compared to a control dendritic cell in the absence of the molecule. In certain embodiments, the screening assay is conducted with a population of dendritic cells, such that the population of dendritic cells is contacted with the test molecule. The percentage of mature dendritic cells in the population may be the read-out if a population of dendritic cells is used for the screening assay.

In certain embodiments, the invention provides screening methods for identifying a molecule that preferentially blocks inhibitory Fc gamma receptors over activating Fc gamma receptors. For the identification of such molecules, an immature dendritic cell that co-expresses activating Fc gamma receptors and inhibitory Fc gamma receptors is contacted with a test molecule. Subsequently, the dendritic cell is contacted with immobilized IgG and the degree of maturation of the dendritic cell is measured. A molecule that blocks inhibitory Fc gamma receptors preferentially over activating Fc gamma receptors is identified if the dendritic cell is more mature in the presence of the molecule as compared to a control dendritic cell in the absence of the molecule. In certain embodiments, the invention provides screening methods for identifying a molecule that blocks CD32b receptor more than CD32a receptor. For the identification of such molecules, an immature dendritic cell that co-expresses CD32a and CD32b is contacted with a test molecule. Subsequently, the dendritic cell is contacted with immobilized IgG and the degree of maturation of the dendritic cell is measured. A molecule that blocks CD32b preferentially over CD32a is identified if the dendritic cell is more mature in the presence of the molecule as compared to a control dendritic cell in the absence of the molecule. In certain embodiments, the screening assay is conducted with a population of dendritic cells, such that the population of dendritic cells is contacted with the test molecule. The percentage of mature dendritic cells in the population may be the read-out if a population of dendritic cells is used for the screening assay.

In the screening assays of the invention, the degree of maturation of a dendritic cell can be determined by measuring the expression levels of, e.g., the markers CD83 and/or ILT3. Maturation of dendritic cells is demonstrated by phenotypic changes such as, but not limited to, the upregulation of CD83 and downregulation of ILT3. Inhibition of maturation of a dendritic cell is demonstrated by phenotypic changes such as, but not limited to, an increase in ILT3 expression. To facilitate screening, reporter genes that are under the control of the CD83 promoter and/or the ILT3 promoter can be used. For example, a first construct harbors a first reporter gene under the control of the CD83 promoter and a second construct harbors a second reporter gene under the control of the ILT3 promoter. The two constructs are transfected into dendritic cells and the dendritic cells are then subjected to a screening method of the invention. To evaluate maturation of the dendritic cells, expression levels of the first reporter gene relative to the second reporter gene are measured. An increase in the ratio of the first reporter gene (under CD83 control) relative to the second reporter gene (under ILT3 control) demonstrates activation of maturation of the dendritic cell. A decrease in the ratio of the first reporter gene (under CD83 control) relative to the second reporter gene (under ILT3 control) demonstrates inhibition of maturation of the dendritic cell. If the proteins that are encoded by the first and the second reporter genes are fluorescent proteins with differing emission and/or absorption spectra, FACS can be used directly to determine the differentiation state of the dendritic cells.

In certain other embodiments, the degree of maturation of a dendritic cell can be determined by measuring the levels of inflammatory cytokines that are secreted by the dendritic cell. Exemplary inflammatory cytokines, the secretion of which can be determined, include IL-8 and TNF-alpha. Increased secretion of inflammatory cytokines, such as IL-8 or TNF-alpha, demonstrate activated maturation of the dendritic cells. Reporter genes, the transcription of which is under the control of the promoter of a cytokine, can be used to facilitate the screening procedure.

The invention also relates to methods for identifying a molecule that modifies the ratio of activating Fc gamma receptor expression relative to inhibitory Fc gamma receptor expression on a dendritic cell. To identify such molecules, an immature dendritic cell that co-expresses activating Fc gamma receptors and inhibitory Fc gamma receptors is contacted with a test molecule. Subsequently, the ratio of activating Fc gamma receptors relative to inhibitory Fc gamma receptors expressed on the cell surface of the dendritic cell is measured. The molecule modifies the ratio of activating Fc gamma receptors relative to inhibitory Fc gamma receptors on the surface of a dendritic cell if the ratio of activating Fc gamma receptors to inhibitory Fc gamma receptors on the dendritic cell in the presence of the test molecule is different from the ratio of activating Fc gamma receptors to inhibitory Fc gamma receptors expression on a dendritic cell in the absence of the molecule. In certain embodiments, the screening assay is conducted with a population of dendritic cells, such that the population of dendritic cells is contacted with the test molecule. The percentage of dendritic cells in the population with an altered ratio of activating Fc gamma receptors relative to inhibitory Fc gamma receptors may be the read-out if a population of dendritic cells is used for the screening assay. The invention also relates to methods for identifying a molecule that modifies the ratio of CD32a expression relative to CD32b expression on a dendritic cell. To identify such molecules, an immature dendritic cell that co-expresses CD32a and CD32b is contacted with a test molecule. Subsequently, the ratio of CD32a relative to CD32b expressed on the cell surface of the dendritic cell is measured. The molecule modifies the ratio of CD32a relative to CD32b on the surface of a dendritic cell if the ratio of CD32a to CD32b on the dendritic cell in the presence of the test molecule is different from the ratio of CD32a to CD32b expression on a dendritic cell in the absence of the molecule.

The screening methods of the invention may also be used to identify molecules that activate the signaling events downstream of activating Fc gamma receptors, such as CD32a. For example, a population of dendritic cells are (i) contacted with an agent that blocks inhibitory Fc gamma receptors; (ii) contacted with a test molecule; and (iii) maturation of the dendritic cells is measured. If the percentage of matured dendritic cells in the population is higher in the presence of the test molecule compared to the percentage of matured dendritic cells in the absence of the test molecule, the test molecule is identified as an activator of the signaling events downstream of activating Fc gamma receptor.

Conversely, the screening methods of the invention may also be used to identify molecules that activate the signaling events downstream of inhibitory Fc gamma receptors, such as CD32b. For example, a population of dendritic cells are (i) contacted with an agent that blocks activating Fc gamma receptors; (ii) contacted with a test molecule; and (iii) maturation of the dendritic cells is measured. If the percentage of matured dendritic cells in the population is higher in the absence of the test molecule compared to the percentage of matured dendritic cells in the presence of the test molecule, the test molecule is identified as an activator of the signaling events downstream of inhibitory Fc gamma receptor.

### 5.10 GENERATION AND USE OF TOLEROGENIC DENDRITIC CELLS

The methods of inhibiting the maturation of dendritic cells (see section 5.1) can be used to produce tolerogenic dendritic cells. The tolerogenic dendritic cells can for example be used to treat graft-versus-host disease. The tolerogenic cells can also be administered to a subject to treat an autoimmune disease. Exemplary autoimmue diseases include: alopecia areata, ankylosing spondylitis, antiphospholipid syndrome, autoimmune Addison's disease, autoimmune diseases of the adrenal gland, autoimmune hemolytic anemia, autoimmune hepatitis, autoimmune oophoritis and orchitis, autoimmune thrombocytopenia, Behcet's disease, bullous pemphigoid, cardiomyopathy, celiac sprue-dermatitis, chronic fatigue immune dysfunction syndrome (CFIDS), chronic inflammatory demyelinating polyneuropathy, Churg-Strauss syndrome, cicatrical pemphigoid, CREST syndrome, cold agglutinin disease, Crohn's disease, discoid lupus, essential mixed cryoglobulinemia, fibromyalgia-fibromyositis, glomerulonephritis, Graves' disease, Guillain-Barre, Hashimoto's thyroiditis, idiopathic pulmonary fibrosis, idiopathic thrombocytopenia purpura (ITP), IgA neuropathy, juvenile arthritis, lichen planus, lupus erthematosus, Ménière's disease, mixed connective tissue disease, multiple sclerosis, type 1 or immune-mediated diabetes mellitus, myasthenia gravis, pemphigus vulgaris, pernicious anemia, polyarteritis nodosa, polychrondritis, polyglandular syndromes, polymyalgia rheumatica, polymyositis and dermatomyositis, primary agammaglobulinemia, primary biliary cirrhosis, psoriasis, psoriatic arthritis, Raynauld's phenomenon, Reiter's syndrome, Rheumatoid arthritis, sarcoidosis, scleroderma, Sjögren's syndrome, stiff-man syndrome, systemic lupus erythematosus, lupus erythematosus, takayasu arteritis, temporal arteristis/ giant cell arteritis, ulcerative colitis, uveitis, vasculitides such as dermatitis herpetiformis vasculitis, vitiligo, or Wegener's granulomatosis.

In certain embodiments, the invention provides methods for producing antigen-specific tolerogenic dendritic cells. To generate antigen-specific tolerogenic dendritic cells, dendritic cells are subjected to a method of the invention to inhibit the maturation of the dendritic cells, and subsequently or concurrently, the dendritic cells are contacted with one or more antigens against which tolerance is desired. If antigen-specific tolerogenic dendritic cells are to be used to treat an autoimmune disease, the antigen or antigens are the antigen(s) that cause(s) the immune reaction that underlies the autoimmune disease. In certain, more specific embodiments, the tolerogenic dendritic cells *(i.e.,* dendritic cells, the maturation of which has been inhibited by a method of the invention) are contacted with a plurality of different antigens to produce a population of antigen-specific tolerogenic dendritic cells. In other embodiments, for example if the tolerogenic dendritic cells are to be used to treat graft-versus-host disease, tolerogenic dendritic cells (i.e., dendritic cells, the maturation of which has been inhibited by a method of the invention) are contacted with tissue from the graft, wherein the tissue is complexed with or bound to antibody to produce a population of tolerogenic dendritic cells specific for graft antigens.

In certain embodiments, antigen-specific tolerogenic dendritic cells can be generated by (a) contacting an immature dendritic cell with an agent that blocks ligation of activating Fc gamma receptors, e.g., an agent that blocks ligation of CD32a, and (b) exposing the cell to an antigen complexed IgG, wherein the antigen is the antigen against which tolerance is desired, e.g., a self-antigen. In a specific embodiment, steps (a) and (b) are performed sequentially, such that step (a) is performed first followed by step (b). In one aspect, a population of immature dendritic cells is first contacted with anti-CD32a antibody that blocks ligation of CD32a, and subsequently, the population of immature dendritic cells is contacted with one or more antigens complexed with IgG.

In other embodiments, antigen-specific tolerogenic dendritic cells can be generated by contacting an immature dendritic cell with an antibody against inhibitory Fc gamma receptors, e.g., an antibody against CD32b, wherein the antibody is conjugated to an antigen. The antigen is the antigen against which tolerance is desired, e.g., a self-antigen. Optionally, signaling by activating Fc gamma receptors, e.g., signaling by CD32a, can be inhibited. In a specific embodiment, monoclonal antibody 2B6 conjugated to an antigen can be used. Without being bound by theory, the antibody-antigen conjugate is internalized into the dendritic cell.

The dendritic cells that are being used as starting material for the methods of the invention can be autologous to the subject that is to be treated. In other embodiments, the dendritic cells that are being used as starting material for the methods of the invention are heterologous dendritic cells. For example, if graft-versus-host disease is to be treated, the dendritic cells that are being used as starting material are dendritic cells that were obtained from the donor.

The subject can be, e.g., a mouse, a rat, a dog, a chicken, a horse, a goat, a donkey, or a primate. Most preferably, the subject is a human.

### 5.10.1 AUTOIMMUNE DISEASE AND INFLAMMATORY DISEASES

Tolerogenic dendritic cells that have been generated with the methods of the invention may be used to treat or prevent autoimmune diseases or inflammatory diseases. The present invention provides methods of preventing, treating, or managing one or more symptoms associated with an autoimmune or inflammatory disorder in a subject, comprising administering to said subject a therapeutically effective amount of the antibodies or fragments thereof of the invention. The invention also provides methods for preventing, treating, or managing one or more symptoms associated with an inflammatory disorder in a subject further comprising, administering to said subject a therapeutically effective amount of one or more anti-inflammatory agents. The invention also provides methods for preventing, treating, or managing one or more symptoms associated with an autoimmune disease further comprising, administering to said subject a therapeutically effective amount of one or more immunomodulatory agents.

Tolerogenic dendritic cells that have been generated with the methods of the invention can also be used in combination with any of the antibodies known in the art for the treatment and/or prevention of autoimmune disease or inflammatory disease. A non-limiting example of the antibodies that are used for the treatment or prevention of inflammatory disorders is presented in Table 2A, and a non-limiting example of the antibodies that are used for the treatment or prevention of autoimmune disorder is presented in Table 2B. Tolerogenic dendritic cells that have been generated with the methods of the invention can for example, enhance the efficacy of treatment of the therapeutic antibodies presented in Tables 2A and 3B. For example, but not by way of limitation, tolerogenic dendritic cells that have been generated with the methods of the invention can enhance the immune response in the subject being treated with any of the antibodies in Tables 2A or 3B.

Tolerogenic dendritic cells that have been generated with the methods of the invention can also be used in combination with for example but not by way of limitation, Orthoclone OKT3, ReoPro, Zenapex, Simulec, Synagis, and Remicade.

Tolerogenic dendritic cells that have been generated with the methods of the invention can also be used in combination with cytosine-guanine dinucleotides ("CpG")-based products that have been developed (Coley Pharmaceuticals) or are currently being developed as activators of innate and acquired immune responses. For example, the invention encompasses the use of CpG 7909, CpG 8916, CpG 8954 (Coley Pharmaceuticals) in the methods and compositions of the invention for the treatment and/or prevention of autoimmune or inflammatory disorders (Weeratna *et al.,* 2001, *FEMS Immunol Med Microbiol.,* 32(1):65-71, which is incorporated herein by reference).

Examples of autoimmune disorders that may be treated by administering the antibodies of the present invention include, but are not limited to, alopecia areata, ankylosing spondylitis, antiphospholipid syndrome, autoimmune Addison's disease, autoimmune diseases of the adrenal gland, autoimmune hemolytic anemia, autoimmune hepatitis, autoimmune oophoritis and orchitis, autoimmune thrombocytopenia, Behcet's disease, bullous pemphigoid, cardiomyopathy, celiac sprue-dermatitis, chronic fatigue immune dysfunction syndrome (CFIDS), chronic inflammatory demyelinating polyneuropathy, Churg-Strauss syndrome, cicatrical pemphigoid, CREST syndrome, cold agglutinin disease, Crohn's disease, discoid lupus, essential mixed cryoglobulinemia, fibromyalgia-fibromyositis, glomerulonephritis, Graves' disease, Guillain-Barre, Hashimoto's thyroiditis, idiopathic pulmonary fibrosis, idiopathic thrombocytopenia purpura (ITP), IgA neuropathy, juvenile arthritis, lichen planus, lupus erthematosus, Ménière's disease, mixed connective tissue disease, multiple sclerosis, type 1 or immune-mediated diabetes mellitus, myasthenia gravis, pemphigus vulgaris, pernicious anemia, polyarteritis nodosa, polychrondritis, polyglandular syndromes, polymyalgia rheumatica, polymyositis and dermatomyositis, primary agammaglobulinemia, primary biliary cirrhosis, psoriasis, psoriatic arthritis, Raynauld's phenomenon, Reiter's syndrome, Rheumatoid arthritis, sarcoidosis, scleroderma, Sjögren's syndrome, stiff-man syndrome, systemic lupus erythematosus, lupus erythematosus, takayasu arteritis, temporal arteristis/giant cell arteritis, ulcerative colitis, uveitis, vasculitides such as dermatitis herpetiformis vasculitis, vitiligo, and Wegener's granulomatosis. Examples of inflammatory disorders include, but are not limited to, asthma, encephilitis, inflammatory bowel disease, chronic obstructive pulmonary disease (COPD), allergic disorders, septic shock, pulmonary fibrosis, undifferentiated spondyloarthropathy, undifferentiated arthropathy, arthritis, inflammatory osteolysis, and chronic inflammation resulting from chronic viral or bacteria infections. Some autoimmune disorders are associated with an inflammatory condition. Thus, there is overlap between what is considered an autoimmune disorder and an inflammatory disorder. Therefore, some autoimmune disorders may also be characterized as inflammatory disorders. Examples of inflammatory disorders which can be prevented, treated or managed in accordance with the methods of the invention include, but are not limited to, asthma, encephilitis, inflammatory bowel disease, chronic obstructive pulmonary disease (COPD), allergic disorders, septic shock, pulmonary fibrosis, undifferentiated spondyloarthropathy, undifferentiated arthropathy, arthritis, inflammatory osteolysis, and chronic inflammation resulting from chronic viral or bacteria infections.

Tolerogenic dendritic cells that have been generated with the methods of the invention can also be used to reduce the inflammation experienced by animals, particularly mammals, with inflammatory disorders. In a specific embodiment, tolerogenic dendritic cells that have been generated with the methods of the invention reduces the inflammation in an animal by at least 99%, at least 95%, at least 90%, at least 85%, at least 80%, at least 75%, at least 70%, at least 60%, at least 50%, at least 45%, at least 40%, at least 45%, at least 35%, at least 30%, at least 25%, at least 20%, or at least 10% relative to the inflammation in an animal in the absence of administration of tolerogenic dendritic cells that have been generated with the methods of the invention.

Tolerogenic dendritic cells that have been generated with the methods of the invention can also be used to prevent the rejection of transplants.

In one embodiment, the invention encompasses the use of tolerogenic dendrititc cells that have been generated using methods of the invention in combination with any allergy vaccine known in the art, such as, for example, in combination with recombinant hybrid molecules coding for the major timothy grass pollen allergens used for vaccination against grass pollen allergy, as described by Linhart *et al.* (2000, *FASEB Journal,* 16(10):1301-3, which is incorporated by reference). In addition the tolerogenic dendrititc cells that have been generated using methods of the invention can be used in combination with DNA-based vaccinations described by Horner *et al.* (2002, *Allergy, 57* Suppl, 72:24-9, which is incorporated by reference); with Bacille Clamett-Guerin ("BCG") vaccination as described by *Choi et al.* (2002, *Ann. Allergy Asthma Immunology,* 88(6): 584-91) and Barlan *et al.* (2002, *Journal Asthma,* 39(3):239-46), both of which are incorporated herein by reference in entirety, to downregulate IgE secretion; and with vaccines or other immunotherapies known in the art *(see* Hourihane *et al.,* 2002, *Curr. Opin. Allergy Clin. Immunol.* 2(3):227-31) for the treatment of peanut allergies.

**Table 2A: Antibodies for Inflammatory Diseases and Autoimmune Diseases that can be used in combination with the antibodies of the invention.**

| Antibody Name | Target Antigen | Product Type | Isotype | Sponsors | Indication |
|---|---|---|---|---|---|
| 5G1.1 | Complement (C5) | Humanised | IgG | Alexion Pharm Inc | Rheumatoid Arthritis |
| 5G1.1 | Complement (C5) | Humanised | IgG | Alexion Pharm Inc | SLE |
| 5G1.1 | Complement (C5) | Humanised | IgG | Alexion Pharm Inc | Nephritis |
| 5G1.1-SC | Complement (C5) | Humanised | ScFv | Alexion Pharm Inc | Cardiopulmano Bypass |
| SG1.1-SC | Complement (C5) | Humanised | ScFv | Alexion Pharm Inc | Myocardial Infarction |
| 5G1.1-SC | Complement (C5) | Humanised | ScFv | Alexion Pharm Inc | Angioplasty |
| ABX-CBL | CBL | Human | | Abgenix Inc | GvHD |
| ABX-CBL | CD 147 | Murine | IgG | Abgenix Inc | Allograft rejection |
| ABX-IL8 | IL-8 | Human | IgG2 | Abgenix Inc | Psoriasis |
| Antegren | VLA-4 | Humanised | IgG | Athena/Elan | Multiple Sclerosis |
| Anti-CD 11a | CD11a | Humanised | IgG1 | Genentech Inc/Xoma | Psoriasis |
| Anti-CD 18 | CD 18 | Humanised | Fab'2 | Genentech Inc | Myocardial infarction |
| Anti-LFA1 | CD18 | Murine | Fab'2 | Pasteur-Merieux/ Immunotech | Allograft rejection |
| Antova | CD40L | Humanised | IgG | Biogen | Allograft rejection |
| Antova | CD40L | Humanised | IgG | Biogen | SLE |
| BTI-322 | CD2 | Rat | IgG | Medimmune Inc | GvHD, Psoriasis |
| CDP571 | TNF-alpha | Humanised | IgG4 | Celltech | Crohn's |
| CDP571 | TNF-alpha | Humanised | IgG4 | Celltech | Rheumatoid Arthritis |
| CDP850 | E-selectin | Humanised | | Celltech | Psoriasis |
| Corsevin M | Fact VII | Chimeric | | Centocor | Anticoagulant |
| D2E7 | TNF-alpha | Human | | CATBASF | Rheumatoid Arthritis |
| Hu23F2G | CD11/18 | Humanised | | ICOS Pharm Inc | Multiple Sclerosis |
| Hu23F2G | CD11/18 | Humanised | IgG | ICOS Pharm Inc | Stroke |
| IC14 | CD 14 | | | ICOS Pharm Inc | Toxic shock |
| ICM3 | ICAM-3 | Humanised | | ICOS Pharm Inc | Psoriasis |
| IDEC-114 | CD80 | Primatised | | IDEC Pharm/Mitsubishi | Psoriasis |
| IDEC-131 | CD40L | Humanised | | IDEC Pharm/Eisai | SLE |
| IDEC-131 | CD40L | Humanised | | IDEC Pharm/Eisai | Multiple Sclerosis |
| IDEC-151 | CD4 | Primatised | IgG1 | IDEC Pharm/ GlaxoSmithKline | Rheumatoid Arthritis |
| IDEC-152 | CD23 | Primatised | | IDEC Pharm | Asthma/Allergy |
| Infliximab | TNF-alpha | Chimeric | IgG1 | Centocor | Rheumatoid Arthritis |
| Infliximab | TNF-alpha | Chimeric | IgG1 | Centocor | Crohn's |
| LDP-01 | beta2-integrin | Humanised | IgG | Millennium Inc (LeukoSite Inc.) | Stroke |
| LDP-01 | beta2-integrin | Humanised | IgG | Millennium Inc (LeukoSite Inc.) | Allograft rejection |
| LDP-02 | alpha4beta7 | Humanised | | Millennium Inc (LeukoSite Inc.) | Ulcerative Colitis |
| MAK-195F | TNF alpha | Murine | Fab'2 | Knoll Pharm, BASF | Toxic shock |
| MDX-33 | CD64 (FcR) | Human | | Medarex/Centeon | Autoimmune haematogical disorders |
| MDX-CD4 | CD4 | Human | IgG | Medarex/Eisai/ Genmab | Rheumatoid Arthritis |
| MEDI-507 | CD2 | Humanised | | Medimmune Inc | Psoriasis |
| MEDI-507 | CD2 | Humanised | | Medimmune Inc | GvHD |
| OKT4A | CD4 | Humanised | IgG | Ortho Biotech | Allograft rejection |
| OrthoClone OKT4A | CD4 | Humanised | IgG | Ortho Biotech | Autoimmune disease |
| Orthoclone/ anti-CD3 OKT3 | CD3 | Murine | mIgG2 a | Ortho Biotech | Allograft rejection |
| RepPro/ Abciximab | gpIIbIIIa | Chimeric | Fab | Centocor/Lilly | Complications of coronary angioplasty |
| rhuMab-E25 | IgE | Humanised | IgG1 | Genentech/Novar tis/Tanox Biosystems | Asthma/Allergy |
| SB-240563 | IL5 | Humanised | | GlaxoSmithKline | Asthma/Allergy |
| SB-240683 | IL-4 | Humanised | | GlaxoSmithKline | Asthma/Allergy |
| SCH55700 | IL-5 | Humanised | | Celltech/Schering | Asthma/Allergy |
| Simulect | CD25 | Chimeric | IgG1 | Novartis Pharm | Allograft rejection |
| SMART a-CD3 | CD3 | Humanised | | Protein Design Lab | Autoimmune disease |
| SMART a-CD3 | CD3 | Humanised | | Protein Design Lab | Allograft rejection |
| SMART a-CD3 | CD3 | Humanised | IgG | Protein Design Lab | Psoriasis |
| Zenapax | CD25 | Humanised | IgG1 | Protein Design Lab/Hoffman-La Roche | Allograft rejection |

**Table 2B: Antibodies for Autoimmune Disorders**

| Antibody | Indication | Target Antigen |
|---|---|---|
| ABX-RB2 | | antibody to CBL antigen on T cells, B cells and NK cells fully human antibody from the Xenomouse |
| IL1-ra | rheumatoid arthritis | recombinant anti-inflammatory protein |
| sTNF-RI | chronic inflammatory disease rheumatoid arthritis | soluble tumor necrosis factor a - receptor type I blocks TNF action |
| 5c8 (Anti CD-40 ligand antibody) | Phase II trials were halted in Oct. 99 examine "adverse events" | CD-40 |
| IDEC 131 | systemic lupus erythyematous (SLE) | anti CD40 humanized |
| IDEC 151 | rheumatoid arthritis | primatized ; anti-CD4 |
| IDEC 152 | asthma | primatized; anti-CD23 |
| IDEC 114 | psoriasis | primatized anti-CD80 |
| MEDI-507 | rheumatoid arthritis; multiple sclerosis Crohn's disease psoriasis | anti-CD2 |
| LDP-02 (anti-b7 mAb) | inflammatory bowel disease Chron's disease ulcerative colitis | a4b7 integrin receptor on white blood cells (leukocytes) |
| SMART Anti-Gamma Interferon antibody | autoimmune disorders | Anti-Gamma Interferon |
| Verteportin | rheumatoid arthritis | |
| Thalomid (thalidomide) | leprosy - approved for market Chron's disease rheumatoid arthritis | inhibitor of tumor necrosis factor alpha (TNF alpha) |
| SelCIDs (selective cytokine inhibitory drugs) | | highly specific inhibitors of phosphodiesterase type 4 enzyme (PDE-4) increases levels of cAMP (cyclic adenosine monophosphate) activates protein kinase A (PKA) blocks transcription factor NK-kB prevents transcription of TNF-a gene decreases production of TNF-a |
| IMiDs (immunomodulator y drugs) | general autoimmune disorders | structural analogues of thalidomideinhibit TNF-a |
| MDX-33 | blood disorders caused by autoimmune reactions Idiopathic Thrombocytopenia Purpurea (ITP) autoimmune hemolytic anemia | monoclonal antibody against FcRI receptors |
| MDX-CD4 | treat rheumatoid arthritis and other autoimmunity | monoclonal antibody against CD4 receptor molecule |
| VX-497 | autoimmune disorders multiple sclerosis rheumatoid arthritis inflammatory bowel disease lupus psoriasis | inhibitor of inosine monophosphate dehydrogenase (enzyme needed to make new RNA and DNA used in production of nucleotides needed for lymphocyte proliferation) |
| VX-740 | rheumatoid arthritis | inhibitor of ICE interleukin-1 beta (converting enzyme controls pathways leading to aggressive immune response regulates cytokines) |
| VX-745 | specific to inflammation involved in chemical signaling of immune response onset and progression of inflammation | inhibitor of P38MAP kinase mitogen activated protein kinase |
| Enbrel (etanercept) | | targets TNF (tumor necrosis factor) |
| IL-8 | | fully human MAB against IL-8 (interleukin 8) (blocks IL-8 blocks inflammatory response) |
| 5G1.1 | rheumatoid arthritis pemphigoid (dangerous skin rash) psoriasis lupus | a C5 complement inhibitor |
| Apogen MP4 | | recombinant antigen selectively destroys disease associated T-cells induces apoptosis T-cells eliminated by programmed cell death no longer attack body's own cells specific apogens target specific T-cells |

| Company Rankings | Product | Development Stage |
|---|---|---|
| Immunex | Enbrel | on market |
| Amgen | IL1-ra, sTNF-RI | Phase II/III |
| Abgenix | AGX-RB2, IL-8 | preclinical, Phase I |
| Alexion | 5G1.1 , Apogen MP4 | Phase II, preclinical |
| Biogen | 5c8 | Phase II (halted) |
| IDEC | 131, 151, 152, 114 | Phase I and II |
| MedImmune | MEDI 507 | Phase I/II |
| Millennium | LDP-02, | Phase II |
| Protein Design Labs | Anti-Gamma Interferon | preclinical |
| Medarex | MDX-33, MDX-CD4 | Phase II, Phase I |
| QLT PhotoTherapeutics | Verteportin | Phase I |
| Celegene | Thalomid, Se1CIDs, IMiDs | on market, preclinical |
| Vertex | VX-497, VX-740, VX-745 | Phase II, Phase II, Phase II |

### 5.10.2 IMMUNOMODULATORY AGENTS AND ANTI-INFLAMMATORY AGENTS

The present invention provides methods of treatment for autoimmune diseases and inflammatory diseases comprising administration of tolerogenic dendritic cells that have been generated using the methods of the invention in conjunction with other treatment agents. Examples of immunomodulatory agents include, but are not limited to, methothrexate, ENBREL, REMICADE™, leflunomide, cyclophosphamide, cyclosporine A, and macrolide antibiotics (e.g., FK506 (tacrolimus)), methylprednisolone (MP), corticosteroids, steriods, mycophenolate mofetil, rapamycin (sirolimus), mizoribine, deoxyspergualin, brequinar, malononitriloamindes (e.g., leflunamide), T cell receptor modulators, and cytokine receptor modulators.

Anti-inflammatory agents have exhibited success in treatment of inflammatory and autoimmune disorders and are now a common and a standard treatment for such disorders. Any anti-inflammatory agent well-known to one of skill in the art can be used in the methods of the invention. Non-limiting examples of anti-inflammatory agents include non-steroidal anti-inflammatory drugs (NSAIDs), steroidal anti-inflammatory drugs, beta-agonists, anticholingeric agents, and methyl xanthines. Examples of NSAIDs include, but are not limited to, aspirin, ibuprofen, celecoxib (CELEBREX™), diclofenac (VOLTAREN™), etodolac (LODINE™), fenoprofen (NALFON™), indomethacin (INDOCIN™), ketoralac (TORADOL™), oxaprozin (DAYPRO™), nabumentone (RELAFEN™), sulindac (CLINORIL™), tolmentin (TOLECTIN™), rofecoxib (VIOXX™), naproxen (ALEVE™, NAPROSYN™), ketoprofen (ACTRON™) and nabumetone (RELAFEN™). Such NSAIDs function by inhibiting a cyclooxgenase enzyme (e.g., COX-1 and/or COX-2). Examples of steroidal anti-inflammatory drugs include, but are not limited to, glucocorticoids, dexamethasone (DECADRON™), cortisone, hydrocortisone, prednisone (DELTASONE™), prednisolone, triamcinolone, azulfidine, and eicosanoids such as prostaglandins, thromboxanes, and leukotrienes.

### 5.11 GENERATION AND USE OF MATURED DENDRITIC CELLS

The mature dendritic cells that have been generated by methods of the invention can be used as adjuvants in vaccines. The vaccine can be, e.g., a vaccine against cancer, a neoplastic disease, or an infectious disease.

Exemplary cancers and neoplastic diseases that can be treated using the methods of the invention are Leukemia, such as acute leukemia, acute T-cell leukemia, acute lymphocytic leukemia, acute myelocytic leukemia, myeloblastic leukemia, promyelocytic leukemia, myelomonocytic leukemia, Monocytic, erythroleukemia, chronic leukemia, chronic myelocytic (granulocytic) leukemia, and chronic lymphocytic leukemia; Polycythemia vera; Lymphoma, such as Hodgkin's disease, and non-Hodgkin's disease; Multiple myeloma; Waldenström's macroglobulinemia; Heavy chain disease; Solid tumors, such as sarcomas and carcinomas, fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, uterine cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma,neuroblastoma, and retinoblastoma.

The mature dendritic cells that have been generated by methods of the invention can be further pretreated such that the mature dendritic cells elicit an antigen-specific immune response. Accordingly, in certain embodiments, mature dendritic cells that have been generated with the methods of the invention will elicit an antigen-specific immune response. In some embodiments, the dendritic cells can be treated with an additional maturation stimulous, such as a cocktail of inflammatory cytokines (e.g., I11-β, I1-6, TNFα and PGE2) Jonuleit H., (1997) Eur J Immunol. 27:3135-3142.

In certain embodiments, antigen-specific mature dendritic cells can be generated by (a) contacting an immature dendritic cell with an agent that blocks ligation of inhibitory Fc gamma receptors, e.g., an agent that blocks ligation of CD32b, and (b) exposing the cell to an antigen complexed IgG, wherein the antigen is the antigen against which an immune reaction is desired. In a specific embodiment, steps (a) and (b) are performed sequentially, such that step (a) is performed first followed by step (b). In one aspect, a population of immature dendritic cells is first contacted with anti-CD32b antibody that blocks ligation of CD32b, and subsequently, the population of immature dendritic cells is contacted with one or more antigens complexed with IgG.

In other embodiments, antigen-specific mature dendritic cells can be generated by contacting an immature dendritic cell with an antibody against activating Fc gamma receptors, e.g., an antibody against CD32a, wherein the antibody is conjugated to an antigen. The antigen is the antigen against which an immune response is desired. Optionally, signaling by inhibitory Fc gamma receptors, e.g., signaling by CD32b, can be inhibited.

Antigen-specific mature dendritic cells can then be administered directly to a subject as a vaccine or, alternatively, such antigen-specific mature dendritic cells can be used to stimulate and/or expand antigen-specific T cells *ex vivo.* In certain embodiments, the antigen is an antigen that is associated with a cancer or a neoplastic disease. In another aspect, the antigen is specific to a cancer cell or a neoplastic cell. The antigen can also be an antigen of a pathogen, such as, e.g., a virus, a bacterium, or a protozoa. In specific embodiments, the T cell that is stimulated and/or exanded using the methods of the invention is CD4 positive or CD8 positive or is an NKT cell. In certain embodiments, T cells can be stimulated and/or expanded by co-culturing T cells with matured dendritic cells that have been generated using the methods of the invention.

In certain embodiments, T cells from a subject are educated *ex vivo* using the methods of the invention and the educated T cells are subsequently administered to the subject. In an illustrative embodiment, dendritic cells and T cells are isolated from a subject. Using the methods of the invention, maturation of the dendritic cells is promoted, the matured dendritic cells are subsequently targeted with an antigen against which an immune response is desired in the subject, and the antigen-specific dendritic cells are subsequently administered to the subject. The antigen can for example be a tumor-specific antigen or an antigen of a pathogen.

In one embodiment, the invention encompasses the use of the matured dendritic cells or T cells that have been generated using the methods of the invention in combination with any cancer vaccine known in the art, e.g., CanvaxinTM (Cancer Vax, Corporation, melanoma and colon cancer); Oncophage (HSPPC-96; Antigenics; metastatic melanoma); HER-2/neu cancer vaccine, *etc.* The invention encompasses the use of the matured dendritic cells or T cells that have been generated using the methods of the invention with cell-based vaccines as described by Segal *et al.* (US 6,403,080), which is incorporated herein by reference in its entirety. The cell based vaccines used in combination the matured dendritic cells or T cells that have been generated using the methods of the invention can be either autologous or allogeneic. Briefly, the cancer- based vaccines as described by Segal *et al.* are based on Opsonokine (TM) product by Genitrix, LLC. Opsonokines(TM) are genetically engineered cytokines that, when mixed with tumor cells, automatically attach to the surface of the cells. When the "decorated" cells are administered as a vaccine, the cytokine on the cells activates critical antigen presenting cells in the recipient, while also allowing the antigen presenting cells to ingest the tumor cells. The antigen presenting cells are then able to instruct "killer" T cells to find and destroy similar tumor cells throughout the body. Thus, the Opsonokine(TM) product converts the tumor cells into a potent anti-tumor immunotherapeutic.

The methods and compositions of the invention can be used in combination with vaccines, in which immunity for the antigen(s) is desired. Such antigens may be any antigen known in the art. The antibodies of the invention can be used to enhance an immune response, for example, to infectious agents, diseased or abnormal cells such as, but not limited to, bacteria (e.g., gram positive bacteria, gram negative bacteria, aerobic bacteria, Spirochetes, Mycobacteria, Rickettsias, Chlamydias, etc.), parasites, fungi *(e.g.,* Candida albicans, Aspergillus, etc.), viruses (e.g., DNA viruses, RNA viruses, *etc.),* or tumors. Viral infections include, but are not limited to, human immunodeficiency virus (HIV); hepatitis A virus, hepatitis B virus, hepatitis C virus, hepatitis D virus, or other hepatitis viruses; cytomagaloviruses, herpes simplex virus-1 (-2,-3,-4,-5,-6), human papilloma viruses; Respiratory syncytial virus (RSV), Parainfluenza virus (PIV), Epstein Barr virus, or any other viral infections.

The invention encompasses the use of the matured dendritic cells that have been generated using the methods of the invention to enhance a humoral and/or cell mediated response against the antigen(s) of the vaccine composition. The invention further encompasses the use of the matured dendritic cells that have been generated using the methods of the invention to either prevent or treat a particular disorder, where an enhanced immune response against a particular antigen or antigens is effective to treat or prevent the disease or disorder. Such diseases and disorders include, but are not limited to, viral infections, such as HIV, CMV, hepatitis, herpes virus, measles, etc., bacterial infections, fungal and parasitic infections, cancers, and any other disease or disorder amenable to treatment or prevention by enhancing an immune response against a particular antigen or antigens.

In certain embodiments, matured dendritic cells that have been generated using the methods of the invention can be used to elicit a humoral response in a subject by administering the dendritic cells to the subject. In an illustrative embodiment, immature dendritic cells are isolated from the subject, subjected to a method of the invention to promote maturation of the dendritic cells, and subsequently, the matured dendritic cells are administered to the subject. In one aspect, the humoral response comprises expression of IL6 and IL10.

### 5.11.1 CANCERS

Matured dendritic cells or T cells that have been treated with a method of the invention can be used alone or in combination with other therapeutic antibodies known in the art to prevent, inhibit or reduce the growth of primary tumores or metastasis of cancerous cells. In a specific embodiment, dendritic cells or T cells that have been treated with a method of the invention, when administered alone or in combination with a cytotoxic therapeutic antibody, inhibit or reduce the growth of primary tumor or metastasis of cancerous cells by at least 99%, at least 95%, at least 90%, at least 85%, at least 80%, at least 75%, at least 70%, at least 60%, at least 50%, at least 45%, at least 40%, at least 45%, at least 35%, at least 30%, at least 25%, at least 20%, or at least 10% relative to the growth of primary tumor or metastasis in absence dendritic cells or T cells that have been treated with a method of the invention.

In a preferred embodiment, dendritic cells or T cells that have been treated with a method of the invention in combination with a cytotoxic therapeutic antibody inhibit or reduce the growth of primary tumor or metastasis of cancer by at least 99%, at least 95%, at least 90%, at least 85%, at least 80%, at least 75%, at least 70%, at least 60%, at least 50%, at least 45%, at least 40%, at least 45%, at least 35%, at least 30%, at least 25%, at least 20%, or at least 10% relative to the growth or metastasis in absence of said dendritic cells or T cells that have been treated with a method of the invention.

Cancers and related disorders that can be treated or prevented by methods and compositions of the present invention include, but are not limited to, the following: Leukemias including, but not limited to, acute leukemia, acute lymphocytic leukemia, acute myelocytic leukemias such as myeloblastic, promyelocytic, myelomonocytic, monocytic, erythroleukemia leukemias and myelodysplastic syndrome, chronic leukemias such as but not limited to, chronic myelocytic (granulocytic) leukemia, chronic lymphocytic leukemia, hairy cell leukemia; polycythemia vera; lymphomas such as but not limited to Hodgkin's disease, non-Hodgkin's disease; multiple myelomas such as but not limited to smoldering multiple myeloma, nonsecretory myeloma, osteosclerotic myeloma, plasma cell leukemia, solitary plasmacytoma and extramedullary plasmacytoma; Waldenström's macroglobulinemia; monoclonal gammopathy of undetermined significance; benign monoclonal gammopathy; heavy chain disease; bone and connective tissue sarcomas such as but not limited to bone sarcoma, osteosarcoma, chondrosarcoma, Ewing's sarcoma, malignant giant cell tumor, fibrosarcoma of bone, chordoma, periosteal sarcoma, soft-tissue sarcomas, angiosarcoma (hemangiosarcoma), fibrosarcoma, Kaposi's sarcoma, leiomyosarcoma, liposarcoma, lymphangiosarcoma, neurilemmoma, rhabdomyosarcoma, synovial sarcoma; brain tumors including but not limited to, glioma, astrocytoma, brain stem glioma, ependymoma, oligodendroglioma, nonglial tumor, acoustic neurinoma, craniopharyngioma, medulloblastoma, meningioma, pineocytoma, pineoblastoma, primary brain lymphoma; breast cancer including, but not limited to, adenocarcinoma, lobular (small cell) carcinoma, intraductal carcinoma, medullary breast cancer, mucinous breast cancer, tubular breast cancer, papillary breast cancer, Paget's disease, and inflammatory breast cancer; adrenal cancer, including but not limited to, pheochromocytom and adrenocortical carcinoma; thyroid cancer such as but not limited to papillary or follicular thyroid cancer, medullary thyroid cancer and anaplastic thyroid cancer; pancreatic cancer, including but not limited to, insulinoma, gastrinoma, glucagonoma, vipoma, somatostatin-secreting tumor, and carcinoid or islet cell tumor; pituitary cancers including but not limited to, Cushing's disease, prolactin-secreting tumor, acromegaly, and diabetes insipius; eye cancers including but not limited to, ocular melanoma such as iris melanoma, choroidal melanoma, and cilliary body melanoma, and retinoblastoma; vaginal cancers, including but not limited to, squamous cell carcinoma, adenocarcinoma, and melanoma; vulvar cancer, including but not limited to, squamous cell carcinoma, melanoma, adenocarcinoma, basal cell carcinoma, sarcoma, and Paget's disease; cervical cancers including but not limited to, squamous cell carcinoma, and adenocarcinoma; uterine cancers including but not limited to, endometrial carcinoma and uterine sarcoma; ovarian cancers including but not limited to, ovarian epithelial carcinoma, borderline tumor, germ cell tumor, and stromal tumor; esophageal cancers including but not limited to, squamous cancer, adenocarcinoma, adenoid cyctic carcinoma, mucoepidermoid carcinoma, adenosquamous carcinoma, sarcoma, melanoma, plasmacytoma, verrucous carcinoma, and oat cell (small cell) carcinoma; stomach cancers including but not limited to, adenocarcinoma, fungating (polypoid), ulcerating, superficial spreading, diffusely spreading, malignant lymphoma, liposarcoma, fibrosarcoma, and carcinosarcoma; colon cancers; rectal cancers; liver cancers including but not limited to hepatocellular carcinoma and hepatoblastoma, gallbladder cancers including but not limited to, adenocarcinoma; cholangiocarcinomas including but not limited to, pappillary, nodular, and diffuse; lung cancers including but not limited to, non-small cell lung cancer, squamous cell carcinoma (epidermoid carcinoma), adenocarcinoma, large-cell carcinoma and small-cell lung cancer; testicular cancers including but not limited to, germinal tumor, seminoma, anaplastic, classic (typical), spermatocytic, nonseminoma, embryonal carcinoma, teratoma carcinoma, choriocarcinoma (yolk-sac tumor), prostate cancers including but not limited to, adenocarcinoma, leiomyosarcoma, and rhabdomyosarcoma; penal cancers; oral cancers including but not limited to, squamous cell carcinoma; basal cancers; salivary gland cancers including but not limited to, adenocarcinoma, mucoepidermoid carcinoma, and adenoidcystic carcinoma; pharynx cancers including but not limited to, squamous cell cancer, and verrucous; skin cancers including but not limited to, basal cell carcinoma, squamous cell carcinoma and melanoma, superficial spreading melanoma, nodular melanoma, lentigo malignant melanoma, acral lentiginous melanoma; kidney cancers including but not limited to, renal cell cancer, adenocarcinoma, hypernephroma, fibrosarcoma, transitional cell cancer (renal pelvis and/or uterer); Wilms' tumor; bladder cancers including but not limited to, transitional cell carcinoma, squamous cell cancer, adenocarcinoma, carcinosarcoma. In addition, cancers include myxosarcoma, osteogenic sarcoma, endotheliosarcoma, lymphangioendotheliosarcoma, mesothelioma, synovioma, hemangioblastoma, epithelial carcinoma, cystadenocarcinoma, bronchogenic carcinoma, sweat gland carcinoma, sebaceous gland carcinoma, papillary carcinoma and papillary adenocarcinomas (for a review of such disorders, *see* Fishman *et al.,* 1985, *Medicine,* 2d Ed., J.B. Lippincott Co., Philadelphia and Murphy *et al.,* 1997, *Informed Decisions: The Complete Book of Cancer Diagnosis, Treatment, and Recovery,* Viking Penguin, Penguin Books U.S.A., Inc., United States of America).

Accordingly, the methods and compositions of the invention are also useful in the treatment or prevention of a variety of cancers or other abnormal proliferative diseases, including (but not limited to) the following: carcinoma, including that of the bladder, breast, colon, kidney, liver, lung, ovary, pancreas, stomach, cervix, thyroid and skin; including squamous cell carcinoma; hematopoietic tumors of lymphoid lineage, including leukemia, acute lymphocytic leukemia, acute lymphoblastic leukemia, B-cell lymphoma, T-cell lymphoma, Berketts lymphoma; hematopoietic tumors of myeloid lineage, including acute and chronic myelogenous leukemias and promyelocytic leukemia; tumors of mesenchymal origin, including fibrosarcoma and rhabdomyoscarcoma; other tumors, including melanoma, seminoma, tetratocarcinoma, neuroblastoma and glioma; tumors of the central and peripheral nervous system, including astrocytoma, neuroblastoma, glioma, and schwannomas; tumors of mesenchymal origin, including fibrosafcoma, rhabdomyoscarama, and osteosarcoma; and other tumors, including melanoma, xenoderma pegmentosum, keratoactanthoma, seminoma, thyroid follicular cancer and teratocarcinoma. It is also contemplated that cancers caused by aberrations in apoptosis would also be treated by the methods and compositions of the invention. Such cancers may include but not be limited to follicular lymphomas, carcinomas with p53 mutations, hormone dependent tumors of the breast, prostate and ovary, and precancerous lesions such as familial adenomatous polyposis, and myelodysplastic syndromes. In specific embodiments, malignancy or dysproliferative changes (such as metaplasias and dysplasias), or hyperproliferative disorders, are treated or prevented by the methods and compositions of the invention in the ovary, bladder, breast, colon, lung, skin, pancreas, or uterus. In other specific embodiments, sarcoma, melanoma, or leukemia is treated or prevented by the methods and compositions of the invention.

Cancers associated with the cancer antigens may be treated or prevented by administration of the dendritic cells or T cells that have been treated with a method of the invention in combination with an antibody that binds the cancer antigen and is cytotoxic. For example, but not by way of limitation, cancers associated with the following cancer antigen may be treated or prevented by the methods and compositions of the invention. KS 1/4 pan-carcinoma antigen (Perez and Walker, 1990, *J. Immunol.* 142:32-37; Bumal, 1988, *Hybridoma* 7(4):407-415), ovarian carcinoma antigen (CA125) (Yu *et al.,* 1991, *Cancer Res.* 51(2):48-475), prostatic acid phosphate (Tailor *et al.,* 1990, *Nucl. Acids Res.* 18(1):4928), prostate specific antigen (Henttu and Vihko, 1989, *Biochem. Biophys. Res. Comm.* 10(2):903-910; Israeli *et al.,* 1993, *Cancer Res.* 53:227-230), melanoma- associated antigen p97 *(*Estin *et al.,* 1989, *J Natl. Cancer Instit.* 81(6):445-44), melanoma antigen gp75 (Vijayasardahl *et al.,* 1990, *J*. *Exp. Med.* 171(4):1375-1380), high molecular weight melanoma antigen (HMW-MAA) (Natali *et aL,* 1987, *Cancer* 59:55-3; Mittelman *et al.,* 1990, *J Clin. Invest.* 86:2136-2144)), prostate specific membrane antigen, carcinoembryonic antigen (CEA) (Foon *et al.,* 1994, *Proc. Am. Soc. Clin. Oncol.* 13:294), polymorphic epithelial mucin antigen, human milk fat globule antigen, Colorectal tumor-associated antigens such as: CEA, TAG-72 (Yokata *et al., 1992, Cancer Res.* 52:3402-3408), CO17-1A (Ragnhammar *et al.,* 1993, *Int. J Cancer* 53:751-758); GICA 19-9 (Herlyn *et al.,* 1982, *J. Clin. Immunol.* 2:135), CTA-1 and LEA, Burkitt's lymphoma antigen-38.13, CD19 (Ghetie *et al.,* 1994, *Blood* 83:1329-1336), human B-lymphoma antigen-CD20 (Reff *et al.,* 1994, *Blood* 83:435-445), CD33 (Sgouros *et al.,* 1993, *J*. *Nucl. Med.* 34:422-430), melanoma specific antigens such as ganglioside GD2 *(*Saleh *et al.,* 1993, J.Immunol., 151, 3390-3398), ganglioside GD3 (Shitara *et al.,* 1993, *Cancer Immunol. Immunother.* 36:373-380), ganglioside GM2 (Livingston *et al.,* 1994, *J*. *Clin. Oncol.* 12:1036-1044), ganglioside GM3 (Hoon *et al.,* 1993, *Cancer Res.* 53:5244-5250), tumor-specific transplantation type of cell-surface antigen (TSTA) such as virally-induced tumor antigens including T-antigen DNA tumor viruses and envelope antigens of RNA tumor viruses, oncofetal antigen-alpha-fetoprotein such as CEA of colon, bladder tumor oncofetal antigen (Hellstrom *et al.,* 1985, *Cancer. Res.* 45:2210-2188), differentiation antigen such as human lung carcinoma antigen L6, L20 (Hellstrom *et al.,* 1986, *Cancer Res.* 46:3917-3923), antigens of fibrosarcoma, human leukemia T cell antigen-Gp37 (Bhattacharya-Chatterjee *et al.,* 1988, *J. of Immun.* 141:1398-1403), neoglycoprotein, sphingolipids, breast cancer antigen such as EGFR (Epidermal growth factor receptor), HER2 antigen (p185^{HER2}), polymorphic epithelial mucin (PEM) (Hilkens *et al.,* 1992, *Trends in Bio. Chem. Sci.* 17:359), malignant human lymphocyte antigen-APO-1 *(*Bernhard *et al.,* 1989, *Science* 245:301-304), differentiation antigen (Feizi, 1985, *Nature* 314:53-57) such as I antigen found in fetal erthrocytes and primary endoderm, I(Ma) found in gastric adencarcinomas, M18 and M39 found in breast epithelium, SSEA-1 found in myeloid cells, VEP8, VEP9, Myl, VIM-D5,and D₁56-22 found in colorectal cancer, TRA-1-85 (blood group H), C14 found in colonic adenocarcinoma, F3 found in lung adenocarcinoma, AH6 found in gastric cancer, Y hapten, Le^{y} found in embryonal carcinoma cells, TL5 (blood group A), EGF receptor found in A431 cells , E₁ series (blood group B) found in pancreatic cancer, FC10.2 found in embryonal carcinoma cells, gastric adenocarcinoma, CO-514 (blood group Le^{a}) found in adenocarcinoma, NS-10 found in adenocarcinomas, CO-43 (blood group Le^{b}), G49, EGF receptor, (blood group ALe^{b}/Le^{y}) found in colonic adenocarcinoma, 19.9 found in colon cancer, gastric cancer mucins, T₅A₇ found in myeloid cells, R₂₄ found in melanoma, 4.2, G_{D3}, D1.1, OFA-1, G_{M2}, OFA-2, G_{D2}, M1:22:25:8 found in embryonal carcinoma cells and SSEA-3, SSEA-4 found in 4-8-cell stage embryos. In another embodiment, the antigen is a T cell receptor derived peptide from a cutaneous T cell lymphoma *(see* Edelson, 1998, *The Cancer Journal* 4:62).

Dendritic cells or T cells that have been treated with a method of the invention of the invention can also be used in combination with cytosine-guanine dinucleotides ("CpG")-based products that have been developed (Coley Pharmaceuticals) or are currently being developed as activators of innate and acquired immune responses. For example, the invention encompasses the use of CpG 7909, CpG 8916, CpG 8954 (Coley Pharmaceuticals) in the methods and compositions of the invention for the treatment and/or prevention of cancer *(See* also Warren *et al.,* 2002, *Semin Oncol.,* 29(1 Suppl 2):93-7; Warren *et al.,* 2000, *Clin Lymphoma,* 1(1):57-61, which are incorporated herein by reference).

Cancer therapies and their dosages, routes of administration and recommended usage are known in the art and have been described in the literature, *see, e.g., Physician's Desk Reference* (56^{th} ed., 2002, which is incorporated herein by reference).

### 5.11.2 ANTI-CANCER AGENTS AND THERAPEUTIC ANTIBODIES

In a specific embodiment, the methods of the invention relating to the treatment of cancer further encompass the administration of one or more angiogenesis inhibitors such as but not limited to: Angiostatin (plasminogen fragment); antiangiogenic antithrombin III; Angiozyme; ABT-627; Bay 12-9566; Benefin; Bevacizumab; BMS-275291; cartilage-derived inhibitor (CDI); CAI; CD59 complement fragment; CEP-7055; Col 3; Combretastatin A-4; Endostatin (collagen XVIII fragment); Fibronectin fragment; Gro-beta; Halofuginone; Heparinases; Heparin hexasaccharide fragment; HMV833; Human chorionic gonadotropin (hCG); IM-862; Interferon alpha/beta/gamma; Interferon inducible protein (IP-10); Interleukin-12; Kringle 5 (plasminogen fragment); Marimastat; Metalloproteinase inhibitors (TIMPs); 2-Methoxyestradiol; MMI 270 (CGS 27023A); MoAb IMC-1C11; Neovastat; NM-3; Panzem; PI-88; Placental ribonuclease inhibitor; Plasminogen activator inhibitor; Platelet factor-4 (PF4); Prinomastat; Prolactin 16kD fragment; Proliferin-related protein (PRP); PTK 787/ZK 222594; Retinoids; Solimastat; Squalamine; SS 3304; SU 5416; SU6668; SU11248; Tetrahydrocortisol-S; tetrathiomolybdate; thalidomide; Thrombospondin-1 (TSP-1); TNP-470; Transforming growth factor-beta (TGF-b); Vasculostatin; Vasostatin (calreticulin fragment); ZD6126; ZD 6474; farnesyl transferase inhibitors (FTI); and bisphosphonates.

Anti-cancer agents that can be used in combination with matured dendritic cells and T cells that have been generated using the methods of the invention, include, but are not limited to: acivicin; aclarubicin; acodazole hydrochloride; acronine; adozelesin; aldesleukin; altretamine; ambomycin; ametantrone acetate; aminoglutethimide; amsacrine; anastrozole; anthramycin; asparaginase; asperlin; azacitidine; azetepa; azotomycin; batimastat; benzodepa; bicalutamide; bisantrene hydrochloride; bisnafide dimesylate; bizelesin; bleomycin sulfate; brequinar sodium; bropirimine; busulfan; cactinomycin; calusterone; caracemide; carbetimer; carboplatin; carmustine; carubicin hydrochloride; carzelesin; cedefingol; chlorambucil; cirolemycin; cisplatin; cladribine; crisnatol mesylate; cyclophosphamide; cytarabine; dacarbazine; dactinomycin; daunorubicin hydrochloride; decitabine; dexormaplatin; dezaguanine; dezaguanine mesylate; diaziquone; docetaxel; doxorubicin; doxorubicin hydrochloride; droloxifene; droloxifene citrate; dromostanolone propionate; duazomycin; edatrexate; eflornithine hydrochloride; elsamitrucin; enloplatin; enpromate; epipropidine; epirubicin hydrochloride; erbulozole; esorubicin hydrochloride; estramustine; estramustine phosphate sodium; etanidazole; etoposide; etoposide phosphate; etoprine; fadrozole hydrochloride; fazarabine; fenretinide; floxuridine; fludarabine phosphate; fluorouracil; flurocitabine; fosquidone; fostriecin sodium; gemcitabine; gemcitabine hydrochloride; hydroxyurea; idarubicin hydrochloride; ifosfamide; ilmofosine; interleukin II (including recombinant interleukin II, or rIL2), interferon alfa-2a; interferon alfa-2b; interferon alfa-n1 interferon alfa-n3; interferon beta-I a; interferon gamma-I b; iproplatin; irinotecan hydrochloride; lanreotide acetate; letrozole; leuprolide acetate; liarozole hydrochloride; lometrexol sodium; lomustine; losoxantrone hydrochloride; masoprocol; maytansine; mechlorethamine hydrochloride; megestrol acetate; melengestrol acetate; melphalan; menogaril; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazole; nogalamycin; ormaplatin; oxisuran; paclitaxel; pegaspargase; peliomycin; pentamustine; peplomycin sulfate; perfosfamide; pipobroman; piposulfan; piroxantrone hydrochloride; plicamycin; plomestane; porfimer sodium; porfiromycin; prednimustine; procarbazine hydrochloride; puromycin; puromycin hydrochloride; pyrazofurin; riboprine; rogletimide; safingol; safingol hydrochloride; semustine; simtrazene; sparfosate sodium; sparsomycin; spirogermanium hydrochloride; spiromustine; spiroplatin; streptonigrin; streptozocin; sulofenur; talisomycin; tecogalan sodium; tegafur; teloxantrone hydrochloride; temoporfin; teniposide; teroxirone; testolactone; thiamiprine; thioguanine; thiotepa; tiazofurin; tirapazamine; toremifene citrate; trestolone acetate; triciribine phosphate; trimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; vorozole; zeniplatin; zinostatin; zorubicin hydrochloride. Other anti-cancer drugs include, but are not limited to: 20-epi-1,25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; canarypox IL-2; capecitabine; carboxamide-amino-triazole; carboxyamidotriazole; CaRest M3; CARN 700; cartilage derived inhibitor; carzelesin; casein kinase inhibitors (ICOS); castanospermine; cecropin B; cetrorelix; chlorlns; chloroquinoxaline sulfonamide; cicaprost; cis-porphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidemnin B; deslorelin; dexamethasone; dexifosfamide; dexrazoxane; dexverapamil; diaziquone; didemnin B; didox; diethylnorspermine; dihydro-5-azacytidine; dihydrotaxol, 9-; dioxamycin; diphenyl spiromustine; docetaxel; docosanol; dolasetron; doxifluridine; droloxifene; dronabinol; duocarmycin SA; ebselen; ecomustine; edelfosine; edrecolomab; eflornithine; elemene; emitefur; epirubicin; epristeride; estramustine analogue; estrogen agonists; estrogen antagonists; etanidazole; etoposide phosphate; exemestane; fadrozole; fazarabine; fenretinide; filgrastim; finasteride; flavopiridol; flezelastine; fluasterone; fludarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; hepsulfam; heregulin; hexamethylene bisacetamide; hypericin; ibandronic acid; idarubicin; idoxifene; idramantone; ilmofosine; ilomastat; imidazoacridones; imiquimod; immunostimulant peptides; insulin-like growth factor-1 receptor inhibitor; interferon agonists; interferons; interleukins; iobenguane; iododoxorubicin; ipomeanol, 4-; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide+estrogen+progesterone; leuprorelin; levamisole; liarozole; linear polyamine analogue; lipophilic disaccharide peptide; lipophilic platinum compounds; lissoclinamide 7; lobaplatin; lombricine; lometrexol; lonidamine; losoxantrone; lovastatin; loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; menogaril; merbarone; meterelin; methioninase; metoclopramide; MIF inhibitor; mifepristone; miltefosine; mirimostim; mismatched double stranded RNA; mitoguazone; mitolactol; mitomycin analogues; mitonafide; mitotoxin fibroblast growth factor-saporin; mitoxantrone; mofarotene; molgramostim; monoclonal antibody, human chorionic gonadotrophin; monophosphoryl lipid A+myobacterium cell wall sk; mopidamol; multiple drug resistance gene inhibitor; multiple tumor suppressor 1-based therapy; mustard anticancer agent; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin; neridronic acid; neutral endopeptidase; nilutamide; nisamycin; nitric oxide modulators; nitroxide antioxidant; nitrullyn; 06-benzylguanine; octreotide; okicenone; oligonucleotides; onapristone; ondansetron; ondansetron; oracin; oral cytokine inducer; ormaplatin; osaterone; oxaliplatin; oxaunomycin; paclitaxel; paclitaxel analogues; paclitaxel derivatives; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; prednisone; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rogletimide; rohitukine; romurtide; roquinimex; rubiginone B1; ruboxyl; safingol; saintopin; SarCNU; sarcophytol A; sargramostim; Sdi 1 mimetics; semustine; senescence derived inhibitor 1; sense oligonucleotides; signal transduction inhibitors; signal transduction modulators; single chain antigen binding protein; sizofiran; sobuzoxane; sodium borocaptate; sodium phenylacetate; solverol; somatomedin binding protein; sonermin; sparfosic acid; spicamycin D; spiromustine; splenopentin; spongistatin 1; squalamine; stem cell inhibitor; stem-cell division inhibitors; stipiamide; stromelysin inhibitors; sulfinosine; superactive vasoactive intestinal peptide antagonist; suradista; suramin; swainsonine; synthetic glycosaminoglycans; tallimustine; tamoxifen methiodide; tauromustine; tazarotene; tecogalan sodium; tegafur; tellurapyrylium; telomerase inhibitors; temoporfin; temozolomide; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene bichloride; topsentin; toremifene; totipotent stem cell factor; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor; urokinase receptor antagonists; vapreotide; variolin B; vector system, erythrocyte gene therapy; velaresol; veramine; verdins; verteporfin; vinorelbine; vinxaltine; vitaxin; vorozole; zanoterone; zeniplatin; zilascorb; and zinostatin stimalamer. Preferred additional anti-cancer drugs are 5-fluorouracil and leucovorin.

Examples of therapeutic antibodies that can be used in methods of the invention include but are not limited to HERCEPTIN® (Trastuzumab) (Genentech, CA) which is a humanized anti-HER2 monoclonal antibody for the treatment of patients with metastatic breast cancer; REOPRO® (abciximab) (Centocor) which is an anti-glycoprotein IIb/IIIa receptor on the platelets for the prevention of clot formation; ZENAPAX® (daclizumab) (Roche Pharmaceuticals, Switzerland) which is an immunosuppressive, humanized anti-CD25 monoclonal antibody for the prevention of acute renal allograft rejection; PANOREX™ which is a murine anti-17-IA cell surface antigen IgG2a antibody (Glaxo Wellcome/Centocor); BEC2 which is a murine anti-idiotype (GD3 epitope) IgG antibody (ImClone System); IMC-C225 which is a chimeric anti-EGFR IgG antibody (ImClone System); VITAXIN™ which is a humanized anti-αVβ3 integrin antibody (Applied Molecular Evolution/MedImmune); Campath 1H/LDP-03 which is a humanized anti CD52 IgG1 antibody (Leukosite); Smart M195 which is a humanized anti-CD33 IgG antibody (Protein Design Lab/Kanebo); RITUXAN™ which is a chimeric anti-CD20 IgG1 antibody (IDEC Pharm/Genentech, Roche/Zettyaku); LYMPHOCIDE™ which is a humanized anti-CD22 IgG antibody (Immunomedics); ICM3 is a humanized anti-ICAM3 antibody (ICOS Pharm); IDEC-114 is a primatied anti-CD80 antibody (IDEC Pharm/Mitsubishi); ZEVALIN™ is a radiolabelled murine anti-CD20 antibody (IDEC/Schering AG); IDEC-131 is a humanized anti-CD40L antibody (IDEC/Eisai); IDEC-151 is a primatized anti-CD4 antibody (IDEC); IDEC-152 is a primatized anti-CD23 antibody (IDEC/Seikagaku); SMART anti-CD3 is a humanized anti-CD3 IgG (Protein Design Lab); SG1.1 is a humanized anti-complement factor 5 (C5) antibody (Alexion Pharm); D2E7 is a humanized anti-TNF-α antibody (CATBASF); CDP870 is a humanized anti-TNF-α Fab fragment (Celltech); IDEC-151 is a primatized anti-CD4 IgG1 antibody (IDEC Pharm/SmithKline Beecham); MDX-CD4 is a human anti-CD4 IgG antibody (Medarex/Eisai/Genmab); CDP571 is a humanized anti-TNF-α IgG4 antibody (Celltech); LDP-02 is a humanized anti-α4p7 antibody (LeukoSite/Genentech); OrthoClone OKT4A is a humanized anti-CD4 IgG antibody (Ortho Biotech); ANTOVA™ is a humanized anti-CD40L IgG antibody (Biogen); ANTEGREN™ is a humanized anti-VLA-4 IgG antibody (Elan); and CAT-152 is a human anti-TGF-p₂ antibody (Cambridge Ab Tech).

Other examples of therapeutic antibodies that can be used in combination with matured dendritic cells and T cells that have been generated using the methods of the invention are presented in Table 3.

**Table 3: Monoclonal antibodies for Cancer Therapy that can be used in combination with the antibodies of the invention.**

| Company | Product | Disease | Target |
|---|---|---|---|
| Abgenix | ABX-EGF | Cancer | EGF receptor |
| AltaRex | OvaRex | ovarian cancer | tumor antigen CA125 |
| | BravaRex | metastatic cancers | tumor antigen MUC1 |
| Antisoma | Theragyn (pemtumomabytrrium-90) | ovarian cancer | PEM antigen |
| | Therex | breast cancer | PEM antigen |
| Boehringer Ingelheim | blvatuzumab | head & neck cancer | CD44 |
| Centocor/J&J | Panorex | Colorectal cancer | 17-1A |
| | ReoPro | PTCA | gp IIIb/IIIa |
| | ReoPro | Acute MI | gp IIIb/IIIa |
| | ReoPro | Ischemic stroke | gp IIIb/IIIa |
| Corixa | Bexocar | NHL | CD20 |
| CRC Technology | MAb, idiotypic 105AD7 | colorectal cancer vaccine | gp72 |
| Crucell | Anti-EpCAM | cancer | Ep-CAM |
| Cytoclonal | MAb, lung cancer | non-small cell lung cancer | NA |
| Genentech | Herceptin | metastatic breast cancer | HER-2 |
| | Herceptin | early stage breast cancer | HER-2 |
| | Rituxan | Relapsed/refractory low-grade or follicular NHL | CD20 |
| | Rituxan | intermediate & high-grade NHL | CD20 |
| | MAb-VEGF | NSCLC, metastatic | VEGF |
| | MAb-VEGF | Colorectal cancer, metastatic | VEGF |
| | AMD Fab | age-related macular degeneration | CD 18 |
| | E-26 (2^{nd} gen. IgE) | allergic asthma & rhinitis | IgE |
| IDEC | Zevalin (Rituxan + yttrium-90) | low grade of follicular, relapsed or refractory, CD20-positive, B-cell NHL and Rituximab-refractory NHL | CD20 |
| ImClone | Cetuximab + innotecan | refractory colorectal carcinoma | EGF receptor |
| | Cetuximab + cisplatin & radiation | newly diagnosed or recurrent head & neck cancer | EGF receptor |
| | Cetuximab + gemcitabine | newly diagnosed metastatic pancreatic carcinoma | EGF receptor |
| | Cetuximab + cisplatin + 5FU or Taxol | recurrent or metastatic head & neck cancer | EGF receptor |
| | Cetuximab + carboplatin + paclitaxel | newly diagnosed non-small cell lung carcinoma | EGF receptor |
| | Cetuximab + cisplatin | head & neck cancer (extensive incurable local-regional disease & distant metasteses) | EGF receptor |
| | Cetuximab + radiation | locally advanced head & neck carcinoma | EGF receptor |
| | BEC2 + Bacillus Calmette Guerin | small cell lung carcinoma | mimics ganglioside GD3 |
| | BEC2 + Bacillus Calmette Guerin | melanoma | mimics ganglioside GD3 |
| | IMC-1C11 | colorectal cancer with liver metasteses | VEGF-receptor |
| ImmonoGen | nuC242-DM1 | Colorectal, gastric, and pancreatic cancer | nuC242 |
| ImmunoMedics | LymphoCide | Non-Hodgkins lymphoma | CD22 |
| | LymphoCide Y-90 | Non-Hodgkins lymphoma | CD22 |
| | CEA-Cide | metastatic solid tumors | CEA |
| | CEA-Cide Y-90 | metastatic solid tumors | CEA |
| | CEA-Scan (Tc-99m-labeled arcitumomab) | colorectal cancer (radioimaging) | CEA |
| | CEA-Scan (Tc-99m-labeled arcitumomab) | Breast cancer (radioimaging) | CEA |
| | CEA-Scan (Tc-99m-labeled arcitumomab) | lung cancer (radioimaging) | CEA |
| | CEA-Scan (Tc-99m-labeled arcitumomab) | intraoperative tumors (radio imaging) | CEA |
| | LeukoScan (Tc-99m-labeled sulesomab) | soft tissue infection (radioimaging) | CEA |
| | LymphoScan (Tc-99m-labeled) | lymphomas (radioimaging) | CD22 |
| | AFP-Scan (Tc-99m-labeled) | liver 7 gem-cell cancers (radioimaging) | AFP |
| Intracel | HumaRAD-HN (+ yttrium-90) | head & neck cancer | NA |
| | HumaSPECT | colorectal imaging | NA |
| Medarex | MDX-101 (CTLA-4) | Prostate and other cancers | CTLA-4 |
| | MDX-210 (her-2 overexpression) | Prostate cancer | HER-2 |
| | MDX-210/MAK | Cancer | HER-2 |
| MedImmune | Vitaxin | Cancer | αvβ₃ |
| Merck KGaA | MAb 425 | Various cancers | EGF receptor |
| | IS-IL-2 | Various cancers | Ep-CAM |
| Millennium | Campath (alemtuzumab) | chronic lymphocytic leukemia | CD52 |
| NeoRx | CD20-streptavidin (+ biotin-yttrium 90) | Non-Hodgkins lymphoma | CD20 |
| | Avidicin (albumin + NRLU13) | metastatic cancer | NA |
| Peregrine | Oncolym (+ iodine-131) | Non-Hodgkins lymphoma | HLA-DR 10 beta |
| | Cotara (+ iodine-131) | unresectable malignant glioma | DNA-associated proteins |
| Pharmacia Corporation | C215 (+ staphylococcal enterotoxin) | pancreatic cancer | NA |
| | MAb, lung/kidney cancer | lung & kidney cancer | NA |
| | nacolomab tafenatox (C242 + staphylococcal enterotoxin) | colon & pancreatic cancer | NA |
| Protein Design Labs | Nuvion | T cell malignancies | CD3 |
| | SMART M195 | AML | CD33 |
| | SMART 1D10 | NHL | HLA-DR antigen |
| Titan | CEAVac | colorectal cancer, advanced | CEA |
| | TriGem | metastatic melanoma & small cell lung cancer | GD2-ganglioside |
| | TriAb | metastatic breast cancer | MUC-1 |
| Trilex | CEAVac | colorectal cancer, advanced | CEA |
| | TriGem | metastatic melanoma & small cell lung cancer | GD2-ganglioside |
| | TriAb | metastatic breast cancer | MUC-1 |
| Viventia Biotech | NovoMAb-G2 radiolabeled | Non-Hodgkins lymphoma | NA |
| | Monopharm C | colorectal & pancreatic carcinoma | SK-1 antigen |
| | GlioMAb-H (+ gelonin toxin) | gliorna, melanoma & neuroblastoma | NA |
| Xoma | Rituxan | Relapsed/refractory low-grade or follicular NHL | CD20 |
| | Rituxan | intermediate & high-grade NHL | CD20 |
| | ING-1 | adenomcarcinoma | Ep-CAM |

### 5.12 DENDRITIC CELLS

Dendritic cells (DCs) can be isolated or generated from blood or bone marrow, or secondary lymphoid organs of the subject, such as but not limited to spleen, lymph nodes, tonsils, Peyer's patch of the intestine, and bone marrow, by any of the methods known in the art.

Immune cells obtained from such sources typically comprise predominantly recirculating lymphocytes and macrophages at various stages of differentiation and maturation. Dendritic cell preparations can be enriched by standard techniques (see e.g., Current Protocols in Immunology, 7.32.1-7.32.16, John Wiley and Sons, Inc., 1997). In one embodiment, for example, DCs may be enriched by depletion of T cells and adherent cells, followed by density gradient centrifugation. DCs may optionally be further purified by sorting of fuorescence-labeled cells using antibodies against DC markers. DCs may also be isolated using antibodies against DCs, wherein the antibodies are linked to magnetic beads. In a specific embodiment, DCs that co-express CD32a and CD32b are isolated using FACS.

By way of example but not limitation, dendritic cells can be obtained from blood monocytes as follows: peripheral blood monocytes are obtained by standard methods (see, e.g., Sallusto et al., 1994, J. Exp. Med. 179:1109-1118). Leukocytes from healthy blood donors are collected by leukapheresis pack or buffy coat preparation using Ficoll-Paque density gradient centrifugation and plastic adherence.

Optionally, standard techniques such as morphological observation and immunochemical staining, can be used to verify the presence of dendritic cells. For example, the purity of dendritic cells can be assessed by flow cytometry using fluorochrome-labeled antibodies directed against one or more of the characteristic cell surface markers.

Types of dendritic cells that can be used with the methods and compositions of the invention are set forth below.

**Monocyte-derived dendritic cells (moDCs)** (Thurner B, Roder C, Dieckmann D, Heuer M, Kruse M, Glaser A, Keikavoussi P, Kampgen E, Bender A, Schuler G. Generation of large numbers of fully mature and stable dendritic cells from leukapheresis products for clinical application. J.Immunol.Meth. 1999; 223:1-15) Peripheral blood mononuclear cells (PBMCs) can be obtained either from whole blood diluted 1:1 with buffered saline or from leukocyte concentrates ("buffy coat" fractions, MSKCC Blood Bank) by standard centrifugation over Ficoll-Paque PLUS (endotoxin-free, #17- 1440-03, Amersham Pharmacia Biotech AB, Uppsala, Sweden). MoDC precursors are tissue culture plastic-adherent (#35-3003; Falcon, Becton-Dickinson Labware, Franklin Lakes, NJ) PBMCs, and can be cultured in complete RPMI 1640―1% normal human serum (NHS) in the presence of GM-CSF (1000 IU/ml) and IL-4 (500 IU/ml) with replenishment every 2 days as described. (Thurner B, Roder C, Dieckmann D, Heuer M, Kruse M, Glaser A, Keikavoussi P, Kampgen E, Bender A, Schuler G. Generation of large numbers of fully mature and stable dendritic cells from leukapheresis products for clinical application. J.Immunol.Meth. 1999; 223:1-15; Ratzinger G, Baggers J, de Cos MA, Yuan J, Dao T, Reagan JL, Munz C, Heller G, Young JW. Mature human Langerhans cells derived from CD34+ hematopoietic progenitors stimulate greater cytolytic T lymphocyte activity in the absence of bioactive IL-12p70, by either single peptide presentation or cross-priming, than do dermal-interstitial or monocyte-derived dendritic cells. J. Immunol. 2004; 173:2780-2791)

**Dermal-interstitial DCs (DDCs/IDCs)** (Ratzinger G, Baggers J, de Cos MA, Yuan J, Dao T, Reagan JL, Munz C, Heller G, Young JW. Mature human Langerhans cells derived from CD34+ hematopoietic progenitors stimulate greater cytolytic T lymphocyte activity in the absence of bioactive IL-12p70, by either single peptide presentation or cross-priming, than do dermal-interstitial or monocyte-derived dendritic cells. J. Immunol. 2004; 173:2780-2791)

Healthy donors already undergoing bone marrow or G-CSF-elicited peripheral blood stem cell (PBSC) collections for allogeneic transplantation can provide a source of CD34+ HPCs for generating LCs and DDC-IDCs. Mononuclear cells (MNCs) can be separated over Ficoll-Paque PLUS, from which CD34+ HPCs are obtained by positive immunomagnetic selection according to the manufacturer's instructions (CD34+ isolation kit and LS separation columns, Miltenyi Biotec, Bergisch Gladbach, Germany). DDC-IDCs can be generated from these CD34+ HPCs without exposure to xenogeneic Ags like those in FCS. Specific cytokine supplements may include GM-CSF (1000 IU/ml), TNF-alpha (5ng/ml), c-kit-ligand (20ng/ml), and FLT-3 ligand (50ug/ml), with removal of c-*kit*-ligand and FLT-3 ligand from day 5-6 onward. Cytokines and media can be replenished on day 3, and thereafter approximately every other day. For the specific generation of DDC-IDCs, IL-4 (500 IU/ml) is added to suppress macrophage differentiation (Jansen JH, Wientjens G-JHM, Fibbe WE, Willemze R, Kluin-Nelemans HC. Inhibition of human macrophage colony formation by interleukin 4. J.Exp.Med. 1989; 170:577-582, Caux C, Massacrier C, Dubois B, Valladeau J, Dezutter-Dambuyant C, Durand I, Schmitt D, Saeland S. Respective involvement of TGF-β and IL-4 in the development of Langerhans cells and non-Langerhans dendritic cells from CD34+ progenitors. J Leuk Biol 1999; 66:781-91) when the cells are recultured from serum/plasma-replete media into X-VIVO 15 with GM-CSF and TNF-alpha, but without c-*kit*-ligand and FLT-3 ligand.

**Langerhans'cells.** (Ratzinger G, Baggers J, de Cos MA, Yuan J, Dao T, Reagan JL, Munz C, Heller G, Young JW. Mature human Langerhans cells derived from CD34+ hematopoietic progenitors stimulate greater cytolytic T lymphocyte activity in the absence of bioactive IL-12p70, by either single peptide presentation or cross-priming, than do dermal-interstitial or monocyte-derived dendritic cells. J. Immunol. 2004; 173:2780-2791)

Healthy donors already undergoing bone marrow or G-CSF-elicited peripheral blood stem cell (PBSC) collections for allogeneic transplantation may provide the source of CD34+ HPCs. Mononuclear cells (MNCs) can be separated over Ficoll-Paque PLUS, from which which CD34+ HPCs can be obtained by positive immunomagnetic selection according to the manufacturer's instructions (CD34+ isolation kit and LS separation columns, Miltenyi Biotec, Bergisch Gladbach, Germany). LCs can be generated from these CD34+ HPCs without exposure to xenogeneic Ags like those in FCS. Specific cytokine supplements may include GM-CSF (1000 IU/ml), TNF-alpha (5ng/ml), c*-kit-*ligand (20ng/ml), and FLT-3 ligand (50ug/ml), with removal of c-kit-ligand and FLT-3 ligand from day 5-6 onward. Cytokines and media can be replenished on day 3, and thereafter approximately every other day. For the specific generation of LCs, TGF-beta-1 (10ng/ml) is provided throughout the entire culture period (Ratzinger G, Baggers J, de Cos MA, Yuan J, Dao T, Reagan JL, Munz C, Heller G, Young JW. Mature human Langerhans cells derived from CD34+ hematopoietic progenitors stimulate greater cytolytic T lymphocyte activity in the absence of bioactive IL-12p70, by either single peptide presentation or cross-priming, than do dermal-interstitial or monocyte-derived dendritic cells. J. Immunol. 2004; 173:2780-2791, Caux C, Massacrier C, Dubois B, Valladeau J, Dezutter-Dambuyant C, Durand I, Schmitt D, Saeland S. Respective involvement of TGF-β and IL-4 in the development of Langerhans cells and non-Langerhans dendritic cells from CD34+ progenitors. J Leuk Biol 1999; 66:781-91, Borkowski TA, Letterio JJ, Farr AG, Udey MC. A role for endogenous transforming growth factor β1 in Langerhans cell biology: The skin of transforming growth factor β1 null mice is devoid of epidermal Langerhans cells. J.Exp.Med. 1996; 184:2417-2422, Strobl H, Riedl E, Scheinecker C, Bello-Fernandez C, Pickl WF, Rappersberger K, Majdic O, Knapp W. TGF-β1 promotes in vitro development of dendritic cells from CD34+ hemopoietic progenitors. J.Immunol. 1996; 157:1499-1507, Gatti E, Velleca MA, Biedermann BC, Ma W, Unternaehrer J, Ebersold MW, Medzhitov R, Pober JS, Mellman I. Large-scale culture and selective maturation of human Langerhans cells from granulocyte colony-stimulating factor-mobilized CD34+ progenitors. J Immunol 2000; 164:3600-7).

**Myeloid blood dendritic cells (DC1)** (Timmerman JM, Czerwinski DK, Davis TA, Hsu FJ, Benike C, Hao ZM, Taidi B, Rajapaksa R, Caspar CB, Okada CY, van Beckhoven A, Liles TM, Engleman EG, Levy R. Idiotype-pulsed dendritic cell vaccination for B-cell lymphoma: clinical and immune responses in 35 patients. Blood 2002; 99:1517-1526)

DC1 populations can be purified from PBMCs in fresh blood. PBMCs are obtained either from whole blood diluted 1:1 with buffered saline or from leukocyte concentrates ("buffy coat" fractions,) by standard centrifugation over Ficoll-Paque PLUS (endotoxin-free, #17-1440-03, Amersham Pharmacia Biotech AB, Uppsala, Sweden). After incubating PBMCs with anti-CD3, anti-CD14, anti-CD20 and anti-CD56, T cells, monocytes, B cells and NK cells, respectively, are depleted using magnetic beads conjugated to sheep anti-mouse IgG. From the remaining cells, DC1 by positive immunomagentic (or flow cytometric) sorting of cells expressing the blood-dendritic cell antigen (BDCA)-1 can be selected. (Dzionek A, Fuchs A, Schmidt P, Cremer S, Zysk M, Miltenyi S, Buck DW, Schmitz J. BDCA-2, BDCA-3, and BDCA-4: Three markers for distinct subsets of dendritic cells in human peripheral blood. J Immunol 2000; 165:6037-6046) DC1 can be cultured in 10% PHS with GM-CSF (Ito T, Amakawa R, Inaba M, Ikehara S, Inaba K, Fukuhara S. Differential Regulation of Human Blood Dendritic Cell Subsets by IFNs. J Immunol 2001; 166:2961-2969) or without GM-CSF (Timmerman JM, Czerwinski DK, Davis TA, Hsu FJ, Benike C, Hao ZM, Taidi B, Rajapaksa R, Caspar CB, Okada CY, van Beckhoven A, Liles TM, Engleman EG, Levy R. Idiotype-pulsed dendritic cell vaccination for B-cell lymphoma: clinical and immune responses in 35 patients. Blood 2002; 99:1517-1526) in Teflon beakers. Unlike immature moDCs, freshly isolated DC1 can be cultured in the absence of IL-4 without losing characteristic DC phenotype or function. (Timmerman JM, Czerwinski DK, Davis TA, Hsu FJ, Benike C, Hao ZM, Taidi B, Rajapaksa R, Caspar CB, Okada CY, van Beckhoven A, Liles TM, Engleman EG, Levy R. Idiotype-pulsed dendritic cell vaccination for B-cell lymphoma: clinical and immune responses in 35 patients. Blood 2002; 99:1517-1526).

Alternatively, DCs can be isolated from leukapheresis products by a series of density gradient centrifugation steps, as previously described. (Timmerman JM, Czerwinski DK, Davis TA, Hsu FJ, Benike C, Hao ZM, Taidi B, Rajapaksa R, Caspar CB, Okada CY, van Beckhoven A, Liles TM, Engleman EG, Levy R. Idiotype-pulsed dendritic cell vaccination for B-cell lymphoma: clinical and immune responses in 35 patients. Blood 2002; 99:1517-1526; Hsu FJ, Benike C, Fagnoni F, Liles TM, Czerwinski D, Taidi B, Engleman EG, Levy R. Vaccination of patients with B-cell lymphoma using autologous antigen-pulsed dendritic cells. Nature Med. 1996; 2:52-58). PBMCs are obtained by leukapheresis using a COBE cell separator apparatus or from leukocyte concentrates followed by Ficoll-Hypaque sedimentation (Pharmacia, Uppsala, Sweden). Monocytes are then removed by a discontinuous (50%) Percoll gradient (Pharmacia). The high-density fraction is then cultured in RPMI 1640 plus 10% human AB serum in Teflon vessels (Savillex, Minneapolis, MN) The next day, high-density lymphocytes are removed using a 15% metrizamide gradient (Sigma, St Louis, MO), and the low-density fraction is enriched for DCs. (Timmerman JM, Czerwinski DK, Davis TA, Hsu FJ, Benike C, Hao ZM, Taidi B, Rajapaksa R, Caspar CB, Okada CY, van Beckhoven A, Liles TM, Engleman EG, Levy R. Idiotype-pulsed dendritic cell vaccination for B-cell lymphoma: clinical and immune responses in 35 patients. Blood 2002; 99:1517-1526)

**Plasmacytoid dendritic cells** (Bave U, Magnusson M, Eloranta M-L, Perers A, Alm GV, Ronnblom L. Fc{gamma}RIIa Is Expressed on Natural IFN-{alpha}-Producing Cells (Plasmacytoid Dendritic Cells) and Is Required for the IFN-{alpha} Production Induced by Apoptotic Cells Combined with Lupus IgG J Immunol 2003; 171:3296-3302)

pDC2 populations can be purified from PBMCs. After incubating PBMCs with anti-CD3, anti-CD14, anti-CD20 and anti-CD56, T cells, monocytes, B cells and NK cells, respectively, are depleted using magnetic beads conjugated to sheep anti-mouse IgG. From the remaining cells, pDC2 are selected by positive immunomagentic (or flow cytometric) sorting of cells expressing the blood-dendritic cell antigens (BDCA)-2 and BDCA-4. (Dzionek A, Fuchs A, Schmidt P, Cremer S, Zysk M, Miltenyi S, Buck DW, Schmitz J. BDCA-2, BDCA-3, and BDCA-4: Three markers for distinct subsets of dendritic cells in human peripheral blood. J Immunol 2000; 165:6037-6046, Dzionek A, Sohma Y, Nagafune J, Cella M, Colonna M, Facchetti F, Gunther G, Johnston I, Lanzavecchia A, Nagasaka T, Okada T, Vermi W, Winkels G, Yamamoto T, Zysk M, Yamaguchi Y, Schmitz J. BDCA-2, a novel plasmacytoid dendritic cell-specific type II C-type lectin, mediates antigen capture and is a potent inhibitor of interferon alpha/beta induction. J Exp Med 2001; 194:1823-34) We will culture DC2 in 10% NHS supplemented with IL-3, which is requisite for pDC2 survival.

Alternatively, PBMC can first be enriched for BDCA-4-expressing cells using a commercially available immunomagentic selectin kit (BDCA-4 cell isolation kit, Miltenyi Biotec) according to the manufacturer's description. The BDCA-4-enriched PBMC are then stained with a fluourescent-conjugated anti-BDCA-2 mAb (Miltenyi Biotec). Double positive (BDCA2 and BDCA4) cells are then sorted by flow cytometry according to the forward light scatter characteristics and the BDCA-2 expression. All samples are handled and stored on ice until further processing. (Bave U, Magnusson M, Eloranta M-L, Perers A, Alm GV, Ronnblom L. Fc{gamma}RIIa Is Expressed on Natural IFN-{alpha}-Producing Cells (Plasmacytoid Dendritic Cells) and Is Required for the IFN-{alpha} Production Induced by Apoptotic Cells Combined with Lupus IgG J Immunol 2003; 171:3296-3302)

**IL-16 dendritic cells** (TPO dendritic cells; Della Bella, S. et al. 2004, Blood 104(13):4020-4028)

### 5.13 GENERATION OF ANTIBODIES OF MONOCLONAL ANTIBODIES TO BE USED WITH THE METHODS OF THE INVENTION

Monoclonal antibodies to be used with the methods of the invention can be prepared using a wide variety of techniques known in the art including the use of hybridoma, recombinant, and phage display technologies, or a combination thereof. For example, monoclonal antibodies can be produced using hybridoma techniques including those known in the art and taught, for example, in Harlow *et al.,* Antibodies: A Laboratory Manual, (Cold Spring Harbor Laboratory Press, 2nd ed. 1988); Hammerling, *et al.,* in: Monoclonal Antibodies and T-Cell Hybridomas, pp. 563-681 (Elsevier, N.Y., 1981) (both of which are incorporated by reference in their entireties). The term "monoclonal antibody" as used herein is not limited to antibodies produced through hybridoma technology. The term "monoclonal antibody" refers to an antibody that is derived from a single clone, including any eukaryotic, prokaryotic, or phage clone, and not the method by which it is produced.

Methods for producing and screening for specific antibodies using hybridoma technology are routine and well known in the art. In a non-limiting example, mice can be immunized with an antigen of interest, e.g., the IgG binding domain of CD32a or CD32b, or a cell expressing such an antigen. Once an immune response is detected, e.g., antibodies specific for the antigen are detected in the mouse serum, the mouse spleen is harvested and splenocytes isolated. The splenocytes are then fused by well known techniques to any suitable myeloma cells. Hybridomas are selected and cloned by limiting dilution. The hybridoma clones are then assayed by methods known in the art for cells that secrete antibodies capable of binding the antigen. Ascites fluid, which generally contains high levels of antibodies, can be generated by inoculating mice intraperitoneally with positive hybridoma clones.

In an illustrative embodiment, a monoclonal antibody that specifically binds FcγRIIB with greater affinity than said monoclonal antibodies bind FcγRIIA can be produced by a method comprising: immunizing one or more FcγRIIA transgenic mice *(See* U.S. 5,877,396 and U.S. 5,824,487) with the purified extracellular domain of human FcγRIIB, amino acids 1-180; producing hybridoma cell lines from spleen cells of said mice, screening said hybridoma cells lines for one or more hybridoma cell lines that produce antibodies that specifically bind FcγRIIB with greater affinity than said antibodies bind FcγRIIA. Antibodies that bind FcγRIIB, particularly human FcγRIIB, with a greater affinity than said monoclonal antibodies bind FcγRIIA, can be produce by a method comprising: immunizing one or more FcγRIIA transgenic mice with purified FcγRIIB or an immunogenic fragment thereof, booster immunizing said mice sufficient number of times to elicit an immune response, producing hybridoma cells lines from spleen cells of said one or more mice, screening said hybridoma cell lines for one or more hybridoma cell lines that produce antibodies that bind FcγRIIB with a greater affinity than said antibodies bind FcγRIIA. The mice can be immunized with purified FcγRIIB which has been mixed with any adjuvant known in the art to enhance immune response. Adjuvants that can be used in the methods of the invention include, but are not limited to, protein adjuvants; bacterial adjuvants, e.g., whole bacteria (BCG, Corynebacterium parvum, Salmonella minnesota) and bacterial components including cell wall skeleton, trehalose dimycolate, monophosphoryl lipid A, methanol extractable residue (MER) of tubercle bacillus, complete or incomplete Freund's adjuvant; viral adjuvants; chemical adjuvants, e.g., aluminum hydroxide, iodoacetate and cholesteryl hemisuccinateor; naked DNA adjuvants. Other adjuvants that can be used in the methods of the invention include, Cholera toxin, paropox proteins, MF-59 (Chiron Corporation; *See* also Bieg *et al.,* 1999, *Autoimmunity,* 31(1):15-24, which is incorporated herein by reference), MPL® (Corixa Corporation; *See* also Lodmell D.I. *et al.,* 2000 *Vaccine,* 18: 1059-1066; Ulrich *et al.,* 2000, *Methods in Molecular Medicine,* 273-282; Johnson *et al., 1999, Journal of Medicinal Chemistry,* 42: 4640-4649; Baldridge *et al.,* 1999 *Methods,* 19: 103-107, all of which are incorporated herein by reference), RC-529 adjuvant (Corixa Corporation; the lead compound from Corixa's aminoalkyl glucosaminide 4-phosphate (AGP) chemical library, see also www.corixa.com), and DETOX™ adjuvant (Corixa Corporation; DETOX™ adjuvant includes MPL® adjuvant (monophosphoryl lipid A) and mycobacterial cell wall skeleton; *See* also Eton *et al.,* 1998, *Clin. Cancer Res,* 4(3):619-27; and Gubta R. *et al.,* 1995, *Vaccine,* 13(14):1263-76 both of which are incorporated herein by reference.)

Antibody fragments which recognize specific epitopes may be generated by known techniques. For example, Fab and F(ab')₂ fragments may be produced by proteolytic cleavage of immunoglobulin molecules, using enzymes such as papain (to produce Fab fragments) or pepsin (to produce F(ab')₂ fragments). F(ab')₂ fragments contain the complete light chain, and the variable region, the CH1 region and the hinge region of the heavy chain.

For example, antibodies can also be generated using various phage display methods known in the art. In phage display methods, functional antibody domains are displayed on the surface of phage particles which carry the polynucleotide sequences encoding them. In a particular embodiment, such phage can be utilized to display antigen binding domains, such as Fab and Fv or disulfide-bond stabilized Fv, expressed from a repertoire or combinatorial antibody library (e.g., human or murine). Phage expressing an antigen binding domain that binds the antigen of interest can be selected or identified with antigen, e.g., using labeled antigen or antigen bound or captured to a solid surface or bead. Phage used in these methods are typically filamentous phage, including fd and M13. The antigen binding domains are expressed as a recombinantly fused protein to either the phage gene III or gene VIII protein. Examples of phage display methods that can be used to make the immunoglobulins, or fragments thereof, of the present invention include those disclosed in Brinkman *et al., J. Immunol. Methods,* 182:41-50, 1995; Ames *et al., J. Immunol. Methods,* 184:177-186, 1995; Kettleborough *et al., Eur. J. Immunol.,* 24:952-958, 1994; Persic *et al., Gene,* 187:9-18, 1997; Burton *et al., Advances in Immunology,* 57:191-280, 1994; PCT application No. PCT/GB91/01134; PCT publications WO 90/02809; WO 91/10737; WO 92/01047; WO 92/18619; WO 93/11236; WO 95/15982; WO 95/20401; and U.S. Patent Nos. 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108; each of which is incorporated herein by reference in its entirety.

As described in the above references, after phage selection, the antibody coding regions from the phage can be isolated and used to generate whole antibodies, including human antibodies, or any other desired fragments, and expressed in any desired host, including mammalian cells, insect cells, plant cells, yeast, and bacteria, e.g., as described in detail below. For example, techniques to recombinantly produce Fab, Fab' and F(ab')₂ fragments can also be employed using methods known in the art such as those disclosed in PCT publication WO 92/22324; Mullinax *et al., BioTechniques,* 12(6):864-869, 1992; and Sawai *et al., AJRI,* 34:26-34, 1995; and Better *et al., Science,* 240:1041-1043, 1988 (each of which is incorporated by reference in its entirety). Examples of techniques which can be used to produce single-chain Fvs and antibodies include those described in U.S. Patent Nos. 4,946,778 and 5,258,498; Huston *et al., Methods in Enzymology,* 203:46-88, 1991; Shu *et al., PNAS,* 90:7995-7999, 1993; and Skerra *et al., Science,* 240:1038-1040, 1988.

Phage display technology can be used to increase the affinity of an antibody to be used with the methods of the invention for activating Fc gamma receptors or inhibitory Fc gamma receptors, respectively. This technique would be useful in obtaining high affinity antibodies that could be used in the combinatorial methods of the invention. The technology, referred to as affinity maturation, employs mutagenesis or CDR walking and re-selection using an antigenic fragment to identify antibodies that bind with higher affinity to the antigen when compared with the initial or parental antibody *(See,* e.g.,Glaser *et al.,* 1992, *J. Immunology* 149:3903). Mutagenizing entire codons rather than single nucleotides results in a semi-randomized repertoire of amino acid mutations. Libraries can be constructed consisting of a pool of variant clones each of which differs by a single amino acid alteration in a single CDR and which contain variants representing each possible amino acid substitution for each CDR residue. Mutants with increased binding affinity for the antigen can be screened by contacting the immobilized mutants with labeled antigen. Any screening method known in the art can be used to identify mutant antibodies with increased avidity to the antigen (e.g., ELISA) *(See* Wu *et al.,* 1998, *Proc Natl. Acad Sci. USA* 95:6037; Yelton *et al.,* 1995, *J. Immunology 155:1994).* CDR walking which randomizes the light chain is also possible (*See* Schier *et al.,* 1996, *J*. *Mol. Bio.* 263:551).

Antibodies of the invention may be further characterized by epitope mapping, so that antibodies may be selected that have the greatest specificity for FcγRIIB compared to FcγRIIA or for FcγRIIA compared to FcγRIIB, respectively. Epitope mapping methods of antibodies are well known in the art. In certain embodiments fusion proteins comprising one or more regions of FcγRIIB or FcγRIIA, respectively, may be used in mapping the epitope of an antibody of the invention. In a specific embodiment, the fusion protein contains the amino acid sequence of a region of an FcγRIIB fused to the Fc portion of human IgG2. Each fusion protein may further comprise amino acid substitutions and/or replacements of certain regions of the receptor with the corresponding region from a homolog receptor, e.g., FcγRIIA, as shown in Table 4 below. pMGX125 and pMGX132 contain the IgG binding site of the FcγRIIB receptor, the former with the C-terminus of FcγRIIB and the latter with the C-terminus of FcγRIIA and can be used to differentiate C-terminus binding. The others have FcγRIIA substitutions in the IgG binding site and either the FcγIIA or FcγIIB N-terminus. These molecules can help determine the part of the receptor molecule where the antibodies bind.

**Table 4.** List of the fusion proteins that may be used to investigate the epitope of the monoclonal anti-FcγRIIB antibodies. Residues 172 to 180 belong to the IgG binding site of FcγRIIA and B. The specific amino acids from FcγRIIA sequence are in bold.

| Plasmid | Receptor | N-terminus | 172-180 | C-terminus |
|---|---|---|---|---|
| pMGX125 | RIIb | IIb | KKFSRSDPN | APS------SS (IIb) |
| pMGX126 | RIIa/b | IIa | QKFSRLDPN | APS------SS (IIb) |
| pMGX127 | | IIa | QKFSRLDPT | APS------SS (IIb) |
| pMGX128 | | IIb | KKFSRLDPT | APS------SS (IIb) |
| pMGX129 | | IIa | QKFSHLDPT | APS------SS (IIb) |
| pMGX130 | | IIb | KKFSHLDPT | APS------SS (IIb) |
| pMGX131 | | IIa | QKFSRLDPN | VPSMGSSS(IIa) |
| pMGX132 | | IIb | KKFSRSDPN | VPSMGSSS(IIa) |
| pMGX133 | RIIa-131R | IIa | QKFSRLDPT | VPSMGSSS(IIa) |
| pMGX134 | RIIa-131H | IIa | QKFSHLDPT | VPSMGSSS(IIa) |
| pMGX135 | | IIb | KKFSRLDPT | VPSMGSSS(IIa) |
| pMGX136 | | IIb | KKFSHLDPT | VPSMGSSS(IIa) |

The fusion proteins may be used in any biochemical assay for determination of binding to an anti-FcγRIIB antibody, e.g., an ELISA. In other embodiments, further confirmation of the epitope specificity may be done by using peptides with specific residues replaced with those from the FcγRIIA sequence.

The antibodies that can be used with the methods of the invention may be characterized for specific binding to FcγRIIB using any immunological or biochemical based method known in the art for characterizing including quantitating, the interaction of the antibody to FcγRIIB. Specific binding of an antibody to FcγRIIB may be determined for example using immunological or biochemical based methods including, but not limited to, an ELISA assay, surface plasmon resonance assays, immunoprecipitation assay, affinity chromatography, and equilibrium dialysis. Immunoassays which can be used to analyze immunospecific binding and cross-reactivity of the antibodies include, but are not limited to, competitive and non-competitive assay systems using techniques such as western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, protein A immunoassays, to name but a few. Such assays are routine and well known in the art *(see, e.g.,* Ausubel *et al.,* eds, 1994, Current Protocols in Molecular Biology, Vol. 1, John Wiley & Sons, Inc., New York, which is incorporated by reference herein in its entirety).

Antibodies that can be used with the methods of the invention may also be assayed using any surface plasmon resonance (SPR) based assays known in the art for characterizing the kinetic parameters of the interaction of the antibody with FcγRIIB or FcγRIIA, respectively. Any SPR instrument commercially available including, but not limited to, BIAcore Instruments, available from Biacore AB (Uppsala, Sweden); IAsys instruments available from Affinity Sensors (Franklin, MA.); IBIS system available from Windsor Scientific Limited (Berks, UK), SPR-CELLIA systems available from Nippon Laser and Electronics Lab (Hokkaido, Japan), and SPR Detector Spreeta available from Texas Instruments (Dallas, TX) can be used in the instant invention. For a review of SPR-based technology *see* Mullet *et al.,* 2000, *Methods* 22: 77-91; Dong *et al.,* 2002, *Review in Mol. Biotech.,* 82: 303-23; Fivash *et al.,* 1998, *Current Opinion in Biotechnology* 9: 97-101; Rich *et al.,* 2000, *Current Opinion in Biotechnology* 11: 54-61; all of which are incorporated herein by reference in their entirety. Additionally, any of the SPR instruments and SPR based methods for measuring protein-protein interactions described in U.S. Patent No.'s 6,373,577; 6,289,286; 5,322,798; 5,341,215; 6,268,125 are contemplated in the methods of the invention, all of which are incorporated herein by reference in their entirety.

Briefly, SPR based assays involve immobilizing a member of a binding pair on a surface, and monitoring its interaction with the other member of the binding pair in solution in real time. SPR is based on measuring the change in refractive index of the solvent near the surface that occurs upon complex formation or dissociation. The surface onto which the immobilization occur is the sensor chip, which is at the heart of the SPR technology; it consists of a glass surface coated with a thin layer of gold and forms the basis for a range of specialized surfaces designed to optimize the binding of a molecule to the surface. A variety of sensor chips are commercially available especially from the companies listed *supra,* all of which may be used in the methods of the invention. Examples of sensor chips include those available from BIAcore AB, Inc., e.g., Sensor Chip CM5, SA, NTA, and HPA. A molecule of the invention may be immobilized onto the surface of a sensor chip using any of the immobilization methods and chemistries known in the art, including but not limited to, direct covalent coupling via amine groups, direct covalent coupling via sulfhydryl groups, biotin attachment to avidin coated surface, aldehyde coupling to carbohydrate groups, and attachment through the histidine tag with NTA chips.

### 5.13.1 RECOMBINANT EXPRESSION OF ANTIBODIES

Once a nucleic acid sequence encoding an antibody that can be used with the methods of the invention has been obtained, the vector for the production of the antibody may be produced by recombinant DNA technology using techniques well known in the art. Methods which are well known to those skilled in the art can be used to construct expression vectors containing the antibody coding sequences and appropriate transcriptional and translational control signals. These methods include, for example, *in vitro* recombinant DNA techniques, synthetic techniques, and *in vivo* genetic recombination. *(See,* for example, the techniques described in Sambrook *et al.,* 1990, Molecular Cloning, A Laboratory Manual, 2d Ed., Cold Spring Harbor Laboratory, Cold Spring Harbor, NY and Ausubel *et al.* eds., 1998, Current Protocols in Molecular Biology, John Wiley & Sons, NY).

An expression vector comprising the nucleotide sequence of an antibody can be transferred to a host cell by conventional techniques (e.g., electroporation, liposomal transfection, and calcium phosphate precipitation) and the transfected cells are then cultured by conventional techniques to produce the antibody of the invention. In specific embodiments, the expression of the antibody is regulated by a constitutive, an inducible or a tissue, specific promoter.

The host cells used to express the recombinant antibodies of the invention may be either bacterial cells such as *Escherichia coli,* or, preferably, eukaryotic cells, especially for the expression of whole recombinant immunoglobulin molecule. In particular, mammalian cells such as Chinese hamster ovary cells (CHO), in conjunction with a vector such as the major intermediate early gene promoter element from human cytomegalovirus is an effective expression system for immunoglobulins (Foecking *et al.,* 1998, *Gene* 45:101; Cockett *et al.,* 1990, *Bio*/*Technology 8:2).*

A variety of host-expression vector systems may be utilized to express the antibodies of the invention. Such host-expression systems represent vehicles by which the coding sequences of the antibodies may be produced and subsequently purified, but also represent cells which may, when transformed or transfected with the appropriate nucleotide coding sequences, express the antibodies of the invention *in situ.* These include, but are not limited to, microorganisms such as bacteria *(e.g., E. coli* and *B. subtilis)* transformed with recombinant bacteriophage DNA, plasmid DNA or cosmid DNA expression vectors containing immunoglobulin coding sequences; yeast (e.g., *Saccharomyces Pichia)* transformed with recombinant yeast expression vectors containing immunoglobulin coding sequences; insect cell systems infected with recombinant virus expression vectors (e.g., baculovirus) containing the immunoglobulin coding sequences; plant cell systems infected with recombinant virus expression vectors (e.g., cauliflower mosaic virus (CaMV) and tobacco mosaic virus (TMV)) or transformed with recombinant plasmid expression vectors (e.g., Ti plasmid) containing immunoglobulin coding sequences; or mammalian cell systems (e.g., COS, CHO, BHK, 293, 293T, 3T3 cells, lymphotic cells *(see* U.S. *5*,807,715), Per C.6 cells (rat retinal cells developed by Crucell)) harboring recombinant expression constructs containing promoters derived from the genome of mammalian cells (e.g., metallothionein promoter) or from mammalian viruses (e.g., the adenovirus late promoter; the vaccinia virus 7.5K promoter).

In bacterial systems, a number of expression vectors may be advantageously selected depending upon the use intended for the antibody being expressed. For example, when a large quantity of such a protein is to be produced, for the generation of pharmaceutical compositions of an antibody, vectors which direct the expression of high levels of fusion protein products that are readily purified may be desirable. Such vectors include, but are not limited, to the *E. coli* expression vector pUR278 (Ruther *et al.,* 1983, *EMBO J.* 2:1791), in which the antibody coding sequence may be ligated individually into the vector in frame with the *lac* Z coding region so that a fusion protein is produced; pIN vectors (Inouye & Inouye, 1985, *Nucleic Acids Res.* 13:3101-3109; Van Heeke & Schuster, 1989, *J. Biol. Chem.* 24:5503-5509); and the like. pGEX vectors may also be used to express foreign polypeptides as fusion proteins with glutathione S-transferase (GST). In general, such fusion proteins are soluble and can easily be purified from lysed cells by adsorption and binding to a matrix glutathione-agarose beads followed by elution in the presence of free gluta-thione. The pGEX vectors are designed to include thrombin or factor Xa protease cleavage sites so that the cloned target gene product can be released from the GST moiety.

In an insect system, *Autographa californica* nuclear polyhedrosis virus (AcNPV) is used as a vector to express foreign genes. The virus grows in Spodoptera *frugiperda* cells. The antibody coding sequence may be cloned individually into non-essential regions (e.g., the polyhedrin gene) of the virus and placed under control of an AcNPV promoter (e.g., the polyhedrin promoter).

In mammalian host cells, a number of viral-based expression systems may be utilized. In cases where an adenovirus is used as an expression vector, the antibody coding sequence of interest may be ligated to an adenovirus transcription/translation control complex, e.g., the late promoter and tripartite leader sequence. This chimeric gene may then be inserted in the adenovirus genome by *in vitro* or *in vivo* recombination. Insertion in a non-essential region of the viral genome (e.g., region E1 or E3) will result in a recombinant virus that is viable and capable of expressing the immunoglobulin molecule in infected hosts. *(e.g., see* Logan & Shenk, 1984, *Proc. Natl. Acad. Sci. USA* 81:355-359). Specific initiation signals may also be required for efficient translation of inserted antibody coding sequences. These signals include the ATG initiation codon and adjacent sequences. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, *etc. (see* Bittner *et al.,* 1987, *Methods in Enzymol.* 153:51-544*).*

In addition, a host cell strain may be chosen which modulates the expression of the inserted sequences, or modifies and processes the gene product in the specific fashion desired. Such modifications (e.g., glycosylation) and processing (e.g., cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include but are not limited to CHO, VERY, BHK, Hela, COS, MDCK, 293, 293T, 3T3, WI38, BT483, Hs578T, HTB2, BT20 and T47D, CRL7030 and Hs578Bst.

For long-term, high-yield production of recombinant proteins, stable expression is preferred. For example, cell lines which stably express an antibody of the invention may be engineered. Rather than using expression vectors which contain viral origins of replication, host cells can be transformed with DNA controlled by appropriate expression control elements (e.g., promoter, enhancer, sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the antibodies of the invention. Such engineered cell lines may be particularly useful in screening and evaluation of compounds that interact directly or indirectly with the antibodies of the invention.

A number of selection systems may be used, including but not limited to the herpes simplex virus thymidine kinase *(*Wigler *et al.,* 1977, *Cell* 11:223), hypoxanthine-guanine phosphoribosyltransferase (Szybalska & Szybalski, 1992, *Proc. Natl. Acad. Sci. USA* 48:202), and adenine phosphoribosyltransferase *(*Lowy *et al.,* 1980, *Cell* 22:817) genes can be employed in tk-, hgprt- or aprt- cells, respectively. Also, antimetabolite resistance can be used as the basis of selection for the following genes: dhfr, which confers resistance to methotrexate (Wigler *et al.,* 1980, *Proc. Natl.* Acad. *Sci. USA* 77:357; O'Hare *et al.,* 1981, *Proc. Natl. Acad. Sci. USA* 78:1527); gpt, which confers resistance to mycophenolic acid (Mulligan & Berg, 1981, *Proc. Natl. Acad. Sci.* USA 78:2072); neo, which confers resistance to the aminoglycoside G-418 *Clinical Pharmacy* 12:488-505; Wu and Wu, 1991, 3:87-95; Tolstoshev, 1993, *Ann. Rev. Pharmacol. Toxicol.* 32:573-596; Mulligan, 1993, *Science* 260:926-932; and Morgan and Anderson, *1993, Ann. Rev. Biochem.* 62:191-217; May, 1993, *TIB TECH* 11(5):155-215). Methods commonly known in the art of recombinant DNA technology which can be used are described in Ausubel *et al.* (eds.), 1993, *Current Protocols in Molecular Biology,* John Wiley & Sons, NY; Kriegler, 1990, Gene Transfer and Expression, A Laboratory Manual, Stockton Press, NY; and in Chapters 12 and 13, Dracopoli *et al.* (eds), 1994, Current Protocols in Human Genetics, John Wiley & Sons, NY.; Colberre-Garapin *et al.,* 1981, *J*. *Mol. Biol.* 150:1; and hygro, which confers resistance to hygromycin (Santerre *et al.,* 1984, *Gene* 30:147).

The expression levels of an antibody of the invention can be increased by vector amplification (for a review, *see* Bebbington and Hentschel, The use of vectors based on gene amplification for the expression of cloned genes in mammalian cells in DNA cloning, Vol.3. (Academic Press, New York, 1987)). When a marker in the vector system expressing an antibody is amplifiable, increase in the level of inhibitor present in culture of host cell will increase the number of copies of the marker gene. Since the amplified region is associated with the nucleotide sequence of the antibody, production of the antibody will also increase (Crouse *et al.,* 1983, *Mol. Cell. Biol.* 3:257).

The host cell may be co-transfected with two expression vectors of the invention, the first vector encoding a heavy chain derived polypeptide and the second vector encoding a light chain derived polypeptide. The two vectors may contain identical selectable markers which enable equal expression of heavy and light chain polypeptides. Alternatively, a single vector may be used which encodes both heavy and light chain polypeptides. In such situations, the light chain should be placed before the heavy chain to avoid an excess of toxic free heavy chain (Proudfoot, 1986, *Nature* 322:52; Kohler, 1980, *Proc. Natl. Acad. Sci. USA* 77:2197). The coding sequences for the heavy and light chains may comprise cDNA or genomic DNA.

Once an antibody that can be used with the methods of the invention has been recombinantly expressed, it may be purified by any method known in the art for purification of an antibody, for example, by chromatography (e.g., ion exchange, affinity, particularly by affinity for the specific antigen after Protein A, and sizing column chromatography), centrifugation, differential solubility, or by any other standard technique for the purification of proteins.

### 5.14 ADMINISTRATION OF DENDRITIC CELLS OR T CELLS

The present invention encompasses therapies which involve administering a dendritic cell or a T cell that has been treated with a method of the invention to an animal, preferably a mammal, and most preferably a human. In some embodiments, administration of a dendritic cell or a T cell that has been treated with a method of the invention can be combined with one or more other therapies such as, but not limited to, chemotherapies, radiation therapies, hormonal therapies, and/or biological therapies/immunotherapies.

Dendritic cells or T cells may be provided in pharmaceutically acceptable compositions as known in the art or as described herein. As discussed in sections 5.10 and 5.11, matured dendritic cells and educted T cells can be used in methods of treating cancer (particularly to enhance passive immunotherapy or efficacy of a cancer vaccine); and tolerogenic dendritic cells can be used in methods of treating autoimmune disease, inflammatory disorders or allergies (e.g., to enhance efficacy of a vaccine for treatment of allergy).

In certain embodiments, a matured dendritic cell or a T cell that has been treated with a method of the invention is administered to a mammal, preferably a human, concurrently with one or more other therapeutic agents useful for the treatment of cancer. The term "concurrently" is not limited to the administration of prophylactic or therapeutic agents at exactly the same time, but rather it is meant that antibodies of the invention and the other agent are administered to a subject in a sequence and within a time interval such that the antibodies of the invention can act together with the other agent to provide an increased benefit than if they were administered otherwise. For example, each prophylactic or therapeutic agent may be administered at the same time or sequentially in any order at different points in time; however, if not administered at the same time, they should be administered sufficiently close in time so as to provide the desired therapeutic or prophylactic effect. Each therapeutic agent can be administered separately, in any appropriate form and by any suitable route.

In various embodiments, the prophylactic or therapeutic agents are administered less than 1 hour apart, at about 1 hour apart, at about 1 hour to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, no more than 24 hours apart or no more than 48 hours apart. In preferred embodiments, two or more components are administered within the same patient visit.

The dosage amounts and frequencies of administration provided herein are encompassed by the terms therapeutically effective and prophylactically effective. The dosage and frequency further will typically vary according to factors specific for each patient depending on the specific therapeutic or prophylactic agents administered, the severity and type of cancer, the route of administration, as well as age, body weight, response, and the past medical history of the patient. Suitable regimens can be selected by one skilled in the art by considering such factors and by following, for example, dosages reported in the literature and recommended in the *Physician's Desk Reference* (56^{th} ed., 2002).

### 5.15 COMPOSITIONS AND METHODS OF ADMINISTERING

The invention provides methods and pharmaceutical compositions comprising matured dendritic cells, tolerogenic dendritic cells, or T cells that have been generated using the methods of the invention. The composition formulations of the invention comprise an effective immunizing amount of the matured dendritic cells, tolerogenic dendritic cells, or T cells that have been generated using the methods of the invention. Suitable preparations of matured dendritic cells, tolerogenic dendritic cells, or T cells that have been generated using the methods of the invention include injectables, preferably as a liquid solution.

Many methods may be used to introduce the composition formulations of the invention; these include but are not limited to subcutaneous injection, intralymphatically, intradermal, intramuscular, intravenous, and via scarification (scratching through the top layers of skin, e.g., using a bifurcated needle). In a specific embodiment, compositions comprising a matured dendritic cells, tolerogenic dendritic cells, or T cells that have been generated using the methods of the invention are injected intradermally.

In addition, if desired, the composition preparation may also include minor amounts of auxiliary substances such as wetting or emulsifying agents, pH buffering agents, and/or compounds which enhance the effectiveness of the composition. The mammal to which the composition is administered is preferably a human, but can also be a non-human animal including but not limited to cows, horses, sheep, pigs, fowl (e.g., chickens), goats, cats, dogs, hamsters, mice and rats.

### 5.15.1 EFFECTIVE DOSE

The compositions can be administered to a patient at therapeutically effective doses to treat or prevent cancer or infectious disease. A therapeutically effective amount refers to that amount of the matured dendritic cells, tolerogenic dendritic cells, or T cells that have been generated using the methods of the invention sufficient to ameliorate the symptoms of such a disease or disorder, such as, e.g., regression of a tumor. Effective doses (immunizing amounts) of the compositions of the invention may also be extrapolated from dose-response curves derived from animal model test systems. The precise dose to be employed in the composition formulation will also depend on the particular type of disorder being treated. Other important considerations are the route of administration, and the nature of the patient. Thus the precise dosage should be decided according to the judgment of the practitioner and each patient's circumstances, e.g., the immune status of the patient, according to standard clinical techniques.

### 5.16 KITS

The invention also provides kits. In certain embodiments, a kit of the invention includes (i) an agent, such as an antibody, that blocks inhibitory Fc gamma receptors preferentially over activating Fc gamma receptors; and (ii) IgG. In certain embodiments, a kit of the invention includes (i) an agent, such as an antibody, that blocks CD32b preferentially over CD32a; and (ii) IgG. In a specific embodiment, the antibody that blocks CD32b preferentially over CD32a is monoclonal antibody 2B6. In a more specific embodiment, the kit further includes IL-6, IFN-gamma, and PGE2.

In certain embodiments, a kit of the invention includes (i) an agent, such as an antibody, that blocks activating Fc gamma receptors preferentially over inhibitory Fc gamma receptors; and (ii) IgG. In certain embodiments, a kit of the invention includes (i) an agent, such as an antibody, that blocks CD32a preferentially over CD32b; and (ii) IgG. In a specific embodiment, the antibody that blocks CD32a preferentially over CD32b is monoclonal antibody IV.3. In a more specific embodiment, the kit further includes soluble IgG, TGF-beta, and IFN-alpha.

The invention also provides a kit that includes (i) IL-6, IFN-gamma, PGE2, or LPS and CD40L; and (ii) IgG.

Kits of the invention can further include materials required to immobilize IgG to a solid surface and/or materials to isolate dendritic cells from a subject.

### 5.17 CHARACTERIZATION AND DEMONSTRATION OF THERAPEUTIC UTILITY

Several aspects of the pharmaceutical compositions or prophylactic or therapeutic agents of the invention are preferably tested *in vitro,* e.g., in a cell culture system, and then in vivo, e.g., in an animal model organism, such as a rodent animal model system, for the desired therapeutic activity prior to use in humans. For example, assays which can be used to determine whether administration of a specific pharmaceutical composition is indicated, include cell culture assays in which a patient tissue sample is grown in culture, and exposed to or otherwise contacted with a pharmaceutical composition, and the effect of such composition upon the tissue sample is observed, e.g., inhibition of or decrease in growth and/or colony formation in soft agar or tubular network formation in three-dimensional basement membrane or extracellular matrix preparation. The tissue sample can be obtained by biopsy from the patient. This test allows the identification of the therapeutically most effective prophylactic or therapeutic molecule(s) for each individual patient. Alternatively, instead of culturing cells from a patient, therapeutic agents and methods may be screened using cells of a tumor or malignant cell line. In various specific embodiments, *in vitro* assays can be carried out with representative cells of cell types involved in an autoimmune or inflammatory disorder (e.g., T cells), to determine if a pharmaceutical composition of the invention has a desired effect upon such cell types. Many assays standard in the art can be used to assess such survival and/or growth; for example, cell proliferation can be assayed by measuring ³H-thymidine incorporation, by direct cell count, by detecting changes in transcriptional activity of known genes such as proto-oncogenes (e.g., fos, myc) or cell cycle markers; cell viability can be assessed by trypan blue staining, differentiation can be assessed visually based on changes in morphology, decreased growth and/or colony formation in soft agar or tubular network formation in three-dimensional basement membrane or extracellular matrix preparation, etc.

Combinations of prophylactic and/or therapeutic agents can be tested in suitable animal model systems prior to use in humans. Such animal model systems include, but are not limited to, rats, mice, chicken, cows, monkeys, pigs, dogs, rabbits, *etc.* Any animal system well-known in the art may be used. In a specific embodiment of the invention, combinations of prophylactic and/or therapeutic agents are tested in a mouse model system. Such model systems are widely used and well-known to the skilled artisan. Prophylactic and/or therapeutic agents can be administered repeatedly. Several aspects of the procedure may vary such as the temporal regime of administering the prophylactic and/or therapeutic agents, and whether such agents are administered separately or as an admixture.

Once the prophylactic and/or therapeutic agents of the invention have been tested in an animal model they can be tested in clinical trials to establish their efficacy. Establishing clinical trials will be done in accordance with common methodologies known to one skilled in the art, and the optimal dosages and routes of administration as well as toxicity profiles of the compositions of the invention can be established using routine experimentation.

The anti-inflammatory activity of the combination therapies of invention can be determined by using various experimental animal models of inflammatory arthritis known in the art and described in Crofford L.J. and Wilder R.L., "Arthritis and Autoimmunity in Animals", in Arthritis and Allied Conditions: A Textbook of Rheumatology, McCarty *et al*.(eds.). Chapter 30 (Lee and Febiger, 1993). Experimental and spontaneous animal models of inflammatory arthritis and autoimmune rheumatic diseases can also be used to assess the anti-inflammatory activity of the combination therapies of invention. The following are some assays provided as examples, and not by limitation.

The principle animal models for arthritis or inflammatory disease known in the art and widely used include: adjuvant-induced arthritis rat models, collagen-induced arthritis rat and mouse models and antigen-induced arthritis rat, rabbit and hamster models, all described in Crofford L.J. and Wilder R.L., "Arthritis and Autoimmunity in Animals", in Arthritis and Allied Conditions: A Textbook of Rheumatology, McCarty *et al*.(eds.), Chapter 30 (Lee and Febiger, 1993), incorporated herein by reference in its entirety.

The anti-inflammatory activity of the combination therapies of invention can be assessed using a carrageenan-induced arthritis rat model. Carrageenan-induced arthritis has also been used in rabbit, dog and pig in studies of chronic arthritis or inflammation. Quantitative histomorphometric assessment is used to determine therapeutic efficacy. The methods for using such a carrageenan-induced arthritis model is described in Hansra P. *et al.,* "Carrageenan-Induced Arthritis in the Rat," *Inflammation,* 24(2): 141-155, (2000). Also commonly used are zymosan-induced inflammation animal models as known and described in the art.

The anti-inflammatory activity of the combination therapies of invention can also be assessed by measuring the inhibition of carrageenan-induced paw edema in the rat, using a modification of the method described in Winter C. A. *et al.,* "Carrageenan-Induced Edema in Hind Paw of the Rat as an Assay for Anti-inflammatory Drugs" *Proc. Soc. Exp. Biol Med.* 111, 544-547, (1962). This assay has been used as a primary *in vivo* screen for the anti-inflammatory activity of most NSAIDs, and is considered predictive of human efficacy. The anti-inflammatory activity of the test prophylactic or therapeutic agents is expressed as the percent inhibition of the increase in hind paw weight of the test group relative to the vehicle dosed control group.

Additionally, animal models for inflammatory bowel disease can also be used to assess the efficacy of the combination therapies of invention (Kim *et al.,* 1992, Scand. *J. Gastroentrol.* 27:529-537; Strober, 1985, *Dig. Dis. Sci.* 30(12 Suppl):3S-10S). Ulcerative cholitis and Crohn's disease are human inflammatory bowel diseases that can be induced in animals. Sulfated polysaccharides including, but not limited to amylopectin, carrageen, amylopectin sulfate, and dextran sulfate or chemical irritants including but not limited to trinitrobenzenesulphonic acid (TNBS) and acetic acid can be administered to animals orally to induce inflammatory bowel diseases.

Animal models for asthma can also be used to assess the efficacy of the combination therapies of invention. An example of one such model is the murine adoptive transfer model in which aeroallergen provocation of TH1 or TH2 recipient mice results in TH effector cell migration to the airways and is associated with an intense neutrophilic (TH1) and eosinophilic (TH2) lung mucosal inflammatory response (Cohn *et* al., 1997, *J.* Exp. *Med.* 1861737-1747).

Animal models for autoimmune disorders can also be used to assess the efficacy of the combination therapies of invention. Animal models for autoimmune disorders such as type 1 diabetes, thyroid autoimmunity, systemic lupus eruthematosus, and glomerulonephritis have been developed (Flanders *et al.,* 1999, *Autoimmunity* 29:235-246; Krogh *et al.,* 1999, *Biochimie* 81:511-515; Foster, 1999, *Semin. Nephrol.* 19:12-24).

Further, any assays known to those skilled in the art can be used to evaluate the prophylactic and/or therapeutic utility of the combinatorial therapies disclosed herein for autoimmune and/or inflammatory diseases.

Toxicity and efficacy of the prophylactic and/or therapeutic protocols of the instant invention can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Prophylactic and/or therapeutic agents that exhibit large therapeutic indices are preferred. While prophylactic and/or therapeutic agents that exhibit toxic side effects may be used, care should be taken to design a delivery system that targets such agents to the site of affected tissue in order to minimize potential damage to uninfected cells and, thereby, reduce side effects.

The data obtained from the cell culture assays and animal studies can be used in formulating a range of dosage of the prophylactic and/or therapeutic agents for use in humans. The dosage of such agents lies preferably within a range of circulating concentrations that include the ED₅₀ with little or no toxicity. The dosage may vary within this range depending upon the dosage form employed and the route of administration utilized. For any agent used in the method of the invention, the therapeutically effective dose can be estimated initially from cell culture assays. A dose may be formulated in animal models to achieve a circulating plasma concentration range that includes the IC₅₀ *(i.e.,* the concentration of the test compound that achieves a half-maximal inhibition of symptoms) as determined in cell culture. Such information can be used to more accurately determine useful doses in humans. Levels in plasma may be measured, for example, by high performance liquid chromatography.

The anti-cancer activity of the therapies used in accordance with the present invention also can be determined by using various experimental animal models for the study of cancer such as the SCID mouse model or transgenic mice or nude mice with human xenografts, animal models, such as hamsters, rabbits, *etc.* known in the art and described in *Relevance of Tumor Models for Anticancer Drug Development* (1999, eds. Fiebig and Burger); *Contributions to Oncology* (1999, Karger); *The Nude Mouse in Oncology Research* (1991, eds. Boven and Winograd); and *Anticancer Drug Development Guide* (1997 ed. Teicher), herein incorporated by reference in their entireties.

Further, any assays known to those skilled in the art can be used to evaluate the prophylactic and/or therapeutic utility of the combinatorial therapies disclosed herein for treatment or prevention of cancer, inflammatory disorder, or autoimmune disease.

### 6. EXAMPLES

### 6.1 INTRODUCTION

Monoclonal antibodies (mAb) are among the most rapidly growing therapies for the treatment of cancer¹ and autoimmunity². The common perception is that antibodies bind target cells and either fix complement or engage cells of the innate immune system to mediate target cell lysis.³ The latter, known as antibody-dependent cellular cytotoxicity (ADCC), requires that the Fc portion of a mAb binds or ligates activating immunoglobulin (Ig) Fc receptors (FcγRs), e.g., FcγRI (CD64), FcγRIIa (CD32a), FcγRIIc (CD32c), or FcγRIII (CD16), on monocytes, NK cells, neutrophils, or dendritic cells (DCs)^{4,5}, whereas co-ligation of the unique inhibitory FcγR, CD32b, abrogates these effects.⁶ ADCC is largely mediated by NK cells in vitro⁷, though recent evidence revealed monocytes to be the predominant effectors in vivo⁴.

Several lines of evidence show that optimum immune rejection of tumors or infectious pathogens requires coordinated cellular and humoral immune responses. In a mouse tumor model, efficacy of an anti-tumor mAb requires the presence of both tumor-specific CD8⁺ T cells and CD11b+, FcγR+ phagocytic cells.⁸ This was supported by the demonstration that coating tumor cells with mAbs leads to FcγR-dependent enhancement of antigen presentation and T cell stimulation by DCs.⁹ FcγRs on DCs thus link humoral immunity with the most potent stimulators of innate and adaptive cellular immunity. Activating FcγRs on DCs mediate phagocytosis¹⁰ and enhanced cross-presentation¹¹ of antibody-coated antigens, leading to effective stimulation of both Th1 CD4 and CD8 effector responses.¹² In mice, co-ligation of the inhibitory FcγR, CD32b, limits these processes.¹³

Alterations in the balance between activating and inhibitory FcγRs can have profound impact on immunity. For example, CD32b-deficient mice are predisposed to spontaneous autoimmunity, and generate pathologically enhanced responses to vaccinations ^{14, 15}. More indirect evidence comes from clinical studies in which common genetic polymorphisms of activating FcγRs with greater avidity for human IgG are associated with improved clinical outcomes in patients with follicular non-Hodgkin's lymphoma treated with an anti-CD20 mAb, rituximab.^{16,17}

The coordinated expression and function of these receptors therefore merit investigation, especially on dendritic cells. DCs and their activating or inhibitory FcγRs offer rational targets for immunotherapy, because DCs play critical roles in immunity and tolerance, respectively.¹⁸ Thus, compositions and methods for use in regulating DC-mediated immunity would be desirable.
1. Waldmann TA. Immunotherapy: past, present and future. Nat Med 2003; 9:269-77.
2. Edwards JCW, Szczepanski L, Szechinski J, Filipowicz-Sosnowska A, Emery P, Close DR, Stevens RM, Shaw T. Efficacy of B-Cell-Targeted Therapy with Rituximab in Patients with Rheumatoid Arthritis. N Engl J Med 2004; 350:2572-2581.
3. Cartron G, Watier H, Golay J, Solal-Celigny P. From the bench to the bedside: ways to improve rituximab efficacy. Blood 2004; 104:2635-2642.
4. Uchida J, Hamaguchi Y, Oliver JA, Ravetch JV, Poe JC, Haas KM, Tedder TF. The innate mononuclear phagocyte network depletes B lymphocytes through Fc receptor-dependent mechanisms during anti-CD20 antibody immunotherapy. J Exp Med 2004; 199:1659-69.
5. Schmitz M, Zhao S, Schakel K, Bornhauser M, Ockert D, Rieber EP. Native human blood dendritic cells as potent effectors in antibody-dependent cellular cytotoxicity. Blood 2002; 100:1502-1504.
6. Clynes RA, Towers TL, Presta LG, Ravetch JV. Inhibitory Fc receptors modulate in vivo cytoxicity against tumor targets. Nat Med 2000; 6:443-6.
7. Niwa R, Hatanaka S, Shoji-Hosaka E, Sakurada M, Kobayashi Y, Uehara A, Yokoi H, Nakamura K, Shitara K. Enhancement of the Antibody-Dependent Cellular Cytotoxicity of Low-Fucose IgG1 Is Independent of Fc{gamma}RIIIa Functional Polymorphism. Clin Cancer Res 2004; 10:6248-6255.
8. Dyall R, Vasovic LV, Clynes RA, Nikolic-Zugic J. Cellular requirements for the monoclonal antibody-mediated eradication of an established solid tumor. Eur J Immunol 1999; 29:30-7.
9. Dhodapkar KM, Krasovsky J, Williamson B, Dhodapkar MV. Antitumor Monoclonal Antibodies Enhance Cross-Presentation of Cellular Antigens and the Generation of Myeloma-specific Killer T Cells by Dendritic Cells. J. Exp. Med. 2002; 195:125-133.
10. Fanger NA, Wardwell K, Shen L, Tedder TF, Guyre PM. Type 1 [CD64] and type 11 [CD32] Fc gamma receptor-mediated phagocytosis by human blood dendritic cells. J.Immunol. 1996; 157:541-548.
11. Regnault A, Lankar D, Lacabanne V, Rodriguez A, Thery C, Rescigno M, Saito T, Verbeek S, Bonnerot C, Ricciardi-Castagnoli P, Amigorena S. Fcgamma receptor-mediated induction of dendritic cell maturation and major histocompatibility complex class I-restricted antigen presentation after immune complex internalization. J Exp Med 1999; 189:371-80.
12. Regnault A, Lankar D, Lacabanne V, Rodriguez A, Thery C, Rescigno M, Saito T, Verbeek S, Bonnerot C, Ricciardi-Castagnoli P, Amigorena S. Fcgamma Receptor-mediated Induction of Dendritic Cell Maturation and Major Histocompatibility Complex Class I-restricted Antigen Presentation after Immune Complex Internalization. J Exp Med 1999; 189:371-380.
13. Kalergis AM, Ravetch JV. Inducing tumor immunity through the selective engagement of activating Fcgamma receptors on dendritic cells. J Exp Med 2002; 195:1653-9.
14. Yajima K, Nakamura A, Sugahara A., Takai T. FcgammaRIIB deficiency with Fas mutation is sufficienct for the develpment of systemic autoimmune disease. European Journal of Immunology 2003; 33:1020-9.
15. Clatworthy MR, Smith KG. FcgammaRIIb balances efficient pathogen clearance and the cytokine-mediated consequences of sepsis. J Exp Med 2004; 199:717-23.
16. Weng WK, Levy R. Two immunoglobulin G fragment C receptor polymorphisms independently predict response to rituximab in patients with follicular lymphoma. J Clin Oncol 2003; 21:3940-7.
17. Cartron G, Dacheux L, Salles G, Solal-Celigny P, Bardos P, Colombat P, Watier H. Therapeutic activity of humanized anti-CD20 monoclonal antibody and polymorphism in IgG Fc receptor FcgammaRIIIa gene. Blood 2002; 99:754-8.
18. Steinman RM, Nussenzweig MC. Avoiding horror autotoxicus: the importance of dendritic cells in peripheral T cell tolerance. Proc Natl Acad Sci U S A 2002; 99:351-8.

### 6.2 RESULTS

We have used a recently developed monoclonal antibody, which can target only the inhibitory CD32b isoform on intact human cells. We have evaluated the relative expression of the activating CD 16, CD32a, and CD64, as well as the inhibitory CD32b, on all currently known human dendritic cell types. We have identified factors that regulate the expression of these receptors, which in turn affect the IgG-mediated changes in phenotypic maturation and activation of the dendritic cells themselves. Finally, we have demonstrated the functional sequelae of targeting either or both the activating CD32a and inhibitory CD32b with respect to dendritic cell immunogenicity. Our findings have important implications for determining or optimizing efficacy of targeted antibody therapies.

### Specific Monoclonal Antibodies Identify CD32 Isoforms and CD32a Allelic Variants by Flow Cytometry

We first validated specificity of MAbs for this study using neutrophils and B cells that express CD32a or CD32b, respectively, as the exclusive isoform of CD32. The novel murine MAb clone 2B6, which detects an extracellular domain of CD32b exclusive of CD32a binding, stained B cells (B) but not neutrophils (N) (Figure 1A). Clone FL18.26 is not isoform-specific¹, ² and stained neutrophils and B cells (Figure 1B). In contrast CD32a-specific Fab' fragments of clone IV.3³, ⁴ detected neutrophils, but not B cells (Figure 1C). These data confirm the specificity of 2B6 for CD32b, which obviates the confused detection of activating and inhibitory isoforms of CD32.

An arginine (R) to histidine (H) amino acid substitution at position 131 of CD32a yields polymorphic variants with differing avidities for mouse and human IgG.5 MAbs 3D3 and 41H16 recognize only the R131 variant of CD32a,^{6,7} whereas MAb FL18.26 recognizes R131 and H131 variants.^{1,2} For all samples we compared staining of CD32a on neutrophils by MAbs 3D3 or 41H16 and FL18.26. As shown in Figure 1D, FL18.26 stained neutrophils from all CD32a phenotypes with equal intensity. In contrast, 3D3 stained neutrophils from homozygous R/R131 individuals, did not detect neutrophils from homozygous H/H131 individuals, and had intermediate staining of heterozygous H/R samples. These data validate this quick method for distinguishing the polymorphic phenotypes of CD32a by flow cytometry.^{8,9}

### Subtypes of Freshly Isolated DCs and DC Precursors Have Distinct FcγR Expression Profiles

We studied the differential expression of the activating and inhibitory FcγRs on freshly isolated populations of dendritic cells (DCs) and their precursors in peripheral blood. We identified myeloid blood DCs (DC1) from freshly isolated PBMCs as lineage (CD3, CD 14, CD20, and CD56)^{negative}, HLA-DR^{bright}, and CD123^{low}, whereas precursors to plasmacytoid DCs (pDC2) were lineage ^{negative}, HLADR^{bright} and CD123^{bright 10}. As published, we found that most DC1 in peripheral blood co-expressed the activating FcγRs CD32a and CD64, but lacked expression of CD16.¹¹ We further determined that almost all DC1 also expressed the inhibitory CD32b (n=10, mean= 92%, SD=3.3) (figure 2A). Conversely, freshly isolated pDC2 did not express detectable levels of any FcγRs (Figure 2A). CD14⁺ monocytes may differentiate into immature DC1 and are designated DC1 precursors (pDCl)¹². All monocytes expressed CD32a and CD64 (Figure 2B). Among a sampling of 30 healthy volunteers, monocyte co-expression of the inhibitory CD32b was variable, ranging from 1% to 48% (mean= 18.1 %, SD= 8.3). The ratio of monocytes expressing only activating FcγRs to those also co-expressing inhibitory FcγRs therefore ranged from 1:1 to 99:1. A separate, but overlapping, small subpopulation of monocytes expressed CD 16 (Figure 2B).

### MoDCs Express a Pattern of FcγRs Similar to DC1 in Fresh Blood

Macrophages cultured from plastic-adherent monocytes in 10% normal human serum (NHS)-RPMI without additional cytokines expressed FcγR profiles similar to monocytes. Immature monocyte-derived DCs (moDCs) cultured from identical precursors but in the presence of GM-CSF and IL-4 were similar to DC1 in fresh blood. Approximately 50% of moDCs expressed CD32a and 50% expressed CD32b (Figure 2B). Most often these divergent receptors were co-expressed on the same population of cells (Figure 2C). Unlike their monocyte precursors, moDCs lost expression of CD16 and CD64 by day 1-2.

Other have demonstrated that LCs in situ express CD32a, but that CD32a is shed upon cell culture/activation.¹³ We found that CD34⁺hematopoietic progenitor cell (HPC)-derived Langerhans cells (LCs) and dermal/interstitial dendritic cells (DDCs/IDCs) lacked detectable expression of any FcγRs (Figure 2B). These data reveal that immature moDCs display both CD32a and CD32b and thus serve as an excellent system in which to study the modulation and function of these divergent receptors.

### Various Stimuli Modulate the Balanced Expression of CD32a and CD32b on Immature moDCs

Based on the range of CD32b surface expression on monocytes among a group of healthy donors, we identified other factors that modulated the cell surface expression of this and other FcγRs using flow cytometry. We studied immature moDCs, which have balanced co-expression of both activating and inhibitory FcγRs by day 3 of culture. All cultures used 1% NHS, except where indicated. Figure 3A shows mean fold changes in the percentage of cells expressing the respective FcγRs (A), and the relative changes in FcγR density on the cell surface (B) compared with untreated moDCs from the same donors (n= 6 independent experiments). FcγR density was measured as the number of anti-CD32a or anti-CD32b detection antibodies bound per cell. MFIs and shifts in MFIs were identical for Fab' and anti- FcγR antibodies, indicating that FcγR staining was mediated by Fab'-specific binding, and not by interactions with the Fc portions of the detection antibodies.

All factors affected only CD32a and/or CD32b expression, or neither, except IL-10 and IFNγ, which induced expression of CD 16 and CD64, respectively. IL-10 and IL-6 led to proportional increases in expression of both activating and inhibitory FcγRs without a clear shift favoring either isoform. IFNγ most potently shifted the balance in favor of activating FcγRs by increasing the frequency and density of CD32a expression, and by exerting opposing effects on CD32b. PGE-2 also potently increased CD32a expression, though it had less suppressive effects on CD32b. Conversely, therapeutic concentrations of soluble monomeric IgG (0.15mM) decreased CD32a and slightly increased CD32b, leading to the greatest relative shift in favor of the inhibitory FcγRs. The low affinity receptors did not bind monomeric IgG, which was not detected on the surface with anti-human IgG antibodies (not shown), so receptor occupancy could not account for any change in detection of CD32 isoforms. IL-2, which is studied as an adjuvant to MAb therapies,¹⁴ yielded no clear shift in favor of either type of FcγR. IFNα has also been studied as an adjuvant to MAb therapy¹⁵ and led to a modest shift in favor of CD32b. Culturing cells in the presence of 10% fetal calf serum (FCS) or TNFα both potently reduced the frequency and density of FcγR expression. All tested maturation stimuli decreased the frequency of CD32a- and CD32b-expressing cells. However, LPS and CD40L, but not the combination of IL1β, IL-6, TNFα, and PGE2,¹⁶ shifted the balance of remaining FcγR -expressing cells in favor of CD32a.

Most factors that affected CD32a and/or CD32b modulated both frequency and density of expression for a given FcγR. Some factors discordantly affected these parameters. For example, TGFβ similarly decreased density of CD32a and CD32b, but yielded an overall increase in the frequency of CD32b⁺ cells. Conversely, maturation with CD40L or LPS both decreased the frequency of CD32a and CD32b expression, but led to relatively greater increases in CD32a density per cell.

We tested the effects of factors on DC1 and pDC2 enriched from whole blood after negative selection and cultured in Teflon beakers in 10% NHS-RPMI. We added IL-3 to support the viability of pDC2 in culture.¹⁷ We noted similar modulations of CD32a and/or CD32b on DC1 upon adding IFNα, IFNγ, TGFβ, IL-10, or soluble IgG to cultures. Unlike freshly isolated pDC2, pDC2 in culture expressed CD32a, which was modulated by these factors, as well (data not shown). None of the tested factors was able to induce detectable FcγR expression on CD34⁺ HPC-derived DDC/IDC or LCs (data not shown).

### CD32a and CD32b Have Opposing Effects on DC Maturation

We studied the effects of ligating human IgG to CD32a, CD32b, or both, using immobilized IgG as a model of complexed/bound IgG.¹⁸ We incubated immature moDCs with blocking MAbs against CD32a or CD32b for 30 minutes at 4°C. We then targeted the unblocked FcγR by reculturing moDCs in GM-CSF and IL-4 at 37°C in 96-well plates pre-treated with immobilized human IgG. We targeted ligation of both CD32a and CD32b simultaneously by pre-incubating moDCs with isotype control antibodies that would not bind/block FcγRs or by pre-incubation in the absence of antibodies. For control conditions without FcγR ligation, we re-cultured moDCs on untreated plates, or on plates pre-treated with immobilized Fab fragments of IgG.

Selective targeting of CD32a on immature DCs led to maturation of a DC subpopulation, as evidenced by upregulation of the DC-maturation marker, CD83,¹⁹ and simultaneous down-regulation of the inhibitory molecule, ILT3²⁰(Figure 4A, Left). The frequency of moDC maturation was proportional to the percentage of cells that expressed CD32a. Targeting CD32b alone did not significantly affect DC maturation and led to a slight increase in expression of ILT3 (Figure A, middle). When targeted simultaneously, CD32b limited CD32a-induced maturation (Figure 4A, right).

### Cytokine-or Soluble IgG-Mediated Shifts In the Balance Between Activating and Inhibitory FcγRs Are Significant for IgG -Mediated Maturation Effects

We pre-treated moDCs with factors that modulated the balance in favor of either activating or inhibitory FcγRs. IFNγ treatment supported expression of the CD32a, and soluble IgG favored CD32b, as already shown in Figure 3. We first incubated IFNγ-treated DCs with a blocking mAb against CD64, which was induced by IFNγ. Culturing IFNγ-treated DCs in the presence of immobilized IgG led to significant increases in DC maturation compared with cultures lacking immobilized IgG (Figure 4b). Conversely, immobilized IgG did not significantly affect maturation and led to increased ILT3 expression on moDCs pre-treated with soluble IgG or IFNα (not shown). (One representative experiment of three is shown).

### Effectual Modulation of the CD32a/CD32b is Brought About By Differences in Ligand (IgG) Affinity

On average, targeting human immobilized IgG to CD32a and CD32b on moDCs from HH or HR individuals yielded a greater increase in maturation compared with moDCs from RR individuals (averages= 27% vs. 9%, respectively, p=.007). (Figure 4C). Conversely, the use of immobilized mouse IgG in the same individuals led to substantial maturation and a loss of the discrepancy between the different CD32a131 phenotypes.

### Co-ligation of CD32b Limits CD32a-Mediated Cytokine Release

After targeting of CD32a, CD32b, or both, by immobilized IgG as outlined above, we collected cell-free supernatants at 24-48 hours and measured a panel of cytokines using a multiplexed bead assay. Simultaneous targeting of CD32b and CD32a let to suppressed levels of inflammatory cytokines compared with targeting CD32a alone. Samples from individuals bearing the HH and HR type of CD32a are shown in Figure 5A. Differences between conditions in which CD32a and CD32b were targeted simultaneously, compared with conditions in which CD32a was targeted alone, were statistically significant by the paired t-test for IL8 secretion and TNFα secretion.

We similarly tested cytokine release following ligation of immobilized human IgG to FcγRs on INFγ-and soluble IgG-treated immature moDCs, which express a predominance of CD32a and CD32b, respectively. Co-culturing IgG treated DCs with immobilized IgG did not lead to increased secretion of IL-8 or TNFa (Figure 5B). Conversely, the enhanced secretion of IL-8 and TNFa after co-culture with immobilized IgG was greater for IFNγ -treated DCs than it was for untreated DCs (Figure 5B), and was more similar to the condition of CD32b blockade of untreated DCs (Figure 5A).

### The HH/HR vs. RR Phenotypes Have Functional Significance in Cytokine Release Assays

We compared cytokine release after targeting FcγRs from RR vs. HH and HR individuals. (Results from HH and HR individuals are shown in Figure 5A.) Cytokine release was not significantly enhanced after co-culturing immature moDCs from RR individuals with immobilized human IgG (Figure 5C), and results were not enhanced by pre-treating with anti-CD32b (not shown). However, since the RR subtype has a high affinity for murine IgG1,⁵ using immobilized mouse IgG as a ligand for these samples led to marked increase in TNFα and IL-8 that was similar to DCs from HH or HR individuals pre-treated with anti-CD32b (Figures 5A, 5C). Blocking CD32b in these samples did not further enhance cytokine relase (not shown), as mouse IgG does not bind human CD32b.

### Targeting CD32a vs. CD32b Has Opposing Effects on DC Allo-stimulatory Capacity In Allogeneic Mixed Leukocyte Reaction (MLR)

We ligated CD32a, CD32b, both, or neither, on day 5 immature moDCs as described above. After 2 days, cells were harvested and washed. MoDCs were re-plated without additional cytokines at a range of doses in triplicate with a fixed number of allogeneic T cells. After 4-5 days, 3H-TdR uptake by proliferating T cells was measured as an index of DC immunogenicity. CD32a-targeted DCs were the most potent stimulators of allogeneic T cells (upward triangles). Co-targeting of CD32b limited the absolute increase in stimulatory capacity mediated by targeting CD32a (circles). Targeting CD32b (downward triangles) exerted little to no change in DC allo-stimulatory function compared to untreated controls (squares).

### The HH/HR vs. RR Phenotypes Have Functional Significance For Allogeneic MLRs

We compared stimulatory capacity after targeting FCγRs from RR vs. HH and HR individuals. (Results from HH and HR individuals are shown in Figure 6A. Stimulation of allogeneic T cells was not significantly enhanced after co-culturing immature moDCs from RR individuals with immobilized human IgG (Figure 6B), and results were not enhanced by pre-treating with anti-CD32b (not shown). Co-culturing these samples with immobilized mouse IgG, however, led to marked increases in stimulation of allogeneic T cells. Again, no added effects were noted upon blocking CD32b, which does not bind mouse IgG.
1. Schlossman SF, Boumsell L, Gilks W, Harlan JM, Kishimoto T, Morimoto C, Ritz J, Shaw S. Silverstein RL, Springer TA, Tedder TF, Todd RF. Leucocyte typing V: White cell differentiation antigens, Leucocyte typing V: White cell differentiation antigens., 1995. Oxford University Press.
2. Micklem KJ, Stross WP, Willis AC, Cordell JL, Jones M, Mason DY. Different isoforms of human FcRII distinguished by CDw32 antibodies. J Immunol 1990; 144:2295-303.
3. Maresco DL, Osborne JM, Cooney D, Coggeshall KM, Anderson CL. The SH2-Containing 5'-Inositol Phosphatase (SHIP) Is Tyrosine Phosphorylated after Fc{gamma} Receptor Clustering in Monocytes. J Immunol 1999; 162:6458-6465.
4. Gamberale R, Geffner JR, Sanjurjo J, Fernandez-Calotti PX, Arrosagaray G, Avalos JS, Giordano M. Expression of Fc{gamma} receptors type II (Fc{gamma}RII) in chronic lymphocytic leukemia B cells. Blood 2003; 102:2698-2699.
5. Van Sorge NM, Van der Pol WL, Van de Winkel JG. FcgammaR polymorphisms: Implications for function, disease susceptibility and immunotherapy. Tissue Antigens 2003; 61:189-202.
6. Vely F, Gruel N, Moncuit J, Cochet O, Rouard H, Dare S, Galon J, Sautes C, Fridman WH, Teillaud JL. A new set of monoclonal antibodies against human Fc gamma RII (CD32) and Fcgamma RIII (CD16): characterization and use in various assays. Hybridoma 1997; 16:519-28.
7. Gosselin E, Brown M, Anderson C, Zipf T, Guyre P. The monoclonal antibody 41H16 detects the Leu 4 responder form of human Fc gamma RII. J Immunol 1990; 144:1817-1822.
8. Parren PW, Warmerdam PA, Boeije LC, Arts J, Westerdaal NA, Vlug A, Capel PJ, Aarden LA, van de Winkel JG. On the interaction of IgG subclasses with the low affinity Fc gamma RIIa (CD32) on human monocytes, neutrophils, and platelets. Analysis of a functional polymorphism to human IgG2. J Clin Invest 1992; 90:1537-46.
9. Tse WY, Abadeh S, McTiernan A, Jefferis R, Savage COS, Adu D. No association between neutrophil FcgammaRIIa allelic polymorphism and anti-neutrophil cytoplasmic antibody (ANCA)-positive systemic vasculitis. Clin Exp Immunol 1999; 117:198-205.
10. Siegal FP, Kadowaki N, Shodell M, Fitzgerald-Bocarsly PA, Shah K, Ho S, Antonenko S, Liu YJ. The nature of the principal type 1 interferon-producing cells in human blood. Science 1999; 284:1835-7.
11. Fanger NA, Wardwell K, Shen L, Tedder TF, Guyre PM. Type 1 [CD64] and type 11 [CD32] Fc gamma receptor-mediated phagocytosis by human blood dendritic cells. J.Immunol. 1996; 157:541-548.
12. Rissoan MC, Soumelis V, Kadowaki N, Grouard G, Briere F, de Waal Malefyt R, Liu YJ. Reciprocal control of T helper cell and dendritic cell differentiation. Science 1999; 283:1183-6.
13. Astier A, de la Salle H, de la Salle C, Bieber T, Esposito-Farese M-E, Freund M, Cazenave JP, Fridman W-H, Teillaud J-L, Hanau D. Human epidermal langerhans cells secrete a soluble receptor for IgG (FctR11/CD32) that inhibits the binding of immune complexes to FctR+ cells. J.Immunol. 1994; 152:201-212.
14. Eisenbeis CF, Grainger A, Fischer B, Baiocchi RA, Carrodeguas L, Roychowdhury S, Chen L, Banks AL, Davis T, Young D, Kelbick N, Stephens J, Byrd JC, Grever MR, Caligiuri MA, Porcu P. Combination Immunotherapy of B-Cell Non-Hodgkin's Lymphoma with Rituximab and Interleukin-2: A Preclinical and Phase I Study. Clin Cancer Res 2004; 10:6101-6110.
15. Davis TA, Maloney DG, Grillo-Lopez AJ, White CA, Williams ME, Weiner GJ, Dowden S, Levy R. Combination immunotherapy of relapsed or refractory low-grade or follicular non-Hodgkin's lymphoma with rituximab and interferon-alpha-2a. Clin Cancer Res 2000; 6:2644-52.
16. Jonuleit H, Kuhn U, Muller G, Steinbrink K, Paragnik L, Schmitt E, Knop J, Enk AH. Proinflammatory cytokines and prostaglandins induce maturation of potent Immunostimulatory dendritic cells under fetal calf serum-free conditions. Eur.J.Immunol. 1997; 27:3135-3142.
17. Grouard G, Rissoan M-C, Filgueira L, Durand I, Banchereau J, Liu Y-J. The enigmatic plasmacytoid T cells develop into dendritic cells with IL-3 and CD40-ligand. J.Exp.Med. 1997; 185:1101-1111.
18. Banki Z, Kacani L, Mullauer B, Wilflingseder D, Obermoser G, Niederegger H, Schennach H, Sprinzl GM, Sepp N, Erdei A, Dierich MP, Stoiber H. Cross-Linking of CD32 Induces Maturation of Human Monocyte-Derived Dendritic Cells Via NF-kappaB Signaling Pathway. J Immunol 2003; 170:3963-3970.
19. Zhou L-J, Tedder TF. Human blood dendritic cells selectively express CD83, a member of the immunoglobulin superfamily. J.Immunol. 1995; 154:3821-3835.
20. Cella M, Dohring C, Samaridis J, Dessing M, Brockhaus M, Lanzavecchia A, Colonna M. A Novel Inhibitory Receptor (ILT3) Expressed on Monocytes, Macrophages, and Dendritic Cells Involved in Antigen Processing. J. Exp. Med. 1997; 185:1743-1751.

## Claims

1. A method for promoting the maturation of an immature dendritic cell, wherein the method comprises:
a) contacting the immature dendritic cell with an anti-CD32b antibody that blocks ligation of CD32b but not ligation of CD32a; and
b) activating CD32a signaling in the immature dendritic cell.

2. A method for promoting the maturation of an immature dendritic cell, wherein the method comprises contacting the immature dendritic cell with an anti-CD32b antibody that blocks ligation of CD32b but not ligation of CD32a, wherein the anti-CD32b antibody is an IgG.

3. The method of claim 1, wherein CD32a signaling is activated by contacting the immature dendritic cell with complexed or immobilized IgG.

4. A method for promoting the maturation of a population of immature dendritic cells, wherein the method comprises:
a) enriching CD32a-expressing cells in the population; and
b) activating CD32a signaling in cells of the population resulting from step (a).

5. The method of claim 4, wherein the method further comprises inhibiting CD32b signaling in cells of the population resulting from step (a).

6. The method of claim 5, wherein CD32b signaling is inhibited by contacting the cells with an antagonist of CD32b signaling.

7. The method of claim 1, 2 or 3, wherein the method further comprises contacting the immature dendritic cell with one or more of IL-6, IFN-gamma, and PGE2, optionally before step (a) in claim 1.

8. The method of claim 4, wherein the enriching step comprises contacting the population of immature dendritic cells with one or more of IL-6, IFN-gamma, and PGE2.

9. The method of claim 4, wherein the enriching step comprises FACS.

10. A method for preventing the maturation of an immature dendritic cell, wherein the method comprises:
a) contacting the immature dendritic cell with an anti-CD32a antibody that blocks ligation of CD32a but not ligation of CD32b; and
b) activating CD32b signaling in the immature dendritic cell.

11. A method for preventing the maturation of an immature dendritic cell, wherein the method comprises contacting the immature dendritic cell with an anti-CD32a antibody that blocks ligation of CD32a but not ligation of CD32b, wherein the anti-CD32a antibody is an IgG.

12. The method of claim 10 or 11, wherein the method further comprises contacting the immature dendritic cell with soluble IgG monomer, TGF-beta, or IFN-alpha, optionally before step (a) of claim 10.

13. A method for promoting the maturation of a population of immature dendritic cells, wherein the method comprises:
a) contacting the population with one or more of IFN-gamma, PGE2, LPS and CD40L; and
b) activating CD32a signaling in the cells of the population resulting from step (a).

14. The method of claim 8 or 13, wherein the method further comprises inhibiting CD32b signaling in a cell resulting from step (a), optionally wherein CD32b signaling is inhibited by contacting a cell resulting from step (a) with an antagonist of CD32b signaling.

15. A method for preventing the maturation of an immature dendritic cell, wherein the method comprises:
a) contacting the immature dendritic cell with one or more of soluble IgG monomer, TGF-beta, and IFN-alpha; and
b) activating CD32b signaling in the immature dendritic cell.

16. The method of claim 15, wherein the method further comprises inhibiting CD32a signaling, optionally wherein CD32a signaling is inhibited by contacting the cell resulting from step (a) with an antagonist of CD32a signaling.

17. A method for promoting the maturation of an immature dendritic cell, wherein the method comprises contacting the immature dendritic cell with IgG that has a higher avidity for CD32a than for CD32b.

18. The method of claim 17, wherein the immature dendritic cell has at least one allele of the H variant of human CD32a and the IgG is human IgG2.

19. A method for preventing the maturation of an immature dendritic cell, wherein the method comprises contacting the immature dendritic cell with IgG that has a lower avidity for CD32a than for CD32b.

20. The method of claim 19, wherein the IgG is IgG4 or IgG3.

21. The method of claim 19, wherein the immature dendritic cell is homozygous for the R variant of human CD32a and the IgG is human IgG2.

22. The method of claim 1, 4 or 13, wherein CD32a signaling is activated in a cell by contacting the cell with an agonist of CD32a signaling, or by contacting the cell with complexed IgG or with immobilized IgG.

23. The method of claim 10 or 15, wherein CD32b signaling is activated in a cell by contacting the cell with an agonist of CD32b signaling, or by contacting the cell with complexed IgG or with immobilized IgG.

24. A method for identifying a molecule that inhibits CD32a signaling or blocks ligation of CD32a receptor more than CD32b signaling or ligation of CD32b receptor, or that inhibits CD32b signaling or blocks ligation of CD32b receptor more than CD32a signaling or ligation of 32a receptor, said method comprising:
a) contacting an immature dendritic cell that co-expresses CD32a and CD32b with a molecule;
b) contacting the immature dendritic cell with complexed IgG or with immobilized IgG;
c) determining the degree of maturation of the dendritic cell,
wherein the molecule inhibits CD32a signaling or blocks a ligation of CD32a receptor more than CD32b signaling or ligation of CD32b receptor if the dendritic cell is less matured in the presence of the molecule as compared to a control dendritic cell in the absence of the molecule, and
wherein the molecule inhibits CD32b signaling or blocks ligation of CD32b receptor more than CD32a signaling or ligation of CD32a receptor if the dendritic cell is more matured in the presence of the molecule as compared to a control dendritic cell in the absence of the molecule.

25. The method of claim 24, wherein the degree of maturation of the dendritic cell is determined by measuring the expression levels of CD83 and/or ILT3, or by measuring the levels of cytokines secreted by the dendritic cell.

26. The method of claim 25, wherein the cytokine is IL-8 or TNF-alpha.

27. A method of claims 24 to 26, wherein a population of immature dendritic cells is used.

28. A method for identifying a molecule that modifies the ratio of CD32a to CD32b expression on an immature dendritic cell, wherein the method comprises:
a) contacting an immature dendritic cell that co-expresses CD32a and CD32b with a molecule;
b) measuring the ratio of CD32a to CD32b expression on the dendritic cell, wherein the molecule modifies the ratio of CD32a to CD32b expression on a dendritic cell if the ratio of CD32a to CD32b expression on the dendritic cell as determined in step (b) is different from the ratio of CD32a to CD32b expression on a dendritic cell in the absence of the molecule.

29. A method for producing a tolerogenic dendritic cell, wherein the method comprises:
a) contacting an immature dendritic cell with an anti-CD32a specific antibody that blocks ligation of CD32a; and
b) activating CD32b signaling in the immature dendritic cell.

30. A method for producing an antigen-specific tolerogenic dendritic cell, wherein the method comprises:
a) contacting an immature dendritic cell with an anti-CD32a specific antibody that blocks ligation of CD32a;
b) activating CD32b signaling in the immature dendritic cell; and
c) targeting an antigen to the dendritic cell.

31. A method for producing an antigen-specific tolerogenic dendritic cell that induces tolerance against an antigen, wherein the method comprises:
a) contacting an immature dendritic cell with an anti-CD32a specific antibody that blocks ligation of CD32a;
b) contacting the dendritic cell with a complex of an antigen and an antibody; and
c) activating CD32b signaling in the immature dendritic cell.

32. A dendritic cell, for use in treating an autoimmune disease or graft versus host disease in a subject, wherein the dendritic cell is produced by a method comprising:
a) contacting an immature dendritic cell obtained from the subject with an anti-CD32a specific antibody that blocks ligation of CD32a;
b) activating CD32b signaling in the immature dendritic cell.

33. A dendritc cell according to claim 32, wherein the method of producing the dendritic cell further comprises:
c) targeting the mature dendritic cell obtained from steps (a) to (b) with a self-antigen or the antigen that is the target of graft versus host disease in the host, or
contacting the mature dendritic cell obtained from steps (a) to (b) with a tissue that is the target of autoimmune disease or with graft tissue bound to an antibody.

34. The dendritic cell of claim 33, wherein the self-antigen is the target of the autoimmune disease in the subject.

35. The dendritic cell of claim 32 to 34, wherein the autoimmune disease is alopecia areata, ankylosing spondylitis, antiphospholipid syndrome, autoimmune Addison's disease, autoimmune diseases of the adrenal gland, autoimmune hemolytic anemia, autoimmune hepatitis, autoimmune oophoritis and orchitis, autoimmune thrombocytopenia, Behcet's disease, bullous pemphigoid, cardiomyopathy, celiac sprue-dermatitis, chronic fatigue immune dysfunction syndrome (CFIDS), chronic inflammatory demyelinating polyneuropathy, Churg-Strauss syndrome, cicatrical pemphigoid, CREST syndrome, cold agglutinin disease, Crohn's disease, discoid lupus, essential mixed cryoglobulinemia, fibromyalgia-fibromyositis, glomerulonephritis, Graves' disease, Guillain-Barre, Hashimoto's thyroiditis, idiopathic pulmonary fibrosis, idiopathic thrombocytopenia purpura (ITP), IgA neuropathy, juvenile arthritis, lichen planus, lupus erthematosus, Ménière's disease, mixed connective tissue disease, multiple sclerosis, type 1 or immune-mediated diabetes mellitus, myasthenia gravis, pemphigus vulgaris, pernicious anemia, polyarteritis nodosa, polychrondritis, polyglandular syndromes, polymyalgia rheumatica, polymyositis and dermatomyositis, primary agammaglobulinemia, primary biliary cirrhosis, psoriasis, psoriatic arthritis, Raynauld's phenomenon, Reiter's syndrome, Rheumatoid arthritis, sarcoidosis, scleroderma, Sjögren's syndrome, stiff-man syndrome, systemic lupus erythematosus, lupus erythematosus, takayasu arteritis, temporal arteristis/ giant cell arteritis, ulcerative colitis, uveitis, vasculitides such as dermatitis herpetiformis vasculitis, vitiligo, or Wegener's granulomatosis.

36. The method of claim 29, 30 or 31, or dendritic cell of claim 32, 33, 34 or 35, wherein the method further comprises contacting the immature dendritic cell with soluble IgG monomer, TGF-beta, or IFN-alpha.

37. The method of claim 29, 30 or 31, or the dendritic cell of claim 32, 33, 34 or 35, wherein CD32b signaling is activated in a cell by contacting the cell with complexed IgG or with immobilized IgG, or by contacting the cell with an CD32b agonist.

38. A method for producing mature dendritic cells suitable as adjuvant in a vaccine, wherein the method comprises:
a) contacting an immature dendritic cell with an anti-CD32b specific antibody that blocks ligation of CD32b;
b) activating CD32a signaling in the immature dendritic cell; and
c) formulating the mature dendritic cell obtained in step (b) into the vaccine.

39. A method for producing a vaccine against an antigen, wherein the method comprises:
a) contacting an immature dendritic cell with an anti-CD32b specific antibody that blocks ligation of CD32b;
b) activating CD32a signaling in the immature dendritic cell; and
c) targeting the antigen to the mature dendritic cell obtained in step (b).

40. The method of claim 38 or 39, wherein the vaccine is an anti-cancer vaccine or a vaccine against an infectious disease.

41. A method for stimulating a T cell *ex vivo,* wherein the method comprises:
a) contacting an immature dendritic cell with an anti-CD32b specific antibody that blocks ligation of CD32b;
b) targeting an antigen to the mature dendritic cell obtained in steps (a) to (b);
c) activating CD32a signaling in the immature dendritic cell; and
d) contacting a T cell with the mature antigen-specific dendritic cell obtained in step (c).

42. The stimulated T cells produced by the method of claim 42, for use in the treatment of cancer of neoplastic disease in a patient, wherein the antigen is an antigen associated with a cancer or neoplastic disease.

43. The method of claim 38, 39 or 40, wherein the method further comprises contacting the immature dendritic cell with IL-6, IFN-gamma, or PGE2.

44. The method of claim 40, wherein the T cell is CD4 positive or CD8 positive.

45. The method of any one of claims 1 to 31, 36 to 41 or 43 or 44, wherein the immature dendritic cell is a monocyte-derived dendritic cell (moDCs).

46. The method of claim 38, 39, or 40, wherein CD32a signaling is activated in a cell by contacting the cell with complexed IgG or with immobilized IgG.

47. A dendritic cell generated by a process according to claim 3 or claim 7.

48. A kit comprising an anti-CD32b antibody that blocks ligation of CD32b but not ligation of CD32a; and IgG, and optionally one or more of IL-6, IFN-gamma, and PGE2.

49. The method of claim 1 or the kit of claim 48, wherein the anti-CD32b antibody is monoclonal antibody 2B6.

50. A kit comprising an anti-CD32a antibody that blocks ligation of CD32a but not ligation of CD32b; and IgG, and optionally one or more of soluble IgG monomer, TGF-beta, and IFN-alpha.

51. The method of claim 10 or the kit of claim 50, wherein the anti-CD32a antibody is monoclonal antibody IV.3.

52. A kit comprising (i) IL-6, IFN-gamma, PGE2, or LPS and CD40L; and (ii) IgG.

53. The kit of claim 48, 50 or 52, further comprising means to immobilize IgG or means to complex IgG, and/or further comprising means for isolating immature dendritic cells.

54. Use of a composition comprising antibodies that block ligation of CD32a, for the manufacture of a medicament for treating in a subject a disorder that can be treated by lowering IL10 or IL6 levels in the subject.

55. The use of claim 54, wherein the disorder that can be treated by lowering IL10 levels is rheumatoid arthritis, systemic lupus erythematosus, HIV infection, organ transplant rejection, or burn-induced immunosuppression; and
wherein the disorder that can be treated by lowering IL6 levels is multiple myeloma, lymphoma, Waldenstrom's Macroglobulinemia, Castleman's Disease, rheumatoid arthritis, post-(bone marrow or whole organ) transplant lymphoproliferative disorder (PTLD), prostate cancer, autoimmunity, autoimmune hemolytic anemia (AIHA), amyloidosis, Crohn's disease, renal cell carcinoma, cancer-related cachexia/anorexia, cancer-related muscle atrophy, overwhelming infections/sepsis, herpes virus reactivation, or immunosuppression associated with alcohol consumption prior to burn injuries.
